# EUROPEAN PATENT APPLICATION

(11) **EP 4 015 651 A1**
(43) Date of publication of application: **22.06.2022**
(21) Application number: 20214794.8
(22) Date of filing: 17.12.2020
(51) Int. Cl.: C12Q 1/6883, G01N 33/68

(54) **TREATMENT PREDICTION AND EFFECTIVENESS OF ANTI-TNF ALPHA TREATMENT IN IBD PATIENTS**

(71) Applicant: Koninklijke Philips N.V., 5656 AG Eindhoven (NL)
(72) Inventor: BOUWMAN, Wilbert Hendrik, 5656 AE Eindhoven (NL); VAN DE STOLPE, Anja, 5656 AE Eindhoven (NL)
(74) Representative: Philips Intellectual Property & Standards

(57) **Abstract**

The invention relates to methods based on gene expression levels of at least 3 genes, e.g. ANGPTL4 and two other genes, to predict treatment response to an anti-TNFalpha compound in inflammatory bowel disease patients based on a pre-treatment biopsy, and a method of determining the treatment effectiveness of such treatment based on a biopsy obtained after initiation of the treatment with an anti-TNFalpha compound. The invention further relates to the use of anti-TNFalpha compounds in the treatment of Inflammatory bowel disease wherein the treatment is combined with the method for predicting treatment response and only administered when a favorable response is predicted. Lastly, the invention relates to kits and uses thereof in the methods described herein.

## Description

### FIELD OF THE INVENTION

The present invention relates to the field of diagnostics, in particular in the field of Inflammatory bowel diseases such as Crohn's disease and Ulcerative colitis. The invention in particular relates to methods based on gene expression levels to predict treatment response to an anti-TNFalpha compound based on a pre-treatment biopsy, and to method of determining the treatment effectiveness of such treatment based on a biopsy obtained after initiation of the treatment with an anti-TNFalpha compound. The invention further relates to the use of anti-TNFalpha compounds in the treatment of Inflammatory bowel disease wherein the treatment is combined with the method for predicting treatment response and only administered when a favorable response is predicted. Lastly, the invention relates to kits and uses thereof in the methods and uses described herein.

### BACKGROUND OF THE INVENTION

Inflammatory bowel disease (IBD) is a group of inflammatory conditions of the colon and small intestine. Crohn's disease (CD) and Ulcerative colitis (UC) are the principal types of inflammatory bowel disease, with each a different auto-immune pathophysiology. Crohn's disease affects the small intestine and large intestine, as well as the mouth, esophagus, stomach and the anus, whereas ulcerative colitis primarily affects the colon and the rectum. In 10% of cases, definitive discrimination in not possible. However, this is important to decide the most suitable surgical therapy, determining the risk of cancer (especially in UC), and systemic drug therapy selection. This holds especially when targeted drugs are used that target the underlying pathophysiology and consequently only work for a specific disease or disease type.

Even when using histopathology analysis in combination with other diagnostic measurements and patient signs and symptoms, it can still be difficult to diagnose the correct type of inflammatory bowel disease and to treat the disease correctly. Treatment can be directed at inhibition of the inflammation and auto-immune reaction, healing of the intestinal wall, and correcting the microbiome. Multiple types of drugs can inhibit the inflammation and immune response, e.g. corticosteroids and TNFalpha blockers. Use of combination therapy (anti-tumour necrosis factor (TNF) α monoclonal antibodies and an immunomodulator) is one of the most effective treatments in Crohn Disease (CD) (Colombel et al. N Engl J Med 2010; 362:1383-1395 DOI: 10.1056/NEJMoa0904492; incorporated by reference) particularly when given early in the disease course (D'Haens, G. Top-down therapy for IBD: rationale and requisite evidence. Nat Rev Gastroenterol Hepatol 7, 86-92 (2010). https://doi.org/10.1038/nrgastro.2009.222; D'Haens et al. 2008 VOLUME 371, ISSUE 9613, P660-667, FEBRUARY 23, 2008 DOI:https://doi.org/10.1016/S0140-6736(08)60304-9, each hereby incorporated by reference), but indiscriminate use of this strategy would be very expensive and expose many patients to side effects of drugs that they do not require. Unfortunately, in IBD, as in most autoimmune and inflammatory diseases, biomarkers that can reliably predict response or resistance to anti-TNFalpha drugs, and to measure the extent of the actual response, as well as emerging drug resistance during treatment are not available, precluding the delivery of personalized therapy.

Therefore novel ways of predicting treatment response and effectiveness of treatment with anti-TNFalpha compounds are desired.

### SUMMARY OF THE INVENTION

In in a first aspect the invention relates to a method for predicting the treatment response of a subject suffering from inflammatory bowel disease (IBD) to treatment with an anti-TNFalpha compound based on the expression levels of three or more genes, wherein the expression levels of the three or more genes are selected from the group consisting of:
ANGPTL4, BCL2L1, BCL2L11, BIRC3, CCL2, CCL22, CCL3, CCL4, CCL5, CCND1, CD274, CD44, CDKN2B, COL18A1, CTGF, CX3CL1, CXCL1, CXCL2, CXCL3, DKK1, DNMT1, ENPP2, FGF2, FOS, FSCN1, GADD45B, GLRX, HIF1A, HMGA2, HNF1A, HSP90AB1, HSP90B1, ICAM1, IFNG, IL11, IL10, IL1B, IL6, IL8, IRF1, INPP5D, JUNB, KIAA1199, LEF1, MCL1, MMP1, MMP2, MMP3, MMP9, MYC, NFKB2, NFKBIA, NFKBIE, NOS2, OVOL1, PDGFB, PLAU, PLAUR, PTGS2, PTHLH, SAA1, SELE, SERPINE1, SKIL, SMAD4, SNAI2, STAT1, STAT5A, TBX3, TCF7L2, TIMP1, TNF, TNFAIP2, TNFRSF1B, TRAF1, TWIST1, VCAM1, VEGFA, VIM, and ZEB1, and
wherein an increased expression level of CDKN2B, HMGA2, OVOL1, SMAD4, BCL2L11, HNF1A or TCF7L2, or a decreased expression level of any of the other genes corresponds to a favorable treatment response, and
wherein a decreased expression level of CDKN2B, HMGA2, OVOL1, SMAD4, BCL2L11, HNF1A or TCF7L2, or an increased expression level of any of the other genes corresponds to a not favorable treatment response.

In a second aspect, the invention relates to a method for determining the effectiveness of a treatment of a subject suffering from IBD with an anti-TNFalpha compound based on the expression levels of three or more genes,
wherein if the IBD is UC, the three or more genes are selected from the group consisting of BCL2L11, CDKN1A, DNMT1, EAF2, ELL2, ENPP2, FKBP5, GLRX, LCP1, MMP1, MMP3, MMP9, PLAU, PLAUR, PMEPA1, PRKACB, PTGS2, SERPINE1, SGK1, TIMP1, TMPRSS2 and VIM; or
wherein the IBD is CD, the three or more genes are selected from the group consisting of BCL2L1, BCL2L11, BIRC3, CCL2, CCL22, CCL3, CCL4, CCL5, CX3CL1, CXCL1, CXCL2, CXCL3, DNMT1, ENPP2, GLRX, ICAM1, IL1B, IL6, IL8, IRF1, MMP1, MMP3, MMP9, NFKB2, NFKBIA, NFKBIE, PLAU, PLAUR, PTGS2, SELE, SERPINE1, STAT5A, TIMP1, TNF, TNFAIP2, TRAF1, VCAM1 and VIM;
wherein the three or more expression levels are used to determine whether the treatment of IBD in the patient with an anti-TNFalpha compound is effective or not effective,
wherein a decreased expression level of BCL2L11 or PRKACB or an increased expression level of any of the other genes corresponds to a not effective treatment, and
wherein an increased expression level of BCL2L11 or PRKACB or a decreased expression level of any of the other genes corresponds to an effective treatment.

In a third aspect, the invention relates to an anti-TNFalpha compound for use of in the treatment of IBD in a subject, wherein the use comprises:
performing the method as defined in any one of the preceding claims to determine the treatment response of the subject to anti-TNFalpha treatment, and
administering the anti-TNFalpha compound of the treatment response to anti-TNFalpha treatment is found to be favorable for the subject.

In a fourth aspect, the invention relates to a kit of parts comprising primers and probes for the detection of the expression levels of three or more target genes, wherein the expression levels of the three or more genes are selected from:
the group consisting of ANGPTL4, BCL2L1, BCL2L11, BIRC3, CCL2, CCL22, CCL3, CCL4, CCL5, CCND1, CD274, CD44, CDKN2B, COL18A1, CTGF, CX3CL1, CXCL1, CXCL2, CXCL3, DKK1, DNMT1, ENPP2, FGF2, FOS, FSCN1, GADD45B, GLRX, HIF1A, HMGA2, HNF1A, HSP90AB1, HSP90B1, ICAM1, IFNG, IL11, IL10, IL1B, IL6, IL8, IRF1, INPP5D, JUNB, KIAA1199, LEF1, MCL1, MMP1, MMP2, MMP3, MMP9, MYC, NFKB2, NFKBIA, NFKBIE, NOS2, OVOL1, PDGFB, PLAU, PLAUR, PTGS2, PTHLH, SAA1, SELE, SERPINE1, SKIL, SMAD4, SNAI2, STAT1, STAT5A, TBX3, TCF7L2, TIMP1, TNF, TNFAIP2, TNFRSF1B, TRAF1, TWIST1, VCAM1, VEGFA, VIM, and ZEB1; or
the group consisting of BCL2L11, CDKN1A, DNMT1, EAF2, ELL2, ENPP2, FKBP5, GLRX, LCP1, MMP1, MMP3, MMP9, PLAU, PLAUR, PMEPA1, PRKACB, PTGS2, SERPINE1, SGK1, TIMP1, TMPRSS2 and VIM; or
the group consisting of BCL2L1, BCL2L11, BIRC3, CCL2, CCL22, CCL3, CCL4, CCL5, CX3CL1, CXCL1, CXCL2, CXCL3, DNMT1, ENPP2, GLRX, ICAM1, IL1B, IL6, IL8, IRF1, MMP1, MMP3, MMP9, NFKB2, NFKBIA, NFKBIE, PLAU, PLAUR, PTGS2, SELE, SERPINE1, STAT5A, TIMP1, TNF, TNFAIP2, TRAF1, VCAM1 and VIM.

In a fifth aspect the invention relates to a use of the kit according to the fourth aspect of the invention in the method according to the first or the second aspect of the invention.

### DETAILED DESCRIPTION OF THE INVENTION

Currently available treatment decisions are based on the disease diagnosis from histopathology with other diagnostic measurements. It was found that using pathway activity models diagnosis can be linked to a pathophysiological disease mechanism, in terms of a specific combined signaling pathway activity profile, and this can be used to predict treatment response, treatment effectiveness and treatment monitoring. The inventors used pathway analysis of public datasets to determine which cellular signaling pathways can be used to predict whether a patient suffering from IBD will have a good treatment response to treatment with an anti-TNFalpha compound, to determine the effectiveness in patients being treated with an anti-TNFalpha compound. It was found that there is a significant elevation in the TGFbeta, NFkB, MAPK-AP1, STAT3 and WNT pathways in biopsies from IBD patients, pre-treatment, that have poor response to anti-TNFalpha treatment as compared to IBD patients that respond well to anti-TNFalpha treatment. These results hold both for Ulcerative Colitis and Crohn's Disease. Further is was found that treatment effectiveness can be determined for Ulcerative Colitis and Crohn's Disease patients undergoing anti-TNFalpha treatment based on the AR and/or the MAPK-AP1 cellular signaling in the case of Ulcerative Colitis, or based on the MAPK-AP1 and/or the NFkB cellular signaling pathway in case of Crohn's Disease.

The inventors investigated further whether the target genes that form the basis of these cellular signaling pathway activity models can be used instead of the pathway models. To this extend, it was determined for each pathway which target genes provided the biggest contribution to the cellular signaling pathway activity score in the publicly available datasets used here. For each application, a selection of the cellular signaling pathway target genes was made that most contributed to the pathway activity score in that respective application. It was then tested whether random selections of target gene expression levels allow for distinguishing between different situations (e.g. responders and non-responders in pre-treatment biopsies) in a significant manner. It was found that a random selection of three target genes selected as described above allow for significant stratification in most cases. Depending on the application and strictness of target gene selection, between approximately 90 to 100 percent of the random selections of three target genes allows for a significant distinction between the different groups (e.g. responders and non-responders, or effective treatment vs non-effective treatment).

Therefore, in a first aspect the invention relates to a method for predicting the treatment response of a subject suffering from inflammatory bowel disease (IBD) to treatment with an anti-TNFalpha compound based on the expression levels of three or more, e.g. three, four, five, six seven, eight, nine, ten, eleven or twelve or more, genes, wherein the expression levels of the three or more, e.g. three, four, five, six seven, eight, nine, ten, eleven or twelve or more, genes are selected from the group consisting of:
ANGPTL4, BCL2L1, BCL2L11, BIRC3, CCL2, CCL22, CCL3, CCL4, CCL5, CCND1, CD274, CD44, CDKN2B, COL18A1, CTGF, CX3CL1, CXCL1, CXCL2, CXCL3, DKK1, DNMT1, ENPP2, FGF2, FOS, FSCN1, GADD45B, GLRX, HIF1A, HMGA2, HNF1A, HSP90AB1, HSP90B1, ICAM1, IFNG, IL11, IL10, IL1B, IL6, IL8, IRF1, INPP5D, JUNB, KIAA1199, LEF1, MCL1, MMP1, MMP2, MMP3, MMP9, MYC, NFKB2, NFKBIA, NFKBIE, NOS2, OVOL1, PDGFB, PLAU, PLAUR, PTGS2, PTHLH, SAA1, SELE, SERPINE1, SKIL, SMAD4, SNAI2, STAT1, STAT5A, TBX3, TCF7L2, TIMP1, TNF, TNFAIP2, TNFRSF1B, TRAF1, TWIST1, VCAM1, VEGFA, VIM, and ZEB1, and
wherein an increased expression level of CDKN2B, HMGA2, OVOL1, SMAD4, BCL2L11, HNF1A or TCF7L2, or a decreased expression level of any of the other genes corresponds to a favorable treatment response, and
wherein a decreased expression level of CDKN2B, HMGA2, OVOL1, SMAD4, BCL2L11, HNF1A or TCF7L2, or an increased expression level of any of the other genes corresponds to a not favorable treatment response.

In an embodiment the three or more expression levels are used to predict whether the treatment response to treatment of IBD in the patient with an anti-TNFalpha compound is favorable or not favorable.

In an embodiment a decreased expression level of CDKN2B, HMGA2, OVOL1, SMAD4, BCL2L11, HNF1A or TCF7L2, or an increased expression level of any of the other genes corresponds to a not favorable treatment response.

When used herein, treatment prediction refers to predicting the likelihood of an IBD patient (e.g. an UC or a CD patient) to having a treatment response as defined herein below to treatment with an anti-TNFalpha compound, wherein said treatment prediction is made prior to treatment, for example based on a biopsy or sample from the patient.

When used herein, treatment effectiveness refers to predicting the likelihood of an IBD patient (e.g. an UC or a CD patient) to having a treatment response as defined herein below to treatment with an anti-TNFalpha compound, wherein said treatment prediction is made after onset of said treatment, for example based on a biopsy or sample from the patient.

It was found that the expression levels of the following genes strongly correlate with pathway activity in the investigated public datasets: ANGPTL4, BCL2L1, BCL2L11, BIRC3, CCL2, CCL22, CCL3, CCL4, CCL5, CCND1, CD274, CD44, CDKN2B, COL18A1, CTGF, CX3CL1, CXCL1, CXCL2, CXCL3, DKK1, DNMT1, ENPP2, FGF2, FOS, FSCN1, GADD45B, GLRX, HIF1A, HMGA2, HNF1A, HSP90AB1, HSP90B1, ICAM1, IFNG, IL11, IL10, IL1B, IL6, IL8, IRF1, INPP5D, JUNB, KIAA1199, LEF1, MCL1, MMP1, MMP2, MMP3, MMP9, MYC, NFKB2, NFKBIA, NFKBIE, NOS2, OVOL1, PDGFB, PLAU, PLAUR, PTGS2, PTHLH, SAA1, SELE, SERPINE1, SKIL, SMAD4, SNAI2, STAT1, STAT5A, TBX3, TCF7L2, TIMP1, TNF, TNFAIP2, TNFRSF1B, TRAF1, TWIST1, VCAM1, VEGFA, VIM, and ZEB1,

Of this set of genes, the expression levels of CDKN2B, HMGA2, OVOL1, SMAD4, BCL2L11, HNF1A or TCF7L2 demonstrate a negative correlation with pathway activity, meaning their expression levels are reduced with increased pathway activity. Therefore when adding up expression levels of multiple target gene the expression levels of the genes CDKN2B, HMGA2, OVOL1, SMAD4, BCL2L11, HNF1A or TCF7L2 are preferably modified with a negative constant, preferably with "-1".

The expression levels of ANGPTL4, BCL2L1, BIRC3, CCL2, CCL22, CCL3, CCL4, CCL5, CCND1, CD274, CD44, COL18A1, CTGF, CX3CL1, CXCL1, CXCL2, CXCL3, DKK1, DNMT1, ENPP2, FGF2, FOS, FSCN1, GADD45B, GLRX, HIF1A, HSP90AB1, HSP90B1, ICAM1, IFNG, IL11, IL10, IL1B, IL6, IL8, IRF1, INPP5D, JUNB, KIAA1199, LEF1, MCL1, MMP1, MMP2, MMP3, MMP9, MYC, NFKB2, NFKBIA, NFKBIE, NOS2, PDGFB, PLAU, PLAUR, PTGS2, PTHLH, SAA1, SELE, SERPINE1, SKIL, SNAI2, STAT1, STAT5A, TBX3, TIMP1, TNF, TNFAIP2, TNFRSF1B, TRAF1, TWIST1, VCAM1, VEGFA, VIM, and ZEB1 demonstrate a positive correlation with pathway activity, meaning their expression levels are increased with increased pathway activity.

As can be deducted from the data presented herein, by using more target genes the accuracy of the prediction can be increased, as is evident from the comparison of three gene pathway models vs full pathway models, and the data comparing statistical accuracy of a prediction based on a random selection of three vs six genes. Therefore it may be desirable to increase the number of target gene expression levels that are used in the prediction, however it is emphasized that as demonstrated here a selection of three target genes suffices to make accurate predictions.

In an embodiment the IBD is Ulcerative Colitis (UC) or Crohn Disease (CD). It was found that these genes were predictive for treatment response both UC and CD patients.

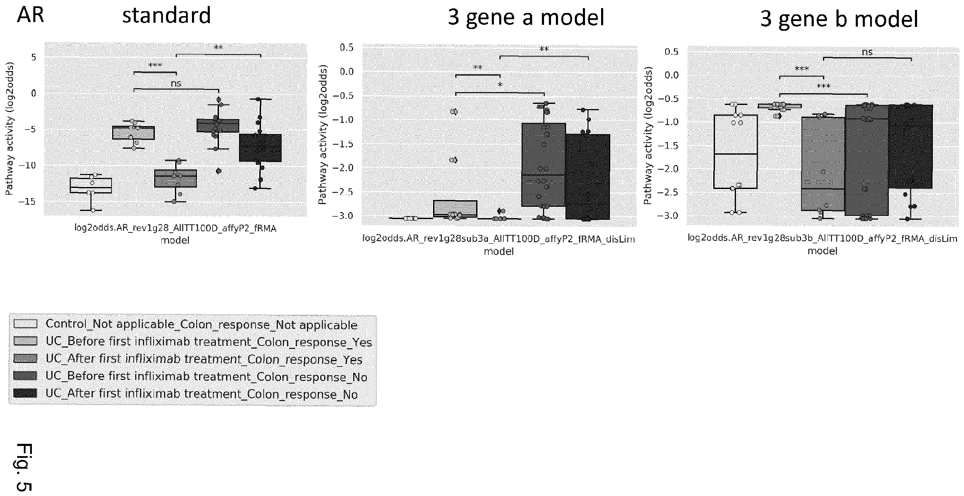

Using a random selection of three genes from the groups listed above a significant distinction can be made between responders and non-responders to anti-TNFalpha treatment in a pre-treatment biopsy in both Ulcerative Colitis and Crohn's Disease patients, as evidenced by Figs. 5 to 20.

Currently no real methods are available to predict successful outcome of anti-TNFalpha treatment in Inflammatory Bowel Disease patients [Lopetuso, Loris Riccardo et al. "Can We Predict the Efficacy of Anti-TNF-a Agents?." International journal of molecular sciences vol. 18,9 1973. 14 Sep. 2017, doi:10.3390/ijmsl8091973; incorporated by reference in its entirety]. Literature data report that primary non-response to anti-TNF-a induction therapy (assessed not before weeks 8-12 after starting treatment) occurs between 20-40% of patients in clinical trials and in 10-20% in "real life" series. Secondary loss of response (LOR) is also a common clinical problem with incidence ranging between 23 and 46% at 12 months after anti-TNF-a initiation [Ben-Horin S., Kopylov U., Chowers Y. Optimizing anti-TNF treatments in inflammatory bowel disease. Autoimmun. Rev. 2014;13:24-30. doi: 10.1016/j.autrev.2013.06.002.]. The use of biological therapies, in particular anti-tumor necrosis factor (TNF)-α agents, has spread increasingly in the last two decades thanks to their potential and unique ability of altering the natural history of IBD. In fact, both controlled trials and "real life" studies showed that anti-TNF-a agents, including infliximab (IFX), adalimumab (ADA), golimumab (GOL) and certolizumab pegol (CER), are able to induce and maintain clinical remission and to reduce the need for surgery and hospitalization. Furthermore, they lead to an improvement in quality of life and also to a reduction in direct and indirect costs associated with these chronic debilitating disorders.

Unfortunately, anti-TNF-a agents are not a magic bullet for all IBD cases. Firstly, side-effects can be severe and life-threatening in some patients, mainly due to infective events and then to immunogenicity, with the formation of antibodies-to-anti-TNF-a and a consequent loss of response (LOR) to these drugs over time. Therefore, there is a great need for accurate prediction methods to anti-TNFalpha therapies in IBD patients. The methods disclosed herein provide an accurate method for predicting treatment response in IBD patients to anti-TNFalpha therapy based on a biopsy, as evidenced by the Experimental results below. The method is based on the expression levels of a carefully curated set of genes which are found to be predictive.

To identify cellular signaling pathways that could be predictive in pre-treatment biopsies, pathway activities were analyzed on public datasets (GSE12251, GSE16879, GSE23597 and GSE52746). It was found that, individually, each of the TGFbeta, NFkB, MAPK-AP1, STAT3 and WNT cellular signaling pathways could be used to predict if a patient would responds to treatment with an anti-TNFalpha compound, by comparing the cellular signaling pathway activities of responders and non-responders in pre-treatment biopsies. Pathway activities for each of the TGFbeta, NFkB, MAPK-AP1, STAT3 and WNT cellular signaling pathways were significantly higher in pre-treatment biopsies from subjects that do not respond to treatment with an anti-TNFalpha compound compared to subjects that respond well to treatment with an anti-TNFalpha compound. Therefore as part of the invention disclosed herein, the invention relates to a method for predicting the treatment response of a subject suffering from inflammatory bowel disease (IBD) to treatment with an anti-TNFalpha compound based on inferred cellular signaling pathway activity or activities in a sample obtained from the subject, the method comprising comparing the inferred cellular signaling pathway activity, or when multiple cellular signaling pathway activities are used the combined cellular signaling pathway activities, with a threshold value, wherein the cellular signaling pathway is one or more cellular signaling pathway selected from TGFbeta, NFkB, MAPK-AP1, STAT3 and WNT, as detailed herein below. The target genes used for determining cellular signaling pathway activity using these pathway models are preferably selected as follows:
genes of the TGFbeta pathway are selected from the group consisting of ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKNIA, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKNIA, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7;
genes of the NFkB pathway are selected from the group consisting of BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKB IE, PTGS2, SELE, STAT5A, TNF, TNFAIP2, TNIP1, TRAF1, and VCAM1;
genes of the MAPK-AP1 pathway are selected from the group consisting of BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably, from the group consisting of: CCND1, EGFR, EZR, GLRX, MMP1, MMP3, PLAU, PLAUR, SERPINE1, SNCG, and TIMP1;
genes of the STAT3 pathway are selected from the group consisting of AKT1, BCL2, BCL2L1, BIRC5, CCND1, CD274, CDKNIA, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JUNB, MCL1, MMP1, MMP3, MMP9, MUC1, MYC, NOS2, POU2F1, PTGS2, SAA1, STAT1, TIMP1, TNFRSF1B, TWIST1, VIM, and ZEB1, preferably, either from the group consisting of: BCL2L1, BIRC5, CCND1, CD274, FOS, HIF1A, HSP90AA1, HSP90AB1, MMP1, and MYC, or from the group consisting of: BCL2L1, CD274, FOS, HSP90B1, HSPA1B, ICAM1, IFNG, JUNB, PTGS2, STAT1, TNFRSF1B, and ZEB1;
genes of the WNT pathway are selected from the group consisting of KIAA1199, AXIN2, R F43, TBX3, TDGF1, SOX9, ASCL2, IL8, SP5, Z RF3, KLF6, CCND1, DEFA6 and FZD7, optionally further comprising one or more target genes selected from NKD1, OAT, FAT1, LEF1, GLUL, REG1B, TCF7L2, COL18A1, BMP7, SLC1A2, ADRA2C, PPARG, DKK1, HNF1A and LECT2.

Next the inventors reviewed whether a specific subset of target genes in the pathway models is responsible for the pathway activity. The pathway models are constructed with a large set of genes keeping in mind that they are broadly applicable, therefore not every gene will be upregulated in a specific context (e.g. cell or tissue type, or specific biological function), although it is found that in most cases a randomly selected subset of three genes should be predictive of pathway activity in any application. For the specific application, predicting response to anti-TNFalpha treatment based on a pre-treatment biopsy of a subject suffering from IBD can be based on a random selection of three genes. Therefore target genes that contribute less to the total pathway activity were identified and removed from the list, resulting in a cured list of target genes. The curation of the target genes list was performed based on total cellular signaling pathway activity regardless of group (responder vs non-responder) as to not introduce any bias. The curated target genes lists are:
group 1 (curated TGFbeta list) consist of the genes: ANGPTL4, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI2, TIMP1, VEGFA
group 2 (curated NFkB list) consist of the genes: BCL2L1, BIRC3, CCL2, CCL22, CCL3, CCL4, CCL5, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TRAF1, VCAM1;
group 3 (curated MAPK-AP1 list) consist of the genes: BCL2L11, CCND1, DNMT1, ENPP2, GLRX, MMP1, MMP3, MMP9, PLAU, PLAUR, PTGS2, SERPINE1, TIMP1, VIM;
group 4(curated STAT3 list) consist of the genes: BCL2L1, CCND1, CD274, FGF2, FOS, FSCN1, HIF1A, HSP90AB1, HSP90B1, ICAM1, IFNG, IL10, JUNB, MCL1, MMP1, MMP3, MMP9, MYC, NOS2, PTGS2, SAA1, STAT1, TIMP1, TNFRSF1B, TWIST1, VIM, ZEB1;
group 5 (curated WNT list) consist of the genes: CCND1, CD44, COL18A1, DKK1, HNF1A, IL8, KIAA1199, LEF1, MYC, TBX3, TCF7L2.

Therefore in an embodiment, the invention relates to a method for predicting the treatment response of a subject suffering from inflammatory bowel disease (IBD) to treatment with an anti-TNFalpha compound based on the expression levels of three or more genes, wherein the expression levels of the three or more genes are selected from one or more groups selected from group 1, group 2, group 3, group 4, and group 5; and wherein group 1 consist of the genes: ANGPTL4, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI2, TIMP1, VEGFA; wherein group 2 consist of the genes: BCL2L1, BIRC3, CCL2, CCL22, CCL3, CCL4, CCL5, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TRAF1, VCAM1; wherein group 3 consist of the genes: BCL2L11, CCND1, DNMT1, ENPP2, GLRX, MMP1, MMP3, MMP9, PLAU, PLAUR, PTGS2, SERPINE1, TIMP1, VIM; wherein group 4 consist of the genes: BCL2L1, CCND1, CD274, FGF2, FOS, FSCN1, HIF1A, HSP90AB1, HSP90B1, ICAM1, IFNG, IL10, JUNB, MCL1, MMP1, MMP3, MMP9, MYC, NOS2, PTGS2, SAA1, STAT1, TIMP1, TNFRSF1B, TWIST1, VIM, ZEB1; wherein group 5 consist of the genes: CCND1, CD44, COL18A1, DKK1, HNF1A, IL8, KIAA1199, LEF1, MYC, TBX3, TCF7L2; and wherein the three or more expression levels are used to predict whether the treatment response to treatment of IBD in the patient with an anti-TNFalpha compound is favorable or not favorable, wherein a decreased expression level of CDKN2B, HMGA2, OVOL1, SMAD4, BCL2L11, HNF1A or TCF7L2, or an increased expression level of any of the other genes corresponds to a not favorable treatment response, and wherein an increased expression level of CDKN2B, HMGA2, OVOL1, SMAD4, BCL2L11, HNF1A or TCF7L2, or a decreased expression level of any of the other genes corresponds to a favorable treatment response. Preferably, wherein: group 1 consist of the genes: ANGPTL4, CDKN2B, CTGF, GADD45B, IL11, INPP5D, JUNB, MMP2, MMP9, PTHLH, SERPINE1, SNAI2, TIMP1; group 2 consist of the genes: BCL2L1, BIRC3, CCL2, CCL22, CCL3, CCL4, CCL5, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TRAF1, VCAM1; wherein group 3 consist of the genes: CCND1, GLRX, MMP1, MMP3, MMP9, PLAU, PLAUR, PTGS2, SERPINE1, TIMP1, VIM; wherein group 4 consist of the genes: BCL2L1, CCND1, CD274, FGF2, FSCN1, HIF1A, HSP90B1, ICAM1, IFNG, JUNB, MCL1, MMP1, MMP3, MMP9, PTGS2, STAT1, TIMP1, TNFRSF1B, TWIST1, VIM, ZEB1; wherein group 5 consist of the genes: CCND1, CD44, COL18A1, DKK1, IL8, KIAA1199, TBX3. The three or more genes may be selected from a single group or may be a combination of different groups. As the strongest correlation was found between the NFkB and TGFbeta cellular signaling pathway activities and treatment response prediction, preferably the three or more genes are chosen form group 1 and/or group 2.

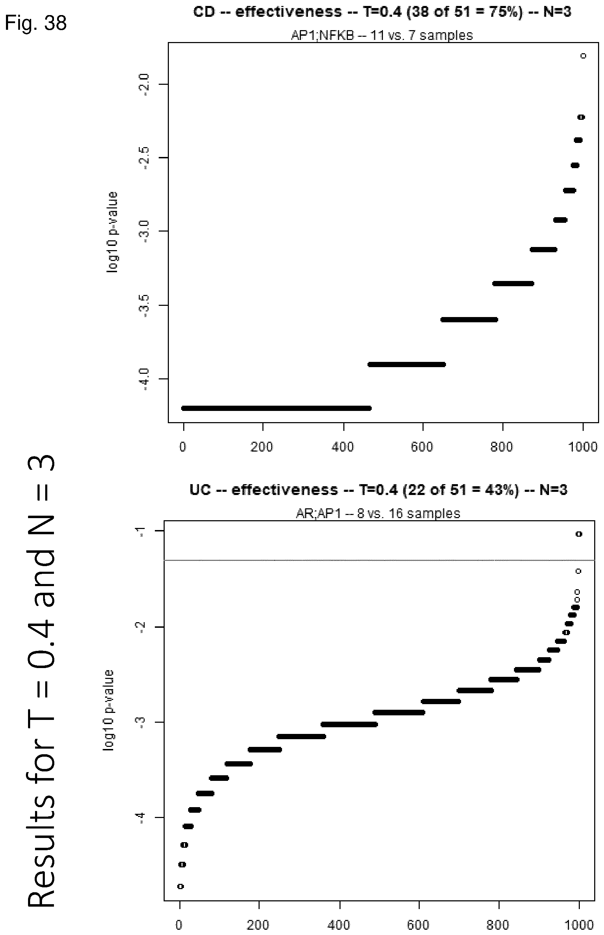

Next all the curated pathway target genes were pooled, and the inventors analyzed if random selections of three genes of the pooled curated target genes would still allow a prediction for treatment response. In order to analyze this, a random selection of three genes was made 1000 times, and a simple linear classifier was made with those 1000 random selections of three genes by assigning each gene a weight +1 or -1 depending on the sign of their correlation (up- or down regulated when pathway activity is increased). Next the linear classifier was applied on the samples from the two sample groups to be distinguished, and a Wilcox Rank Sum test was performed, resulting in a p-value. These p-values are plotted in Figs. 38 to 45. As can be seen, at least 90% percent of the random selections of three genes of the pooled curated target genes has statistically relevant combined expression levels between responders and non-responders (p value of 0.05 or lower).

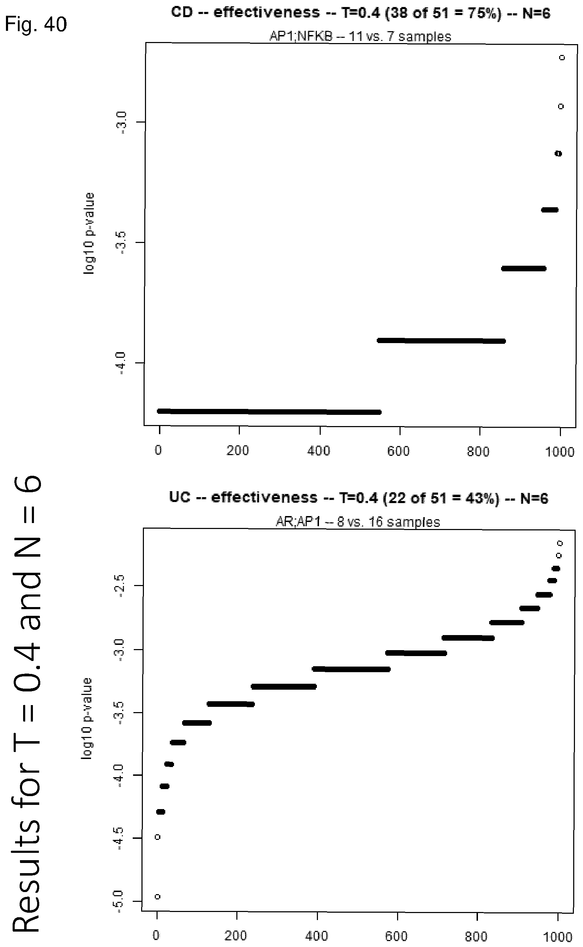

As expected, by selecting more curated target genes for the prediction, the likelihood that a random selection of curated target genes allow for statistically significant prediction is increased (see Figs. 40, 41, 44 and 45). Therefore, in an embodiment, the method is preferably based on three or more, for example three, four, five, six, seven eight, nine, ten, eleven or twelve or more, genes selected from the group consisting of ANGPTL4, BCL2L1, BCL2L11, BIRC3, CCL2, CCL22, CCL3, CCL4, CCL5, CCND1, CD274, CD44, CDKN2B, COL18A1, CTGF, CX3CL1, CXCL1, CXCL2, CXCL3, DKK1, DNMT1, ENPP2, FGF2, FOS, FSCN1, GADD45B, GLRX, HIF1A, HMGA2, HNF1A, HSP90AB1, HSP90B1, ICAM1, IFNG, IL11, IL10, IL1B, IL6, IL8, IRF1, INPP5D, JUNB, KIAA1199, LEF1, MCL1, MMP1, MMP2, MMP3, MMP9, MYC, NFKB2, NFKBIA, NFKBIE, NOS2, OVOL1, PDGFB, PLAU, PLAUR, PTGS2, PTHLH, SAA1, SELE, SERPINE1, SKIL, SMAD4, SNAI2, STAT1, STAT5A, TBX3, TCF7L2, TIMP1, TNF, TNFAIP2, TNFRSF1B, TRAF1, TWIST1, VCAM1, VEGFA, VIM, and ZEB1.

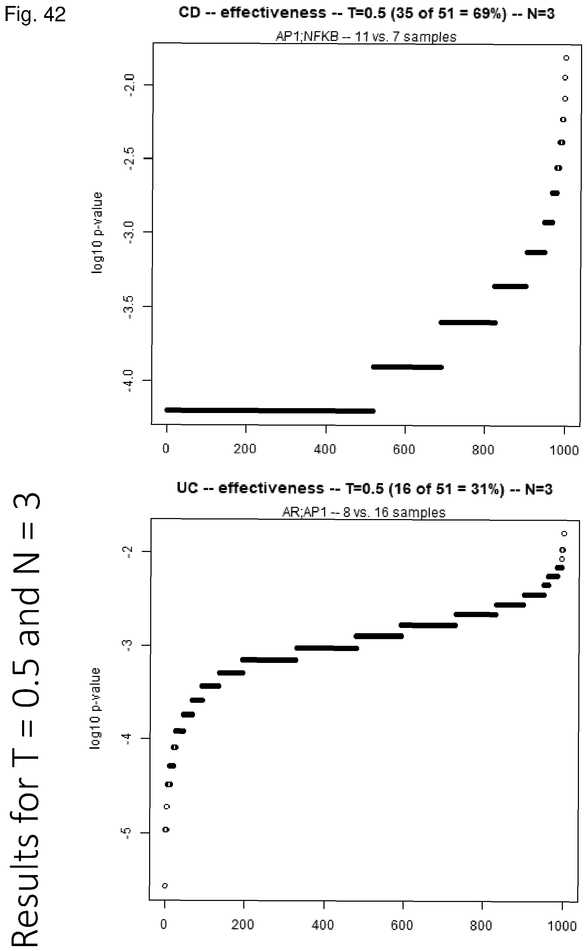

As further indicated in Figs. 42 to 45, by further increasing the threshold of accepting target genes for the curated gene list, the chance that three randomly selected genes are statistically significant for predicting treatment response is even further increased, therefore in a further preferred embodiment the three or more, for example three, four, five, six, seven eight, nine, ten, eleven or twelve or more, genes selected from the group consisting of ANGPTL4, BCL2L1, BIRC3, CCL2, CCL22, CCL3, CCL4, CCL5, CCND1, CD274, CD44, CDKN2B, COL18A1, CTGF, CX3CL1, CXCL1, CXCL2, CXCL3, DKK1, FGF2, FSCN1, GADD45B, GLRX, HIF1A, HSP90B1, ICAM1, IFNG, IL11, IL1B, IL6, IL8, INPP5D, IRF1, JUNB, KIAA1199, MCL1, MMP1, MMP2, MMP3, MMP9, NFKB2, NFKBIA, NFKBIE, PLAU, PLAUR, PTGS2, PTHLH, SELE, SERPINE1, SNAI2, STAT1, STAT5A, TBX3, TIMP1, TNF, TNFAIP2, TNFRSF1B, TRAF1, TWIST1, VCAM1, VIM, ZEB1.

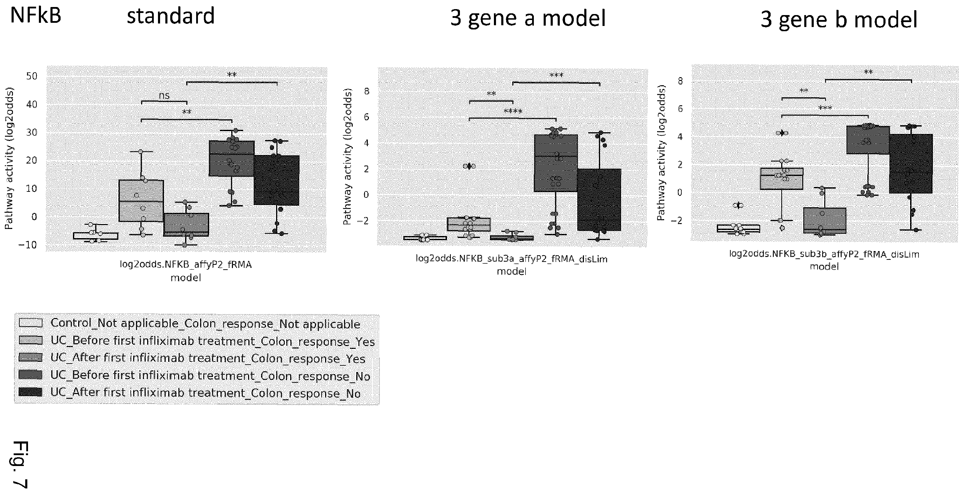

As can be further derived from among others Figs. 7, 11, 15 and 19, the TGFbeta and the NFkB have the strongest correlation with predicting anti-TNFalpha treatment effectiveness based on pre-treatment biopsy, therefore the prediction is preferably based on a selection of the curated TGFbeta or NFkB cellular signaling pathway target genes:
group 1 (curated TGFbeta list) consist of the genes: ANGPTL4, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI2, TIMP1, VEGFA
group 2 (curated NFkB list) consist of the genes: BCL2L1, BIRC3, CCL2, CCL22, CCL3, CCL4, CCL5, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TRAF1, VCAM1; Therefore in an embodiment, the method is preferably based on three or more, for example three, four, five, six, seven eight, nine, ten, eleven or twelve or more, gene selected from the group consisting of, ANGPTL4, BCL2L1, BIRC3, CCL2, CCL22, CCL3, CCL4, CCL5, CDKN2B, CTGF, CX3CL1, CXCL1, CXCL2, CXCL3, GADD45B, HMGA2, ICAM1, IL11, IL1B, IL6, IL8, INPP5D, IRF1, JUNB, MMP2, MMP9, NFKB2, NFKBIA, NFKBIE, OVOL1, PDGFB, PTGS2, PTHLH, SELE, SERPINE1, SKIL, SMAD4, SNAI2, STAT5A,TIMP1, TNF, TNFAIP2, TRAF1, VEGFA and VCAM1, more preferably the method is based on three or more, for example three, four, five, six, seven eight, nine, ten, eleven or twelve or more, gene selected from the group consisting of ANGPTL4, BCL2L1, BIRC3, CCL2, CCL22, CCL3, CCL4, CCL5, CDKN2B, CTGF, CX3CL1, CXCL1, CXCL2, CXCL3, GADD45B, ICAM1, IL11, IL1B, IL6, IL8, INPP5D, IRF1, JUNB, MMP2, MMP9, NFKB2, NFKBIA, NFKBIE, PTGS2, PTHLH, SELE, SERPINE1, SNAI2, STAT5A, TIMP1, TNF, TNFAIP2, TRAF1 and VCAM1.

The conditions for selecting target genes (T value) and number of genes (N values) were further varied to evaluate the conditions that allow for accurate prediction of treatment or treatment effectiveness. These results are set out in Table 15 below. For each condition resulting in 90% or more of the 1000 random selections of N genes having a significant differentiation between responders and non-responders, the representative gene set are listed below in LIST A. Table 15 lists for each UC and CD patient samples (based on dataset GSE16879) prediction (treatment prediction) prediction2 (treatment prediction but only target genes from the NFkB or the TGFbeta pathway are selected) or effectiveness (treatment effectiveness) for T=0.3, 0.4 and 0.5 and N=1, 2, 3 or 6. For each condition, N genes were randomly selected 1000 times and it was reviewed how often the random selection of N genes were statistically significant in distinguishing responders and non-responders to anti-TNFalpha treatment. The results are plotted in Table 15, avgp refers to Average P value (of the 1000 random selections of N genes) and sigper refers to the percentage of the 1000 selections that are significant (P lower or equal to 0.05). For each condition that has at least 90 percent significant (meaning at least 900 out of the 1000 random selections of N genes) the corresponding gene sets are listed in list A.

Therefore, in an embodiment, the method is preferably based on N or more, for example one, two, three, four, five, six, seven eight, nine, ten, eleven or twelve or more, genes selected from a group as listed in LIST A below wherein the application is listed as prediction or prediction2 and N is indicated in LIST A for the respective set, and
wherein an increased expression level of CDKN2B, HMGA2, OVOL1, SMAD4, BCL2L11, HNF1A or TCF7L2, or a decreased expression level of any of the other genes corresponds to a favorable treatment response, and
wherein a decreased expression level of CDKN2B, HMGA2, OVOL1, SMAD4, BCL2L11, HNF1A or TCF7L2, or an increased expression level of any of the other genes corresponds to a not favorable treatment response.

Inflammatory bowel disease (IBD) is the name for a group of conditions that cause the digestive system to become inflamed (red, swollen, and sometimes painful). The most common types of IBD are ulcerative colitis and Crohn's disease. These cause similar symptoms, including diarrhea, rectal bleeding, abdominal pain, fatigue, weight loss and fever. IBD can be debilitating and sometimes leads to life-threatening complications. Crohn's disease affects the small intestine and large intestine, as well as the mouth, esophagus, stomach and the anus, whereas ulcerative colitis primarily affects the colon and the rectum.

When used herein, treatment response refers to an improvement of the grade of the disease when comparing post treatment and pre-treatment grading. Grading systems have been developed for both UC and CD. For UC this has been described in Geboes et al. (Geboes, K et al. "A reproducible grading scale for histological assessment of inflammation in ulcerative colitis." Gut vol. 47,3 (2000): 404-9. doi:10.1136/gut.47.3.404; hereby incorporated by reference in its entirety). Briefly summarized a grade can be assigned for each factor corresponding from grade 0 to 5, with the subgrade indicating the severity, ranging from 0 (no abnormality / not present) to 3 (most severe symptom) as indicated in Table 1 of Geboes et al. (hereby incorporated by reference). Grade 0 scores structural change, grade 1 chronic inflammation infiltrate, grade 2 scores lamina propria neutrophils and eosinophils, grade 3 scores neutrophils in the epithelium grade 4 scores crypt destruction and grade 5 scores erosion or ulceration. Therefore a favorable treatment response as defined herein means that when comparing grades 0-5 prior and after treatment, the subgrades are on average lower after treatment, preferably each subgrade is individually lower or equal after treatment, with the proviso that at least one subgrade is lower after treatment, indicating less severe symptoms. Equally a non-favorable treatment response as defined herein means that when comparing grades 0-5 prior and after treatment, the subgrades are on average equal or higher after treatment, for example each subgrade is individually higher or equal after treatment, indicating equal or more severe symptoms. For CD (D'Haens GR, Geboes K, Peeters M, Baert F, Penninckx F, Rutgeerts P. Early lesions of recurrent Crohn's disease caused by infusion of intestinal contents in excluded ileum. Gastroenterology. 1998 Feb;114(2):262-7. doi: 10.1016/s0016-5085(98)70476-7. PMID: 9453485; hereby incorporated by reference in its entirety), briefly summarized 8 abnormalities are scored a grade 0 to 2 or 0 to 3, with a grade 0 indicating normal / not present and a higher grade indicating more severe symptoms, as indicated in Table 2 of D'Haens et al. 1998 (hereby incorporated by reference). The following abnormalities are scored: epithelial damage, architectural changes, infiltration of mononuclear cells in the lamina propria, infiltration of polymorphonuclear cell in the lamina propria, polymorphonuclear cell in the epithelium, presence of erosion and/or ulcers, presence of granuloma, and number of biopsy specimens affected. Therefore a favorable treatment response as defined herein means that when comparing the grades prior and after treatment, the grades are on average lower after treatment, preferably each grade is individually lower or equal after treatment with the proviso that at least one grade is lower, indicating less severe symptoms. Equally a non-favorable treatment response as defined herein means that when comparing the grades prior and after treatment, the grades are on average equal or higher after treatment, for example each grade is individually equal or higher after treatment, indicating equal or more severe symptoms.

When used herein, subject refers to a human or non-human animal. Preferably the subject is suffering from, diagnosed with, suspected to have, undergoing treatment for or has been cured from an inflammatory bowel disease (IBD) such as Ulcerative Colitis (UC) or Crohn's Disease (CD), unless the subject is a healthy control subject. The subject may also be referred herein as patient.

When used herein, the terms anti-TNFalpha compound or anti-TNFalpha therapy or anti-TNFalpha treatment are used interchangeably, and refer to a compound having an inhibitory effect on TNFalpha signaling, or it may refer to administration of said compound to a subject as defined herein, depending on the context. Compounds having an inhibitory effect on TNF-alpha signaling may exert their effect in the ligand (TNFalpha), e.g. by preventing the ligand to bind to its receptor. Alternatively the compounds may exert their effect directly on the receptor or downstream of the receptor by preventing the receptor or downstream signaling pathways to be activated. Compounds having an inhibitory effect on TNF-alpha signaling are known to the skilled person and may also be referred to as TNFalpha blockers or anti-TNFalpha drugs. Anti-TNFalpha compounds may for example be inhibitory antibodies, small molecule inhibitors or nucleotide based inhibitors such as RNAi, miRNA, however other inhibitors may be known to the person skilled in the art. Non limiting examples of anti-TNFalpha compounds are antibody based compounds such as Adalimumab, Certolizumab, Etanercept, Golimumab, Infliximab, or a biosimilars or chimeric products of any of the preceding compounds, such as for example the CT-P13 Infliximab biosimilar, or small molecule based compound such as thalidomide, lenalidomide, pomalidomide, a xanthane derivative such as pentoxifylline, bupropion, an 5-HT_{2A} agonist such as (R)DOI, TCB2, LSD and LA-SS-Az; or a natural compound such as curcumin, catechins, cannabidiol or Echinacea purpurea.

When used herein, "expression level" refers to quantifying the number of mRNA copies transcribed from a gene. Generally this number will not be an absolute value but a relative value, and therefore is preferably normalized for example in reference to the expression of one or more housekeeping genes. Housekeeping genes are genes which are assumed to have constant expression levels independent of cell type and/or functional status of the cell (i.e. from a diseased or healthy subject), and therefore can be used to normalize experimentally determined relative expression levels. Housekeeping genes are generally known to the skilled person, non-limiting examples of housekeeping genes that may be used for normalization are beta-actin, glyceraldehyde-3-phosphate dehydrogenase (GAPDH) and Transcription factor IID TATA binding protein (TBP).

Therefore the phrase "expression level of a target gene" denotes a value that is representative of the amount, e.g. a concentration, of a target gene present in a sample. This value may be based on the amount of a transcription product of the target gene (e.g. mRNA) or a translation product thereof (e.g. protein). Preferably, the expression level is based on the amount of mRNA formed from the target gene. In order to determine the expression level, techniques such as qPCR, multiple qPCR, multiplexed qPCR, ddPCR, RNAseq, RNA expression array or mass spectrometry may be used. For example, a gene expression microarray, e.g. Affymetrix microarray, or RNA sequencing methods, like an Illumina sequencer, can be used.

When used herein, the term "sample" also encompasses the case where e.g. a tissue of the subject have been taken from the subject and, e.g., have been put on a microscope slide, and where for performing the claimed method a portion of this sample is extracted, e.g., by means of Laser Capture Microdissection (LCM), or by scraping off the cells of interest from the slide, or by fluorescence-activated cell sorting techniques. Preferably the sample is obtained form or is a biopsy. The biopsy or sample may be obtained from a healthy subject (e.g. to serve as a reference sample) or from a subject suffering from IBD. Preferably the sample is a gastric, colon, intestinal or rectal sample, therefore in a preferred embodiment the biopsy used to provide the sample is a gastric, colon, intestinal or rectal biopsy.

The terms "healthy subject" or "healthy reference subject" or "healthy control subject" are used interchangeably, and when used herein refer to a subject not having IBD.

Alternatively, the predictions referred herein may be based on inferred cellular signaling pathway activity. Therefore in an alternative of the first aspect the invention relates to a method for predicting the treatment response of a subject suffering from inflammatory bowel disease (IBD) to treatment with an anti-TNFalpha compound based on inferred cellular signaling pathway activity or activities in a sample obtained from the subject, the method comprising:
comparing the inferred cellular signaling pathway activity, or when multiple cellular signaling pathway activities are used the combined cellular signaling pathway activities, with a threshold value;
wherein the cellular signaling pathway is one or more cellular signaling pathway selected from TGFbeta, NFkB, MAPK-AP1, STAT3 and WNT, preferably wherein the cellular signaling pathway is one or more cellular signaling pathway selected from TGFbeta and NFkB;
wherein the cellular signaling pathway activity or activities are inferred by
determining an activity level of a transcription factor (TF) element of the respective cellular signaling pathway in the sample obtained from the subject, the TF element controlling transcription of three or more target genes of the respective cellular signaling pathway, the determining being based on evaluating a calibrated mathematical pathway model relating the expression levels of the three or more target genes of the respective cellular signaling pathway to the activity level of the TF element of the cellular signaling pathway, and
inferring the activity of the cellular signaling pathway in the subject based on the determined activity level of the TF element of the respective cellular signaling pathway in the sample of the subject;
wherein the treatment response is predicted to be favorable when the inferred cellular signaling pathway activity or the combined cellular signaling pathway activities are below the threshold value, and wherein the treatment response is predicted to be unfavorable when the inferred cellular signaling pathway activity or the combined cellular signaling pathway activities are above the threshold value.

In an embodiment the activity of the respective cellular signaling pathway being defined by the activity level of the respective TF elements. In an embodiment the calibrated mathematical pathway model being a model that is calibrated using a ground truth dataset including samples in which transcription of the three or more target genes is induced by the respective TF elements and samples in which transcription of the three or more target genes is not induced by the respective TF elements. The TF for the relevant cellular signaling pathways described herein are listed below.

In an embodiment the IBD is ulcerative colitis (UC) or Crohn Disease (CD). In an embodiment the threshold value is independently determined in one or more reference samples. In an embodiment the three or more, e.g. three, four, five, six, seven, eight, nine, ten, eleven or twelve or more, target genes of the TGFbeta cellular signaling pathway are selected from ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKNIA, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKNIA, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7; the three or more, e.g. three, four, five, six, seven, eight, nine, ten, eleven or twelve or more, target genes of the NFkB cellular signaling pathway are selected from BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKB IE, PTGS2, SELE, STAT5A, TNF, TNFAIP2, TNIP1, TRAF1, and VCAM1; the three or more, e.g. three, four, five, six, seven, eight, nine, ten, eleven or twelve or more, target genes of the MAPK-AP1 cellular signaling pathway are selected from BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably, from the group consisting of: CCND1, EGFR, EZR, GLRX, MMP1, MMP3, PLAU, PLAUR, SERPINE1, SNCG, and TIMP1; the three or more, e.g. three, four, five, six, seven, eight, nine, ten, eleven or twelve or more, target genes of the STAT3 cellular signaling pathway are selected from AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JUNB, MCL1, MMP1, MMP3, MMP9, MUC1, MYC, NOS2, POU2F1, PTGS2, SAA1, STAT1, TIMP1, TNFRSF1B, TWIST1, VIM, and ZEB1, preferably, either from the group consisting of: BCL2L1, BIRC5, CCND1, CD274, FOS, HIF1A, HSP90AA1 , HSP90AB1 , MMP1 , and MYC, or from the group consisting of: BCL2L1, CD274, FOS, HSP90B1, HSPA1B, ICAM1, IFNG, JUNB, PTGS2, STAT1, TNFRSF1B, and ZEB1; the three or more, e.g. three, four, five, six, seven, eight, nine, ten, eleven or twelve or more, target genes of the WNT cellular signaling pathway are selected from KIAA1199, AXIN2, RNF43, TBX3, TDGF1, SOX9, ASCL2, IL8, SP5, ZNRF3, KLF6, CCND1, DEFA6 and FZD7, optionally further at least one target gene, e.g. one, two three, four, five six, seven, eight, nine, ten or more target gene(s), selected from the group comprising or consisting of: NKD1, OAT, FAT1, LEF1, GLUL, REG1B, TCF7L2, COL18A1, BMP7, SLC1A2, ADRA2C, PPARG, DKK1, HNF1A and LECT2. In an embodiment the sample obtained from the subject is a gastric, colon, intestinal or rectal sample.

By using a calibrated mathematical model to relate the gene expression levels to a cellular signaling pathway activity, a numerical value can be assigned to the pathway activity. Depending on the model, this value can for example be normalize to result in a value from 0 to 100, where 0 is no pathway activity and 100 is theoretical maximum pathway activity. Alternatively the value may be normalized such that the average value is 0 and thus decreased pathway activity is represented by a negative value and increased pathway activity is represented by a positive value. It is understood that the values obtained using such model are dependent on the model used, and do not represent absolute values. Therefore, the same model should be used for calibrating, determining reference values and when used in the method of the invention, so that it allows comparison of the obtained numerical values for pathway activity.

Therefore, the numerical value obtained for a pathway activity in a sample for a specific state (e.g. responder to treatment with an anti-TNF-alpha compound) may be compared to the numerical value obtained for that pathway activity in a reference sample (e.g. non-responder to treatment with an anti-TNF-alpha compound). By performing such comparison a statement can be made about the pathway activity in the sample (e.g. a sample from a biopsy from a subject with IBD) relative to the pathway activity determined in the reference sample (e.g. a sample from a biopsy). Based on the numerical value a statement can be made about the pathway activity in the sample with respect to the pathway activity in the reference sample, e.g. whether the pathway activity in the sample is higher or lower, in correspondence with the numerical value obtained for the pathway activity. E.g. cellular signaling pathway activity can be determined in a biopsy sample from a subject not responding to treatment with an anti-TNF-alpha compound. Since it is determined by the inventors that the TGFbeta, NFkB, MAPK-AP1, STAT3 and WNT cellular signaling pathway activities in non-responders to anti-TNF-alpha treatment are higher (in a sample from a biopsy obtained pre-treatment), these can bet set as a baseline numerical values representing an high pathway activities, i.e. the reference value. Similarly baseline numerical values can be set for low activities for these pathways representing the lower value obtained from samples form biopsies obtained from responders to anti-TNFalpha treatment. By determining the numerical value for one of the TGFbeta, NFkB, MAPK-AP1, STAT3 or WNT cellular signaling pathway activity in the sample from a biopsy of a subject with IBD, the numerical value representing that pathway activity can be compared with the reference values, and it can be determined whether the pathway activity is high or low, by comparing with the references based on the numerical values. The comparison may be made with multiple reference samples to allow for more accurate results and statistics to be performed.

Alternatively a reference sample can be used to set a baseline pathway activity, allowing further comparison of the pathway activity. For example, the average and standard deviation can be calculated which can be used to calculate values for a healthy individual, and define a threshold for abnormal pathway activity. Alternatively a reference can be used with a known state (e.g. severe infection) and used in the comparison of the pathway activity/activities.

Therefore, in a preferred embodiment the pathway activity is determined to be higher or lower when the obtained numerical value for the pathway activity differs with at least one standard deviation from the numerical value obtained for the pathway activity in the reference sample. When the value is within the range of one standard deviation it is said to be equal or comparable to the pathway activity in the reference sample. It is understood that the threshold may also be set higher, for example 2 times the standard deviation or even 3 times the standard deviation. It is further understood that the threshold may be determined using alternative ways, e.g. statistical methods, to determine a significant deviation from the reference value.

It is understood that the reference value for a certain pathway activity in a sample in principle only needs to be determined once. Therefore, the step of determining a reference pathway activity is not part of the methods of the invention, as a predetermined reference pathway activity can be used.

For the purpose of the invention determining the expression levels of the target genes based on the extracted RNA may be a part of the method, meaning the method includes the step of determining the expression levels of the target genes on RNA extracted from the sample obtained from the patient using methods known to the skilled person or described herein. The method may further include the step of obtaining a sample form the patient in order to extract the RNA. Alternatively the expression levels may have been determined separately and the demining step (of the expression levels of the target genes) is not an active step in the method of the invention. In such case the expression levels are provided as an input value, e.g. a relative expression level in reference to one or more control gene expression levels.

Sets of cellular signaling pathway target genes whose expression levels are preferably analyzed have been identified, alternatively methods for identifying suitable target genes are described herein. For use to determine pathway activity, for example by a mathematical model, three or more, for example, three, four, five, six, seven, eight, nine, ten, eleven, twelve or more, target genes from each assessed cellular signaling pathway can be analyzed to determine pathway activities.

In an embodiment the signaling pathway measurements are performed using qPCR, multiple qPCR, multiplexed qPCR, ddPCR, RNAseq, RNA expression array or mass spectrometry. For example, a gene expression microarray data, e.g. Affymetrix microarray, or RNA sequencing methods, like an Illumina sequencer, can be used.

The terms "pathway", "signal transduction pathway", "signaling pathway" and "cellular signaling pathway" are used interchangeably herein.

An "activity of a signaling pathway" may refer to the activity of a signaling pathway associated transcription factor (TF) element in the sample, the TF element controlling transcription of target genes, in driving the target genes to expression, i.e., the speed by which the target genes are transcribed, e.g. in terms of high activity (i.e. high speed) or low activity (i.e. low speed), or other dimensions, such as levels, values or the like related to such activity (e.g. speed). Accordingly, for the purposes of the present invention, the term "activity", as used herein, is also meant to refer to an activity level that may be obtained as an intermediate result during "pathway analysis" as described herein.

The term "transcription factor element" (TF element), as used herein, preferably refers to an intermediate or precursor protein or protein complex of the active transcription factor, or an active transcription factor protein or protein complex which controls the specified target gene expression. For example, the protein complex may contain at least the intracellular domain of one of the respective signaling pathway proteins, with one or more co-factors, thereby controlling transcription of target genes. Preferably, the term refers to either a protein or protein complex transcriptional factor triggered by the cleavage of one of the respective signaling pathway proteins resulting in a intracellular domain.

The term "target gene", as used herein, means a gene whose transcription is directly or indirectly controlled by a respective transcription factor element. The "target gene" may be a "direct target gene" and/or an "indirect target gene" (as described herein).

Pathway analysis enables quantitative measurement of signal transduction pathway activity in cells, e.g. of a sample, based on inferring activity of a signal transduction pathway from measurements of mRNA levels of the well-validated direct target genes of the transcription factor associated with the respective signaling pathway (see for example W Verhaegh et al., 2014, supra; W Verhaegh, A van de Stolpe, Oncotarget, 2014, 5(14):5196).

Preferably the determining of the activity or activities of the signaling pathway(s), the combination of multiple pathway activities and applications thereof is performed as described for example in the following documents, each of which is hereby incorporated in its entirety for the purposes of determining activity of the respective signaling pathway: published international patent applications WO2013011479 (titled "ASSESSMENT OF CELLULAR SIGNALING PATHWAY ACTIVITY USING PROBABILISTIC MODELING OF TARGET GENE EXPRESSION"), WO2014102668 (titled "ASSESSMENT OF CELLULAR SIGNALING PATHWAY ACTIVITY USING LINEAR COMBINATION(S) OF TARGET GENE EXPRESSIONS"), WO2015101635 (titled "ASSESSMENT OF THE PI3K CELLULAR SIGNALING PATHWAY ACTIVITY USING MATHEMATICAL MODELLING OF TARGET GENE EXPRESSION"), WO2016062891 (titled "ASSESSMENT OF TGF-β CELLULAR SIGNALING PATHWAY ACTIVITY USING MATHEMATICAL MODELLING OF TARGET GENE EXPRESSION"), WO2017029215 (titled "ASSESSMENT OF NFKB CELLULAR SIGNALING PATHWAY ACTIVITY USING MATHEMATICAL MODELLING OF TARGET GENE EXPRESSION"), WO2014174003 (titled "MEDICAL PROGNOSIS AND PREDICTION OF TREATMENT RESPONSE USING MULTIPLE CELLULAR SIGNALLING PATHWAY ACTIVITIES"), WO2016062892 (titled "MEDICAL PROGNOSIS AND PREDICTION OF TREATMENT RESPONSE USING MULTIPLE CELLULAR SIGNALING PATHWAY ACTIVITIES"), WO2016062893 (titled "MEDICAL PROGNOSIS AND PREDICTION OF TREATMENT RESPONSE USING MULTIPLE CELLULAR SIGNALING PATHWAY ACTIVITIES"), WO2018096076 (titled "Method to distinguish tumor suppressive FOXO activity from oxidative stress"), and in the patent applications WO2018096076 (titled "Method to distinguish tumor suppressive FOXO activity from oxidative stress"), WO2019068585 (titled "Assessment of Notch cellular signaling pathway activity using mathematical modelling of target gene expression"), WO2019120658 (titled "Assessment of MAPK-MAPK-AP1 cellular signaling pathway activity using mathematical modelling of target gene expression"), WO2019068543 (titled "Assessment of JAK-JAK-STAT3 cellular signaling pathway activity using mathematical modelling of target gene expression"), WO2019068562 (titled "Assessment of JAK-STAT1/2 cellular signaling pathway activity using mathematical modelling of target gene expression"), and WO2019068623 (titled "Determining functional status of immune cells types and immune response").

The models have been biologically validated for ER, AR, PI3K-FOXO, HH, Notch, TGF-β, Wnt, NFkB, JAK-STAT1/2, JAK-JAK-STAT3 and MAPK-MAPK-AP1 pathways on several cell types.

Unique sets of cellular signaling pathway target genes whose expression levels are preferably analyzed have been identified. For use in the mathematical models, three or more, for example, three, four, five, six, seven, eight, nine, ten, eleven, twelve or more, target genes from each assessed cellular signaling pathway can be analyzed to determine pathway activities.

Common to the pathway analysis methods for determining the activities of the different signaling pathways as disclosed herein is a concept, which is preferably applied herein for the purposes of the present invention, wherein the activity of a signaling pathway in a cell such as a cell present in a sample is determinable by receiving expression levels of one or more, preferably three or more, target genes of the signaling pathway, determining an activity level of a signaling pathway associated transcription factor (TF) element in the sample, the TF element controlling transcription of the three or more target genes, the determining being based on evaluating a calibrated mathematical pathway model relating expression levels of the one or more, preferably three or more target genes to the activity level of the signaling pathway, and optionally inferring the activity of the signaling pathway in the cell present in a sample based on the determined activity level of the signaling pathway associated TF element. As described herein, the activity level can be directly used as an input to determine the immune response in a subject and/or determine whether an infection is viral and/or determine the severity of a viral infection and/or determine the cellular immunity conferred by a vaccine, which is also contemplated by the present invention.

The term "activity level" of a TF element, as used herein, denotes the level of activity of the TF element regarding transcription of its target genes.

The calibrated mathematical pathway model may be a probabilistic model, preferably a Bayesian network model, based on conditional probabilities relating the activity level of the signaling pathway associated TF element and the expression levels of the three or more target genes, or the calibrated mathematical pathway model may be based on one or more linear combination(s) of the expression levels of the three or more target genes. For the purposes of the present invention, the calibrated mathematical pathway model is preferably a linear model, or a Bayesian network model based on conditional probabilities.

In particular, the determination of the expression level and optionally the inferring of the activity of a signaling pathway in the subject may be performed, for example, by inter alia (i) evaluating a portion of a calibrated probabilistic pathway model, preferably a Bayesian network, representing the cellular signaling pathways for a set of inputs including the expression levels of the three or more target genes of the cellular signaling pathway measured in a sample of the subject, (ii) estimating an activity level in the subject of a signaling pathway associated transcription factor (TF) element, the signaling pathway associated TF element controlling transcription of the three or more target genes of the cellular signaling pathway, the estimating being based on conditional probabilities relating the activity level of the signaling pathway associated TF element and the expression levels of the three or more target genes of the cellular signaling pathway measured in the sample of the subject, and optionally (iii) inferring the activity of the cellular signaling pathway based on the estimated activity level of the signaling pathway associated TF element in the sample of the subject. This is described in detail in the published international patent application WO 2013/011479 A2 ("Assessment of cellular signaling pathway activity using probabilistic modeling of target gene expression"), the contents of which are herewith incorporated in their entirety.

In an exemplary alternative, the determination of the expression level and optionally the inferring of the activity of a cellular signaling pathway in the subject may be performed by inter alia (i) determining an activity level of a signaling pathway associated transcription factor (TF) element in the sample of the subject, the signaling pathway associated TF element controlling transcription of the three or more target genes of the cellular signaling pathway, the determining being based on evaluating a calibrated mathematical pathway model relating expression levels of the three or more target genes of the cellular signaling pathway to the activity level of the signaling pathway associated TF element, the mathematical pathway model being based on one or more linear combination(s) of expression levels of the three or more target genes, and optionally (ii) inferring the activity of the cellular signaling pathway in the subject based on the determined activity level of the signaling pathway associated TF element in the sample of the subject. This is described in detail in the published international patent application WO 2014/102668 A2 ("Assessment of cellular signaling pathway activity using linear combination(s) of target gene expressions").

Further details regarding the inferring of cellular signaling pathway activity using mathematical modeling of target gene expression can be found in W Verhaegh et al., "Selection of personalized patient therapy through the use of knowledge-based computational models that identify tumor-driving signal transduction pathways", Cancer Research, Vol. 74, No. 11, 2014, pages 2936 to 2945.

To facilitate rapid identification of references, the above-mentioned references have been assigned to each signaling pathway of interest here and exemplarily corresponding target genes suitable for determination of the signaling pathway's activity have been indicated.

Particularly preferred is a method wherein the inferring comprises:
inferring activity of a Wnt cellular signaling pathway in the sample based at least on expression levels of three or more, e.g. three, four five six seven, eight, nine, ten eleven, twelve or thirteen or more, target gene(s) of the Wnt pathway measured in the sample selected from the group comprising or consisting of: KIAA1199, AXIN2, RNF43, TBX3, TDGF1, SOX9, ASCL2, IL8, SP5, ZNRF3, KLF6, CCND1, DEFA6 and FZD7, optionally the inferring is further based on expression levels of at least one target gene, e.g. one, two three, four, five six, seven, eight, nine, ten or more target gene(s), of the Wnt pathway measured in the sample selected from the group comprising or consisting of: NKD1, OAT, FAT1, LEF1, GLUL, REG1B, TCF7L2, COL18A1, BMP7, SLC1A2, ADRA2C, PPARG, DKK1, HNF1A and LECT2;
inferring activity of a AR cellular signaling pathway in the sample based at least on expression levels of three or more, e.g. three, four five six seven, eight, nine, ten eleven, twelve or thirteen, target gene(s) of the AR pathway measured in the sample selected from the group comprising or consisting of: KLK2, PMEPA1, TMPRSS2, NKX3_1, ABCC4, KLK3, FKBP5, ELL2, UGT2B15, DHCR24, PPAP2A, NDRG1, LRIG1, CREB3L4, LCP1, GUCY1A3, AR and EAF2, optionally the inferring is further based on expression levels of at least one target gene, e.g. one, two, three, four, five, six, seven, eight, nine, ten eleven twelve or more target gene(s), of the AR pathway measured in the sample selected from the group comprising or consisting of: APP, NTS, PLAU, CDKN1A, DRG1, FGF8, IGF1, PRKACB, PTPN1, SGK1 and TACC2;
inferring activity of a TGFbeta cellular signaling pathway in the sample based at least on expression levels of three or more, e.g. three, four five six seven, eight, nine, ten eleven, twelve or thirteen, target gene(s) of the TGFbeta pathway measured in the sample selected from the group comprising or consisting of: ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKNIA, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKNIA, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7;
inferring activity of a NFkB cellular signaling pathway in the sample based at least on expression levels of three or more, e.g. three, four five six seven, eight, nine, ten eleven, twelve or thirteen, target gene(s) of the NFkB pathway measured in the sample selected from the group comprising or consisting of: BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKB IE, PTGS2, SELE, STAT5A, TNF, TNFAIP2, TNIP1, TRAF1, and VCAM1;
inferring activity of a JAK-STAT1/2 cellular signaling pathway in the sample based at least on expression levels of three or more, e.g. three, four five six seven, eight, nine, ten eleven, twelve or thirteen, target gene(s) of the JAK-STAT1/2 pathway measured in the sample selected from the group comprising or consisting of: BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFDIL, USP18, and ZNRF3, preferably, from the group consisting of: IRF1, IRF7, IRF8, IRF9, OAS1, PDCD1, ST13, STAT1, and USP18.
inferring activity of a JAK-STAT3 cellular signaling pathway in the sample based at least on expression levels of three or more, e.g. three, four five six seven, eight, nine, ten eleven, twelve or thirteen, target gene(s) of the JAK-STAT3 pathway measured in the sample selected from the group comprising or consisting of: AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JUNB, MCL1, MMP1, MMP3, MMP9, MUC1, MYC, NOS2, POU2F1, PTGS2, SAA1, STAT1, TIMP1, TNFRSF1B, TWIST1, VIM, and ZEB1, preferably, either from the group consisting of: BCL2L1, BIRC5, CCND1, CD274, FOS, HIF1A, HSP90AA1 , HSP90AB1 , MMP1 , and MYC, or from the group consisting of: BCL2L1, CD274, FOS, HSP90B1, HSPA1B, ICAM1, IFNG, JUNB, PTGS2, STAT1, TNFRSF1B, and ZEB1;
inferring activity of a MAPK-AP1 cellular signaling pathway in the sample based at least on expression levels of three or more, e.g. three, four five six seven, eight, nine, ten eleven, twelve or thirteen, target gene(s) of the MAPK-AP1 pathway measured in the sample selected from the group comprising or consisting of: BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably, from the group consisting of: CCND1, EGFR, EZR, GLRX, MMP1, MMP3, PLAU, PLAUR, SERPINE1, SNCG, and TIMP1;

Herein, a Wnt transcription factor (TF) element is defined to be a protein complex containing at least one of the TCF/LEF TF family members, i.e., TCF1, TCF3, TCF4 or LEF1, preferably wherein the Wnt TF element comprises beta-catenin/TCF4, which is capable of binding to specific DNA sequences, thereby controlling transcription of target genes.

Herein, an AR transcription factor (TF) element is defined to be a protein complex containing at least one or preferably a dimer of nuclear Androgen receptor.

Herein, the term "TGFbeta transcription factor element" or "TGFbeta TF element" or "TF element" when referring to the TGFbeta pathway is defined to be a protein complex containing at least one or, preferably, a dimer of the TGFbeta members (SMAD1 , SMAD2, SMAD3 , SMAD5 and SMAD8 with SMAD4) or a trimer (two proteins from SMAD1, SMAD2, SMAD3, SMAD5 and SMAD8 with SMAD4), which is capable of binding to specific DNA sequences, thereby controlling transcription of target genes. Preferably, the term refers to either a protein or protein complex transcriptional factor triggered by the binding of TGFbeta to its receptor or an intermediate downstream signaling agent between the binding of TGFbeta to its receptor and the final transcriptional factor protein or protein complex. For example, it is known that TGFbeta binds to an extracellular TGFbeta receptor that initiates an intracellular "SMAD" signaling pathway and that one or more SMAD proteins (receptor-regulated or R-SMADs (SMAD1, SMAD2, SMAD 3, SMAD5 and SMAD8) and SMAD4) participate in, and may form a hetero-complex which participates in, the TGFbeta transcription signaling cascade which controls expression.

Herein, an NFkB transcription factor (TF) element is defined to be a protein complex containing at least one or, preferably, a dimer of the NFkB members (NFKB 1 or p50/p105, NFKB2 or p52/p100, RELA or p65, REL, and RELB), which is capable of binding to specific DNA sequences, thereby controlling transcription of target genes.

Herein, the term "JAK-STAT1/2 transcription factor element" or "JAK-STAT1/2 TF element" or "TF element" when referring to the JAK-STAT1/2 is defined to be a protein complex containing at least a STAT1-STAT2 heterodimer or a STAT1 homodimer, which is capable of binding to specific DNA sequences, preferably the ISRE (binding motif AGTTTC NTTCNC/T) or GAS (binding motif TTC/A NNG/TAA) response elements, respectively, thereby controlling transcription of target genes. Preferably, the term refers to either a protein or protein complex transcriptional factor that is formed by different stimuli such as IFNs triggered by the binding of the stimulating ligand to its receptor resulting in downstream signaling.

Herein, the term "JAK-STAT3 transcription factor element" or "JAK-STAT3 TF element" or "TF element" when referring to the JAK-STAT3 pathway is defined to be a protein complex containing at least a STAT3 homodimer, which is capable of binding to specific DNA sequences, preferably the response elements with binding motif CTGGGAA, thereby controlling transcription of target genes. Preferably, the term refers to either a protein or protein complex transcriptional factor triggered by the binding of STAT3 inducing ligands such as interleukin-6 (IL-6) and IL-6 family cytokines to its receptor or an intermediate downstream signaling agent between the binding the ligand to its receptor and the final transcriptional factor protein or protein complex.

Herein, the term "AP-1 transcription factor element" or "AP-1 TF element" or "TF element" when referring to the MAPK-AP1 pathway is defined to be a protein complex containing at least a member of the Jun (e.g. c-Jun, JunB and JunD) family and/or a member of the Fos (e.g. c-Fos, FosB, Fra-1 and Fra-2) family and/or a member of the ATF family and/or a member of the JDP family, forming e.g. Jun∼Jun or Jun∼Fos dimers, capable of binding to specific DNA sequences, preferably the response elements 12-0-Tetradecanoylphorbol-13-acetate (TPA) response element (TRE) with binding motif 5'-TGA G/C TCA-3' or cyclic AMP response element (CRE) with binding motif 5'-TGACGTCA-3', thereby controlling transcription of target genes. Preferably, the term refers to either a protein or protein complex transcriptional factor triggered by the binding of AP-1 inducing ligands, such as growth factors (e.g., EGF) and cytokines, to its receptor or an intermediate downstream signaling agent, or triggered by the presence of an AP-1 -activating mutation.

In an embodiment, the three or more, e.g. three, four, five, six seven, eight, nine, ten, eleven or twelve or more, expression levels are combined by adding up their relative expression levels, with the proviso that the expression levels of the genes CDKN2B, HMGA2, OVOL1, SMAD4, BCL2L11, HNF1A or TCF7L2 are multiplied with a weight factor of -1. Preferably wherein the combined expression levels are compared to a threshold value to determine whether the treatment response is favorable or not favorable, wherein the treatment response is predicted to be favorable when the combined three or more expression levels are below the threshold value, and wherein the treatment response is predicted to be unfavorable when the combined three or more expression levels are above the threshold value.

When used herein, relative expression level refers to the quantitative value obtained for the expression level of the gene which has been normalized, e.g. by using a housekeeping gene or genes, to correct for sample variation.

A threshold value for the combined expression levels can be set from reference samples with a known status, meaning reference samples obtained from IBD patient pre-treatment from which it is known whether they are responders or non-responders. Since the combined expression values represents a set of numerical values, an average can be calculated for a specific set of genes (i.e. three or more genes selected form the list identified above) and for a specific status (i.e. responder or non-responder). Based on these values a threshold can be set. For example the threshold can be set as the median between responders and non-responders, or can be set such that a predetermined percentage of responders or non-responders fall above or below the set value. Such methods are known to the skilled artisan. Setting such threshold value for a specific set of genes allows for easy comparison of the value obtained a sample form a subject suffering from IBD, and quickly diagnosing whether the subject would benefit from anti-TNF-alpha treatment. It is particularly preferred to combine the three or more expression levels by adding up the logarithmic values of their relative expression levels, preferably the log2 values.

In an embodiment the IBD is Ulcerative Colitis (UC) or Crohn Disease (CD). As can be derived particularly from figs. 5 to 20, the target genes selected from the TGFbeta, NFkB, MAPK-AP1, STAT3 and WNT cellular signaling pathways are predictive for both subjects suffering from UC and CD.

For patients for whom no pre-treatment sample or biopsy is available to predict the treatment response and who are currently undergoing treatment with an anti-TNF-alpha compound, it may be desirable to determine the effectiveness of the treatment. Alternatively, if a treatment prediction was made and this was followed up with treatment with an anti-TNFalpha compound in the subject, it may still be desirable to follow up this treatment to see if it is effective or not and thus whether treatment should be continued. Therefore, in a second aspect, the invention relates to a method for determining the effectiveness of a treatment of a subject suffering from IBD with an anti-TNFalpha compound based on the expression levels of two or more genes,
wherein if the IBD is UC, the two or more, e.g. two, three, four, five, six, seven, eight, nine, ten, eleven, twelve or more, genes are selected from the group consisting of BCL2L11, CDKN1A, DNMT1, EAF2, ELL2, ENPP2, FKBP5, GLRX, LCP1, MMP1, MMP3, MMP9, PLAU, PLAUR, PMEPA1, PRKACB, PTGS2, SERPINE1, SGK1, TIMP1, TMPRSS2 and VIM; or
wherein the IBD is CD, the two or more, e.g. two, three, four, five, six, seven, eight, nine, ten, eleven, twelve or more, genes are selected from the group consisting of BCL2L1, BCL2L11, BIRC3, CCL2, CCL22, CCL3, CCL4, CCL5, CX3CL1, CXCL1, CXCL2, CXCL3, DNMT1, ENPP2, GLRX, ICAM1, IL1B, IL6, IL8, IRF1, MMP1, MMP3, MMP9, NFKB2, NFKBIA, NFKBIE, PLAU, PLAUR, PTGS2, SELE, SERPINE1, STAT5A, TIMP1, TNF, TNFAIP2, TRAF1, VCAM1 and VIM;
wherein the three or more expression levels are used to determine whether the treatment of IBD in the patient with an anti-TNFalpha compound is effective or not effective,
wherein a decreased expression level of BCL2L11 or PRKACB or an increased expression level of any of the other genes corresponds to a not effective treatment, and
wherein an increased expression level of BCL2L11 or PRKACB or a decreased expression level of any of the other genes corresponds to an effective treatment.

As can be derived from figs. 5 to 20, the AR and MAPK-AP1 (for UC) or MAPK-AP1 and NFkB (for CD) cellular signaling pathway activities in post treatment patient samples undergoing treatment with an anti-TNFalpha compound are indicative if a subject is responding well to treatment with an anti-TNFalpha compound even when no pre-treatment information is available. Therefore the curated lists for these pathways can be used to predict treatment effectiveness. The listed target genes have been selected in the same manner as for treatment response, based on pathways with significant different pathway activity between states (effective treatment vs non-effective treatment), where the pathway genes are curated based on the genes contributing most to the pathway score. For treatment effectiveness predictions, different pathways were found to be significant when comparing UC and CD patients, so distinct target genes groups are selected depending on the exact diagnosis of the IBD (being UC or CD).

When used herein, treatment effectiveness refers to whether a subject having IBD responds favorably to treatment with an anti-TNFalpha compound as determined after onset of the treatment. Favorably can be defined herein as an improvement in the overall grade using the grading systems for UC and CD referred above.

In a preferred embodiment wherein if the IBD is UC and the two or more genes are selected from the group consisting of EAF2, ELL2, FKBP5, GLRX, LCP1, MMP1, MMP3, MMP9, PLAU, PLAUR, PMEPA1, PTGS2, SERPINE1, TIMP1, TMPRSS2 and VIM.

In a preferred embodiment wherein if the IBD is CD and the two or more genes are selected from the group consisting of BCL2L1, BIRC3, CCL2, CCL22, CCL3, CCL4, CCL5, CX3CL1, CXCL1, CXCL2, CXCL3, GLRX, ICAM1, IL1B, IL6, IL8, IRF1, MMP1, MMP3, MMP9, NFKB2, NFKBIA, NFKBIE, PLAU, PLAUR, PTGS2, SELE, SERPINE1, STAT5A, TIMP1, TNF, TNFAIP2, TRAF1, VCAM1 and VIM.

In an embodiment the two or more expression levels are combined by adding up their relative expression levels, with the proviso that the expression levels of the genes BCL2L11 or PRKACB are multiplied with a weight factor of -1,
preferably wherein the combined expression levels are compared to a threshold value to determine whether the treatment is effective or not effective, wherein the treatment is predicted to be effective when the combined two or more expression levels are below the threshold value, and wherein the treatment is predicted not to be effective when the combined two or more expression levels are above the threshold value.

Alternatively, the method of the second aspect of the invention relates to a method for determining the effectiveness of a treatment of a subject suffering from IBD with an anti-TNFalpha compound based on the expression levels of two or more genes, wherein the expression levels of the three or more genes are selected from one or more groups,
wherein if the IBD is UC, the genes are selected from group 3 or group 6,
wherein if the IBD is CD, the genes are selected from group 2 or group 3,
wherein group 2 consist of the genes: BCL2L1, BIRC3, CCL2, CCL22, CCL3, CCL4, CCL5, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TRAF1, VCAM1;
wherein group 3 consist of the genes: BCL2L11, DNMT1, ENPP2, GLRX, MMP1, MMP3, MMP9, PLAU, PLAUR, PTGS2, SERPINE1, TIMP1, VIM;
wherein group 6 consist of the genes: CDKN1A, EAF2, ELL2, FKBP5, LCP1, PLAU, PMEPA1, PRKACB, SGK1, TMPRSS2,
wherein the two or more expression levels are used to determine whether the treatment of IBD in the patient with an anti-TNFalpha compound is effective or not effective,
wherein a decreased expression level of BCL2L11 or PRKACB or an increased expression level of any of the other genes corresponds to a not effective treatment, and
wherein an increased expression level of BCL2L11 or PRKACB or a decreased expression level of any of the other genes corresponds to an effective treatment. Preferably group 2 consist of the genes: BCL2L1, BIRC3, CCL2, CCL22, CCL3, CCL4, CCL5, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TRAF1, VCAM1. Preferably group 3 consist of the genes: GLRX, MMP1, MMP3, MMP9, PLAU, PLAUR, PTGS2, SERPINE1, TIMP1, VIM. Preferably group 6 consist of the genes: EAF2, ELL2, FKBP5, LCP1, PLAU, PMEPA1, TMPRSS2.

The conditions for selecting target genes (T value) and number of genes (N values) were further varied to evaluate the conditions that allow for accurate prediction of treatment or treatment effectiveness. These results are set out in Table 15 below. For each condition resulting in 90% or more of the 1000 random selections of N genes having a significant differentiation between responders and non-responders, the representative gene set are listed below in LIST A. Therefore, in an embodiment, the invention relates to a method for determining the effectiveness of a treatment of a subject suffering from IBD with an anti-TNFalpha compound based on the expression levels of N or more, for example one, two, three, four, five, six, seven eight, nine, ten, eleven or twelve or more, genes selected from a group as listed in LIST A below wherein the application is listed as effectiveness and N is indicated in LIST A for the respective set, and
wherein if the IBD is UC, the N or more, e.g. one, two, three, four, five, six, seven, eight, nine, ten, eleven, twelve or more, genes are selected from the group indicated is UC; or
wherein the IBD is CD, the N or more, e.g. one, two, three, four, five, six, seven, eight, nine, ten, eleven, twelve or more, genes are selected from the indicated is CD; and
wherein the N or more expression levels are used to determine whether the treatment of IBD in the patient with an anti-TNFalpha compound is effective or not effective,
wherein a decreased expression level of BCL2L11 or PRKACB or an increased expression level of any of the other genes corresponds to a not effective treatment, and
wherein an increased expression level of BCL2L11 or PRKACB or a decreased expression level of any of the other genes corresponds to an effective treatment.

Alternatively, the method of the second aspect of the invention relates to a method for determining the effectiveness of a treatment of a subject suffering from IBD with an anti-TNFalpha compound based on inferred cellular signaling pathway activity or activities in a sample obtained from the subject, the method comprising:
comparing the inferred cellular signaling pathway activity, or when multiple cellular signaling pathway activities are used the combined cellular signaling pathway activities, with a threshold value;
wherein if the IBD is UC, the cellular signaling pathway is AR and/or MAPK-AP1,
wherein if the IBD is CD, the cellular signaling pathway is MAPK-AP1 and/or NFkB,
wherein the cellular signaling pathway activity or activities are inferred by
determining an activity level of a transcription factor (TF) element of the respective cellular signaling pathway in the sample obtained from the subject, the TF element controlling transcription of three or more target genes of the respective cellular signaling pathway, the determining being based on evaluating a calibrated mathematical pathway model relating the expression levels of the three or more target genes of the respective cellular signaling pathway to the activity level of the TF element of the cellular signaling pathway, and
inferring the activity of the cellular signaling pathway in the subject based on the determined activity level of the TF element of the respective cellular signaling pathway in the sample of the subject;
wherein the treatment is considered to be successful when the inferred cellular signaling pathway activity or the combined cellular signaling pathway activities are below the threshold value, and wherein the treatment is considered to be unsuccessful when the inferred cellular signaling pathway activity or the combined cellular signaling pathway activities are above the threshold value. In an embodiment, the threshold value is independently determined in one or more reference samples. In an embodiment the three or more, e.g. three, four, five, six, seven, eight, nine, ten, eleven or twelve or more, target genes of the AR cellular signaling pathway are selected from KLK2, PMEPA1, TMPRSS2, NKX3-1, ABCC4, KLK3, FKBP5, ELL2, UGT2B15, DHCR24, PPAP2A, NDRG1, LRIG1, CREB3L4, LCP1, GUCYIA3, AR and EAF2, optionally further at least one target gene, e.g. one, two, three, four, five, six, seven, eight, nine, ten eleven twelve or more target gene(s) selected from the group comprising or consisting of: APP, NTS, PLAU, CDKN1A, DRG1, FGF8, IGF1, PRKACB, PTPN1, SGK1 and TACC2; the three or more, e.g. three, four, five, six, seven, eight, nine, ten, eleven or twelve or more, target genes of the MAPK-AP1 cellular signaling pathway are selected from BCL2L11, CCND1, DDIT3, DNMT1, EGFR, ENPP2, EZR, FASLG, FIGF, GLRX, IL2, IVL, LOR, MMP1, MMP3, MMP9, SERPINE1, PLAU, PLAUR, PTGS2, SNCG, TIMP1, TP53, and VIM, preferably, from the group consisting of: CCND1, EGFR, EZR, GLRX, MMP1, MMP3, PLAU, PLAUR, SERPINE1, SNCG, and TIMP1; the three or more, e.g. three, four, five, six, seven, eight, nine, ten, eleven or twelve or more, target genes of the NFkB cellular signaling pathway are selected from BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKB IE, PTGS2, SELE, STAT5A, TNF, TNFAIP2, TNIP1, TRAF1, and VCAM1.

In an embodiment the method according the first or the second aspect of the invention, the sample obtained from the subject is a gastric, colon, intestinal or rectal sample. The sample may for example be a gastric, colon, intestinal or rectal biopsy.

In an embodiment according the first or the second aspect of the invention, the method further includes determining the expression levels of the target genes based on mRNA extracted from the sample obtained from the subject, preferably wherein the method further includes extracting the mRNA from the sample obtained from the subject.

In an embodiment the method according the first or the second aspect of the invention, the threshold value is independently determined in from one or more reference samples.

It is further envisioned that the method according to the first aspect of the invention can be used in successful stratification of IBD patients (both UC and CD), to predict response to treatment with an anti-TNFalpha compound. Therefore treatment with an anti-TNFalpha compound in an IBD patient is preferably combined with the method as described herein, and the anti-TNFalpha compound is only administered when it is established that the treatment prediction is favorable.

Therefore, in a third aspect the invention relates to an anti-TNFalpha compound for use of in the treatment of IBD in a subject, wherein the use comprises: performing the method as defined in the first or the second aspect of the invention to determine the treatment response of the subject to anti-TNFalpha treatment, and administering the anti-TNFalpha compound of the treatment response to anti-TNFalpha treatment is found to be favorable for the subject. Alternatively, in a third aspect the invention relates to a method of treatment of a subject having IBD by administering an anti-TNFalpha compound, the method comprising performing the method as defined in the first or the second aspect of the invention to determine the treatment response of the subject to anti-TNFalpha treatment, and administering the anti-TNFalpha compound of the treatment response to anti-TNFalpha treatment is found to be favorable for the subject.

In an embodiment the IBD is UC or CD.

In an embodiment the anti-TNFalpha compound is selected from the group consisting of antibody based compounds such as Adalimumab, Certolizumab, Etanercept, Golimumab, Infliximab, or a biosimilars or chimeric products of any of the preceding compounds, such as for example the CT-P13 Infliximab biosimilar, or small molecule based compound such as thalidomide, lenalidomide, pomalidomide, a xanthane derivative such as pentoxifylline, bupropion, an 5-HT_{2A} agonist such as (R)DOI, TCB2, LSD and LA-SS-Az; or a natural compound such as curcumin, catechins, cannabidiol or Echinacea purpurea. An anti TNFalpha compound may be an antibody, a small molecule or a short nucleotide type inhibitor. Other TNFalpha inhibiting compounds may be known to the skilled person, therefore the above list should not be construed as limiting the invention. An antiTNFalpha compound may also be a short nucleotide such as an RNAi, miRNA, siRNA or short nucleotide analogue such as 2'-O-methyl-substituted RNA, locked nucleic acid (LNA) or bridged nucleic acid (BNA), morpholino, or peptide nucleic acid (PNA), targeting a component of the TNFalpha signaling pathway.

In a fourth aspect the invention relates to a kit of parts comprising primers and probes for the detection of the expression levels of three or more target genes, wherein the expression levels of the three or more genes are selected from:
the group consisting of ANGPTL4, BCL2L1, BCL2L11, BIRC3, CCL2, CCL22, CCL3, CCL4, CCL5, CCND1, CD274, CD44, CDKN2B, COL18A1, CTGF, CX3CL1, CXCL1, CXCL2, CXCL3, DKK1, DNMT1, ENPP2, FGF2, FOS, FSCN1, GADD45B, GLRX, HIF1A, HMGA2, HNF1A, HSP90AB1, HSP90B1, ICAM1, IFNG, IL11, IL10, IL1B, IL6, IL8, IRF1, INPP5D, JUNB, KIAA1199, LEF1, MCL1, MMP1, MMP2, MMP3, MMP9, MYC, NFKB2, NFKBIA, NFKBIE, NOS2, OVOL1, PDGFB, PLAU, PLAUR, PTGS2, PTHLH, SAA1, SELE, SERPINE1, SKIL, SMAD4, SNAI2, STAT1, STAT5A, TBX3, TCF7L2, TIMP1, TNF, TNFAIP2, TNFRSF1B, TRAF1, TWIST1, VCAM1, VEGFA, VIM, and ZEB1; or
the group consisting of BCL2L11, CDKN1A, DNMT1, EAF2, ELL2, ENPP2, FKBP5, GLRX, LCP1, MMP1, MMP3, MMP9, PLAU, PLAUR, PMEPA1, PRKACB, PTGS2, SERPINE1, SGK1, TIMP1, TMPRSS2 and VIM; or
the group consisting of BCL2L1, BCL2L11, BIRC3, CCL2, CCL22, CCL3, CCL4, CCL5, CX3CL1, CXCL1, CXCL2, CXCL3, DNMT1, ENPP2, GLRX, ICAM1, IL1B, IL6, IL8, IRF1, MMP1, MMP3, MMP9, NFKB2, NFKBIA, NFKBIE, PLAU, PLAUR, PTGS2, SELE, SERPINE1, STAT5A, TIMP1, TNF, TNFAIP2, TRAF1, VCAM1 and VIM.

Such kit may be advantageously used in the methods and uses described herein. Therefore, in a fifth aspect the invention relates to a use of the kit according to the fourth aspect in the method of the first or the second or the third aspect of the invention. Methods for designing primers and probes for the quantitative detection of expression levels are well known in the art. For example qPCR can be used. The genes named here are listed under their default name. Their sequences can be retrieved from a public database such NCBI genes or genome browsers such as Ensembl or the UCSC genome browser. The kit may additionally comprise buffers and/or enzymes and/or other reagents which can be used in the detection, such as dNTP, MgCl₂ solution, Reverse Transcriptase and/or polymerase.

In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope thereof.

### BRIEF DESCRIPTION OF THE DRAWINGS

General: In all the figures where signal transduction pathway analysis scores are depicted, these are given as log2odds scores for pathway activity, derived from the probability scores for pathway activity provided by the Bayesian pathway model analysis. Log2odds scores indicate the level of activity of a signaling pathway on a linear scale.

Analyzed public datasets are indicated with their GSE number (in the figure legend of each figure).

All validation samples for a signaling pathway model or an immune response/system model are independent samples and have not been used for calibration of the respective model to be validated.

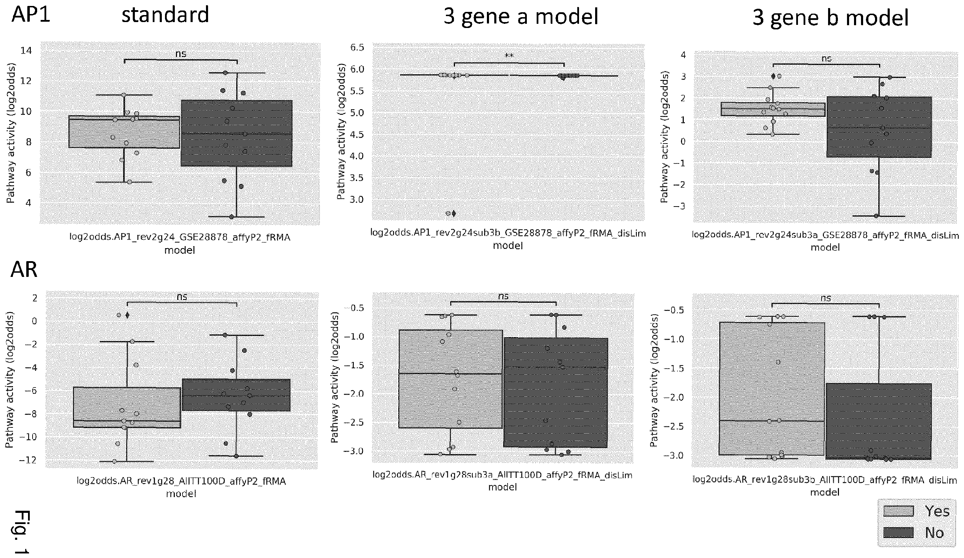

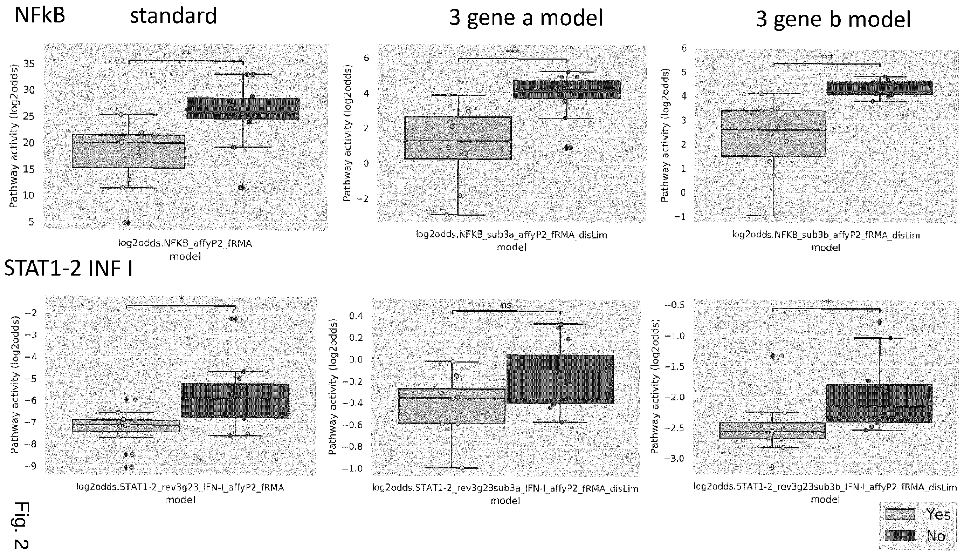

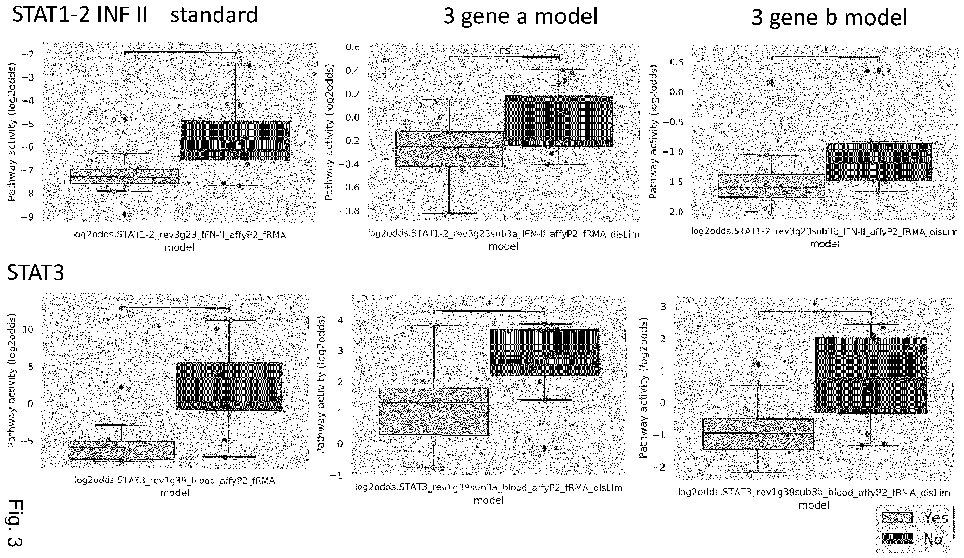

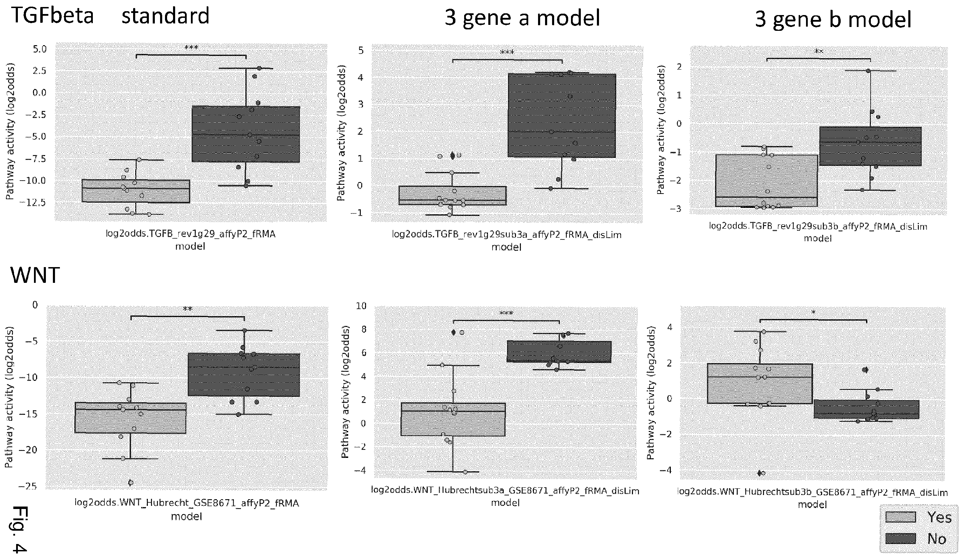

Figs. 1 to 4. Pathway activity scores of dataset GSE12251 including UC patients with responded (yes) or not responded (no) to anti TNFalpha treatment. Pathway activity score on Y-axis in log2odds. Two sided Mann-Whitney-Wilcoxon statistical tests were performed; p-values are indicated in the figures. P-values indicate: *p<0.05; **p<0.01; ***p<0.001. Depicted are cellular signaling pathway activity scores using a model based on the full gene set (standard) or using a random selection of three genes (3 gene a model; 3 gene b model). Fig. 1 shows pathway scores for the MAPK-AP1 and AR pathways. Fig. 2 shows pathway scores for the NFkB and STAT1/2 INF I pathways. Fig. 3 shows pathway scores for the STAT1/2 INF II and STAT3 pathways. Fig. 4 shows pathway scores for the TGFbeta and WNT pathways.

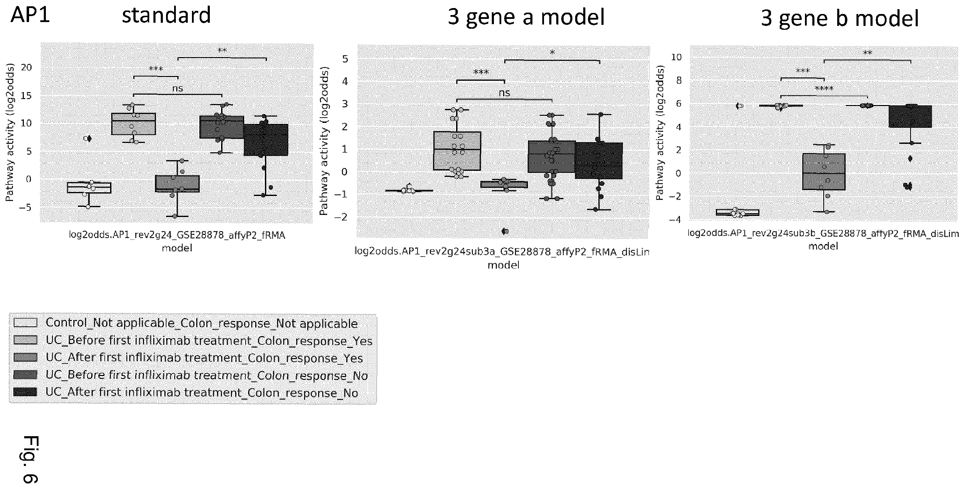

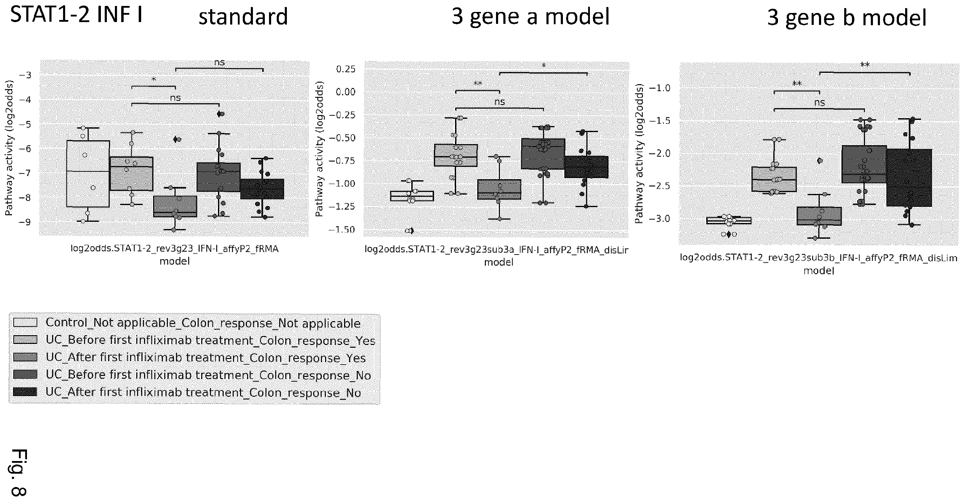

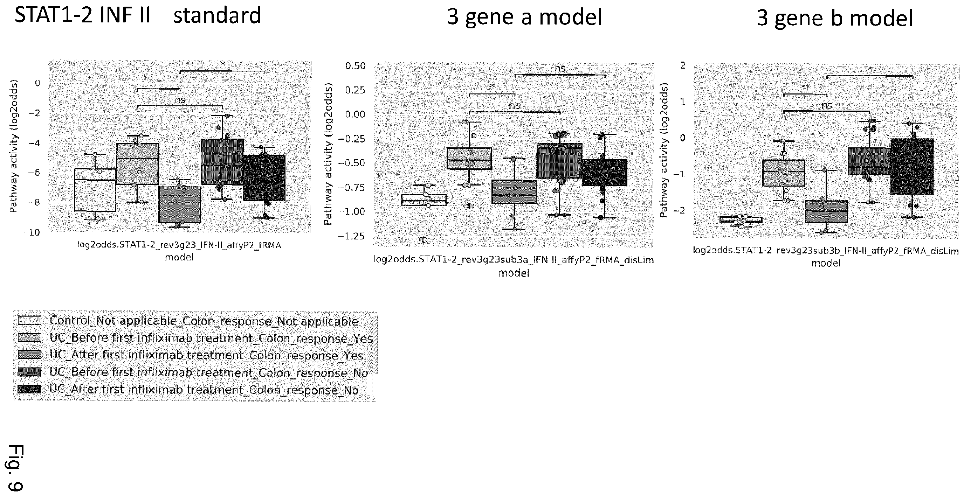

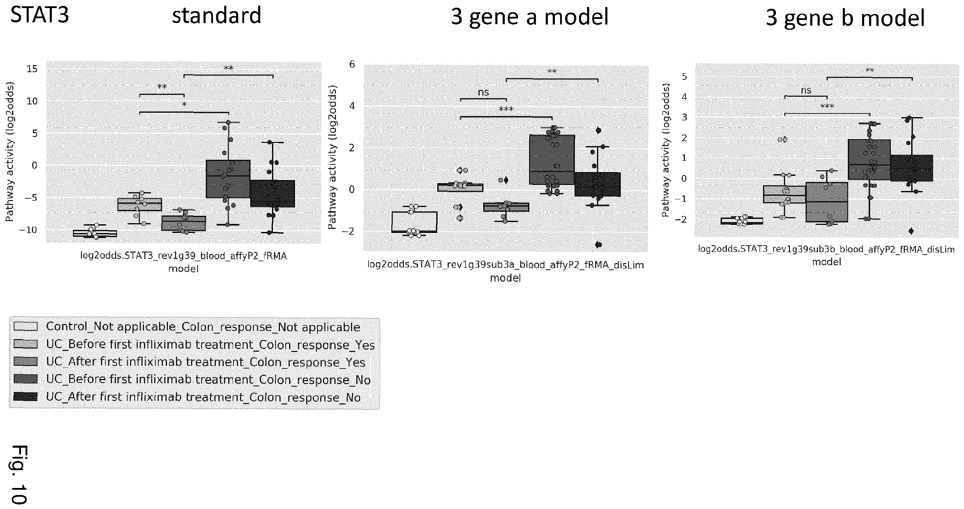

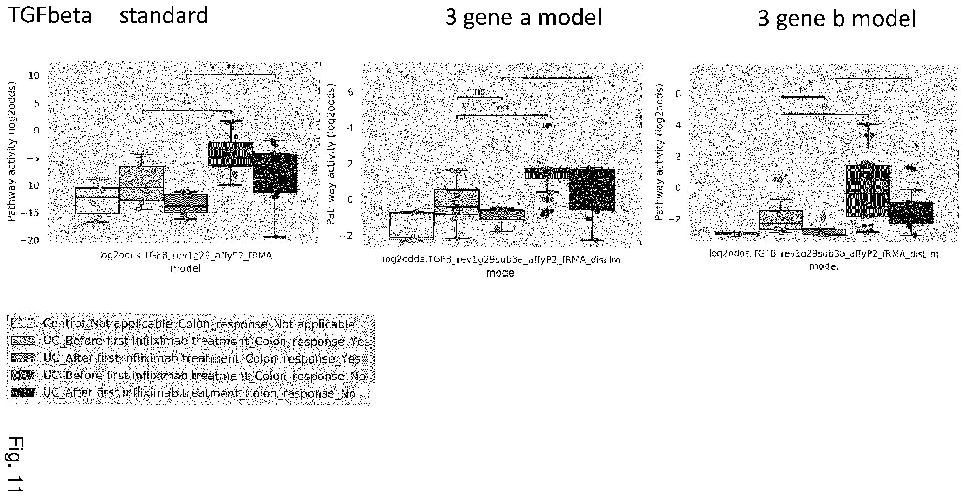

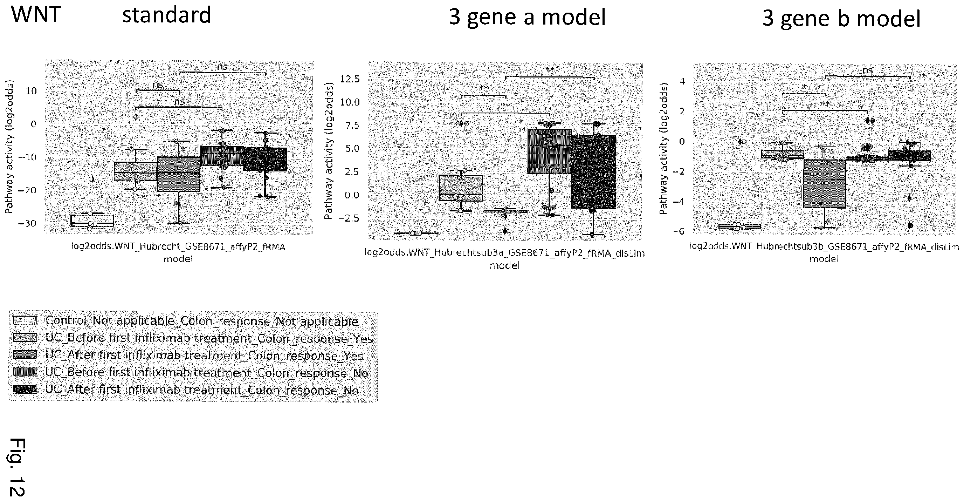

Figs. 5 to 12. Pathway analysis of dataset GSE16879 containing mucosal biopsies were obtained at endoscopy in actively inflamed mucosa from IBD patients (UC) before and 4-6 weeks after their first infliximab infusion (TNF-alpha blocker) and in normal mucosa from control patients. Pathway activity score on Y-axis in log2odds. Two sided Mann-Whitney-Wilcoxon statistical tests were performed; p-values are indicated in the figures. P-values indicate: *p<0.05; **p<0.01; ***p<0.001. Depicted are cellular signaling pathway activity scores using a model based on the full gene set (standard) or using a random selection of three genes (3 gene a model; 3 gene b model). Fig. 5 shows pathway scores for the AR pathway. Fig. 6 shows pathway scores for the MAPK-AP1 pathway. Fig. 7 shows pathway scores for the NFkB pathway. Fig. 8 shows pathway scores for the STAT1/2 INF I pathway. Fig. 9 shows pathway scores for the STAT1/2 INF II pathway. Fig. 10 shows pathway scores for the STAT3 pathway. Fig. 11 shows pathway scores for the TGFbeta pathway. Fig. 12 shows pathway scores for the WNT pathway.

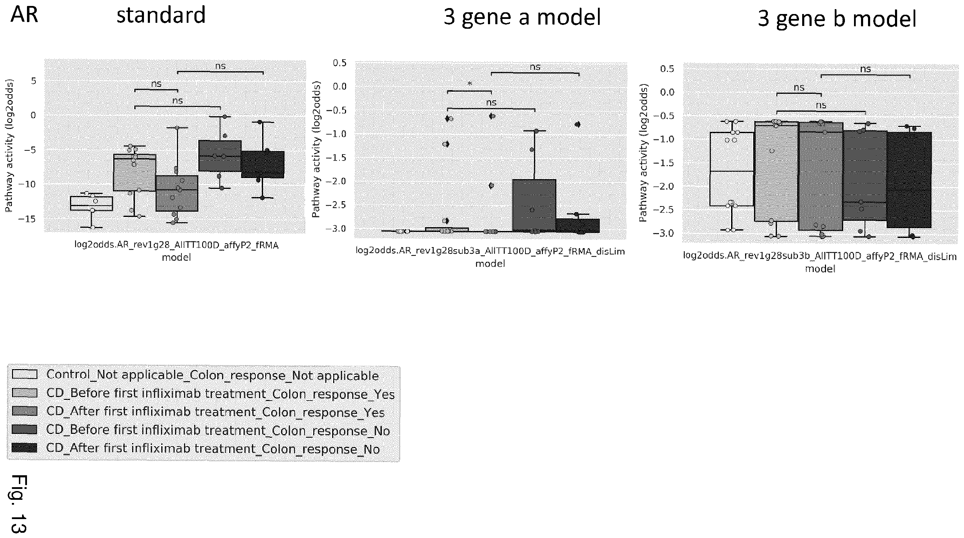

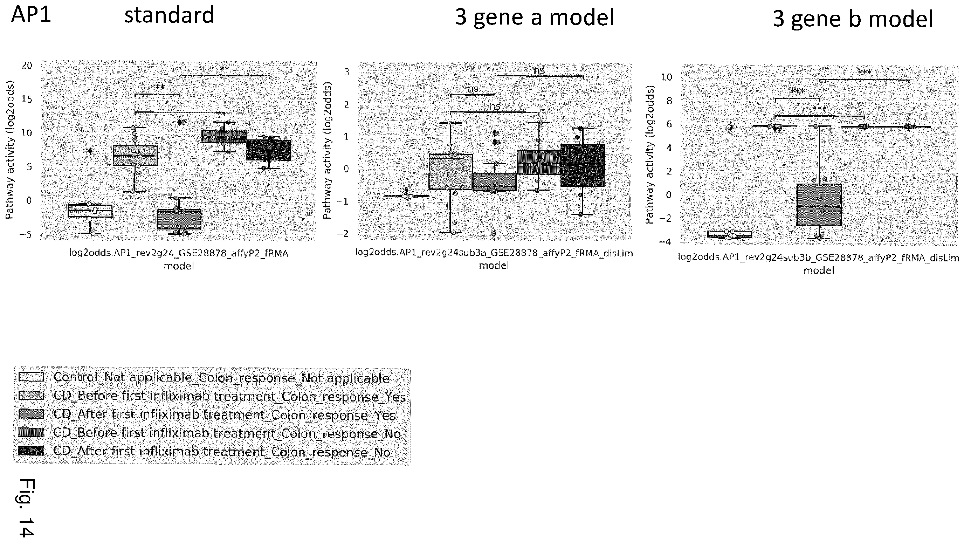

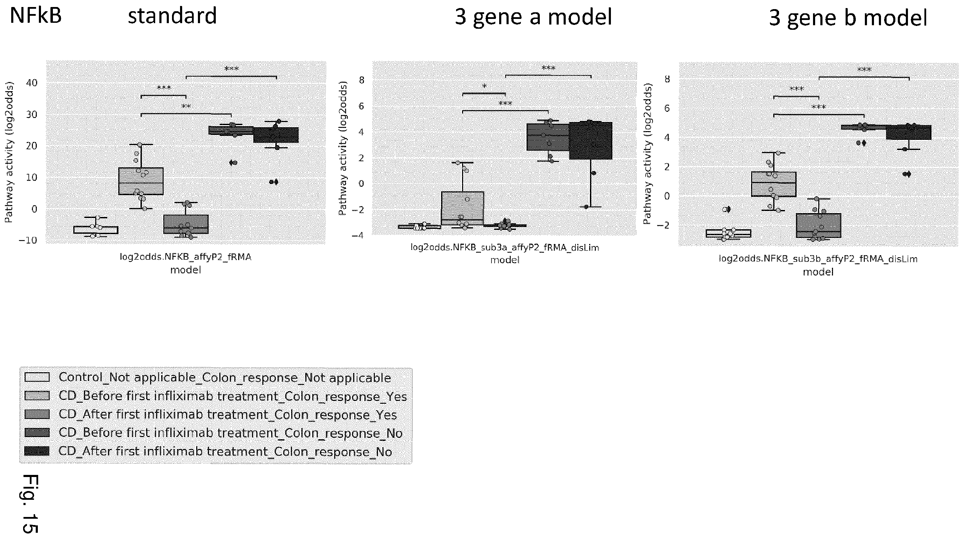

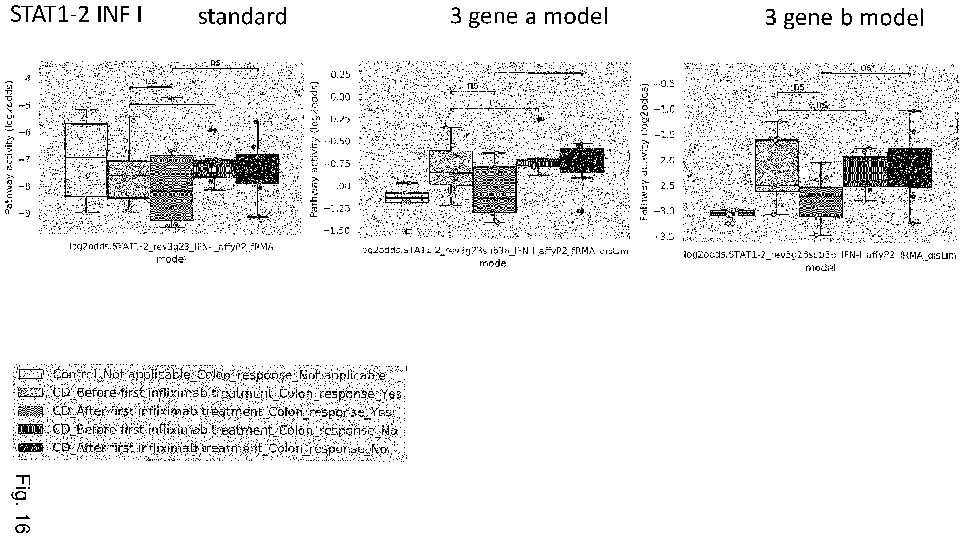

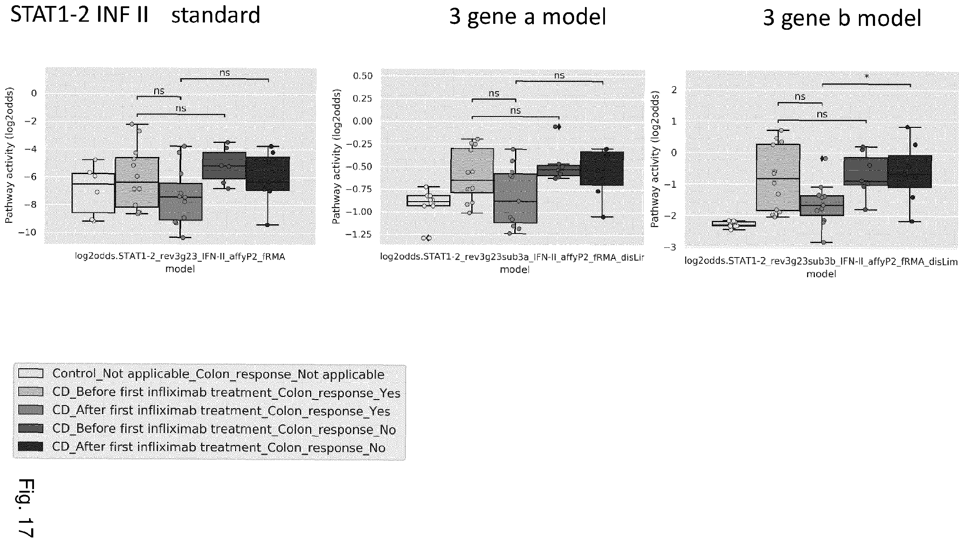

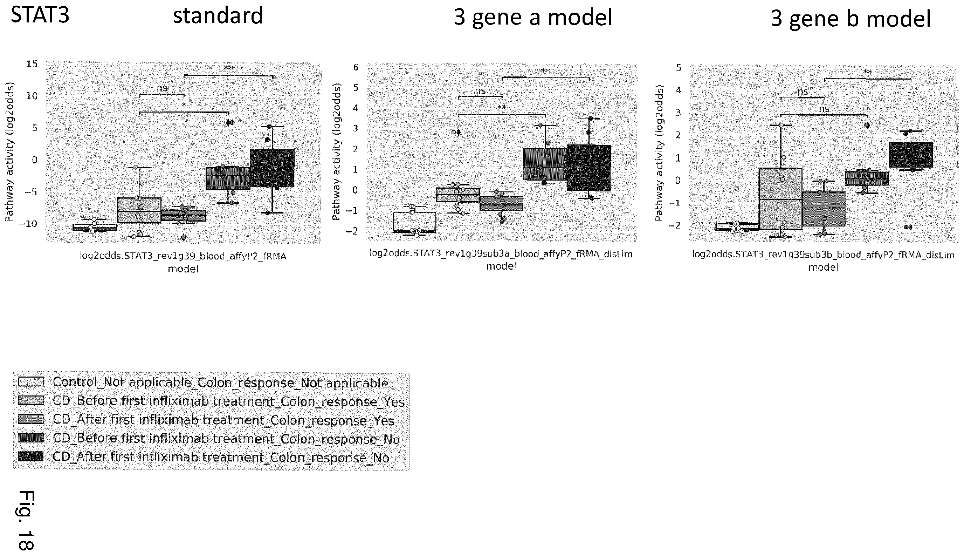

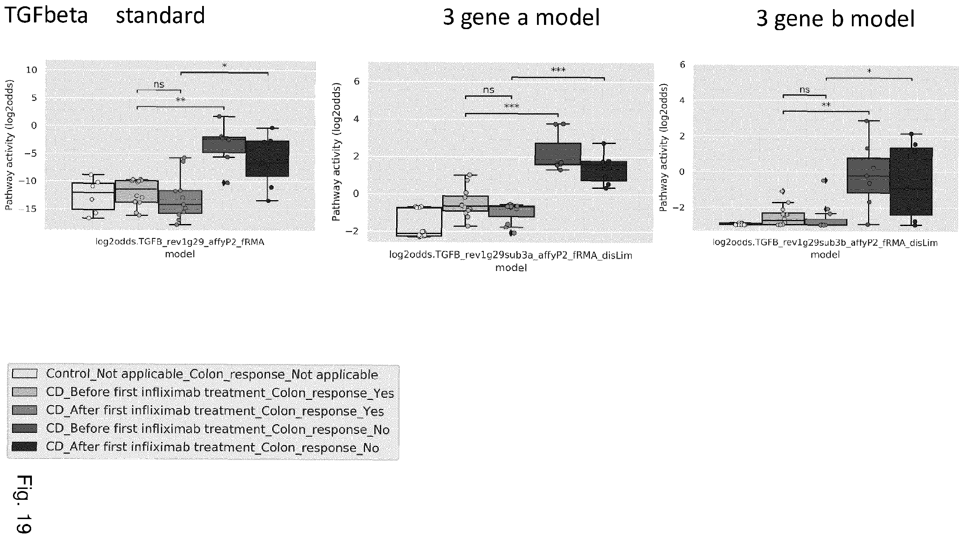

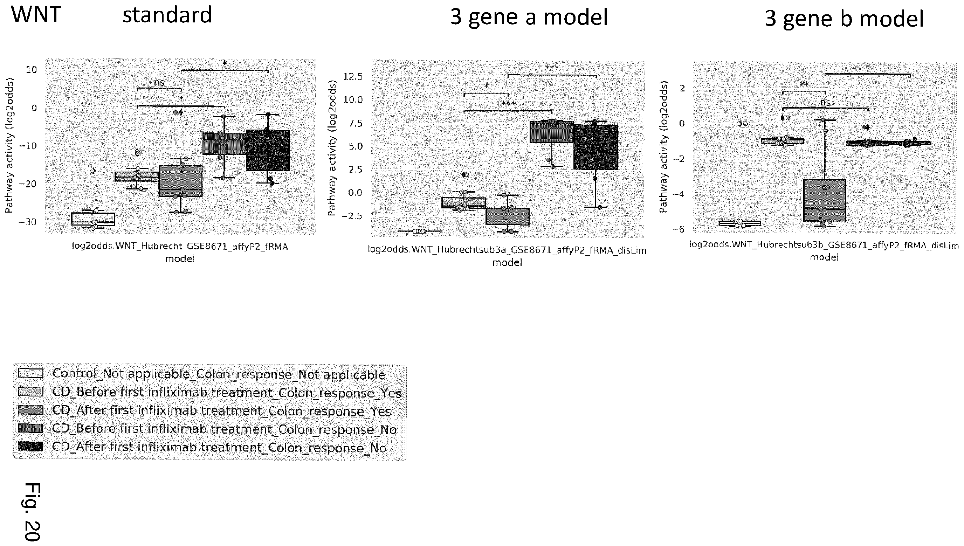

Figs. 13 to 20. Pathway analysis of dataset GSE16879 including actively inflamed mucosa from CD patients, before and 4-6 weeks after their first anti-TNFalpha treatment and in normal mucosa from control patients. Most important pathways include for response prediction; AP1, TGFBeta, WNT, NFkB, STAT3, for treatment effectiveness the NFKB and API. P-values and legends are included in the figure. Pathway activity score on Y-axis in log2odds. Two sided Mann-Whitney-Wilcoxon statistical tests were performed; p-values are indicated in the figures. P-values indicate: *p<0.05; **p<0.01; ***p<0.001. Depicted are cellular signaling pathway activity scores using a model based on the full gene set (standard) or using a random selection of three genes (3 gene a model; 3 gene b model). Fig. 13 shows pathway scores for the AR pathway. Fig. 14 shows pathway scores for the MAPK-AP1 pathway. Fig. 15 shows pathway scores for the NFkB pathway. Fig. 16 shows pathway scores for the STAT1/2 INF I pathway. Fig. 17 shows pathway scores for the STAT1/2 INF II pathway. Fig. 18 shows pathway scores for the STAT3 pathway. Fig. 19 shows pathway scores for the TGFbeta pathway. Fig. 20 shows pathway scores for the WNT pathway.

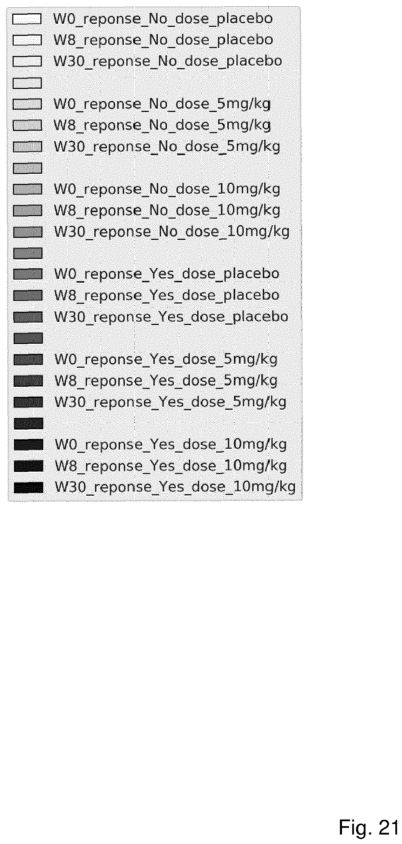

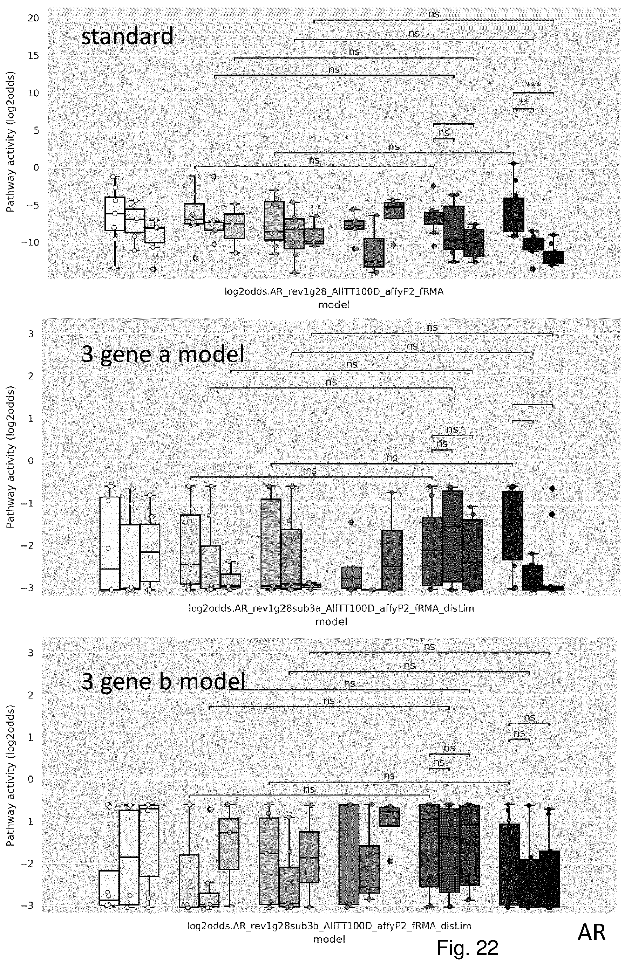

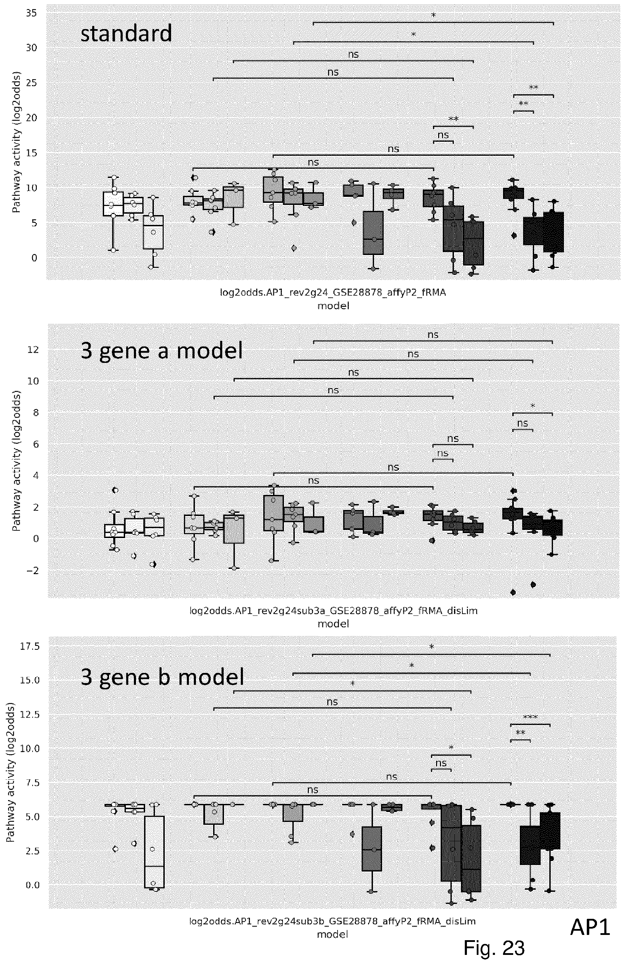

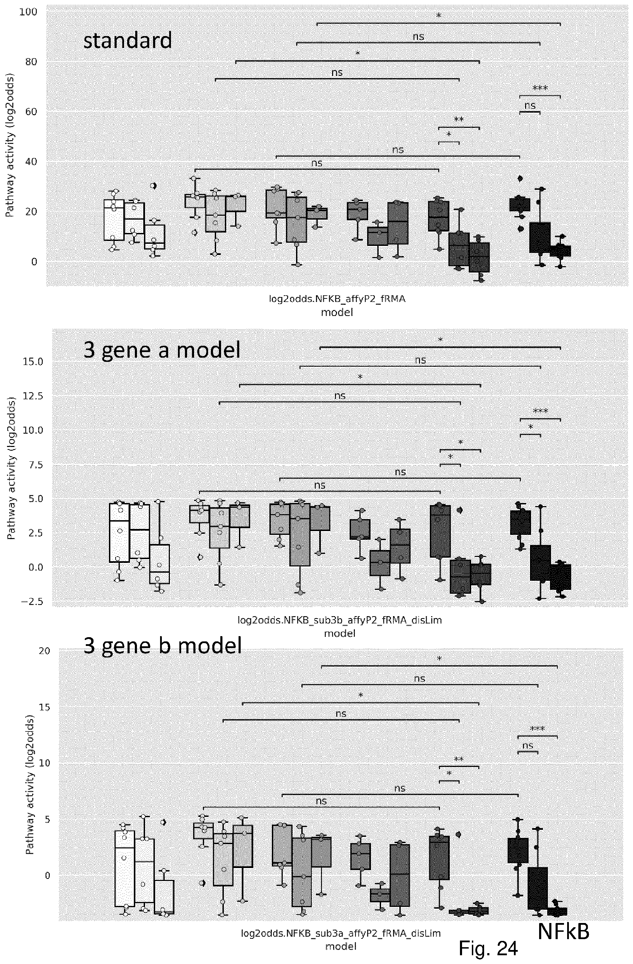

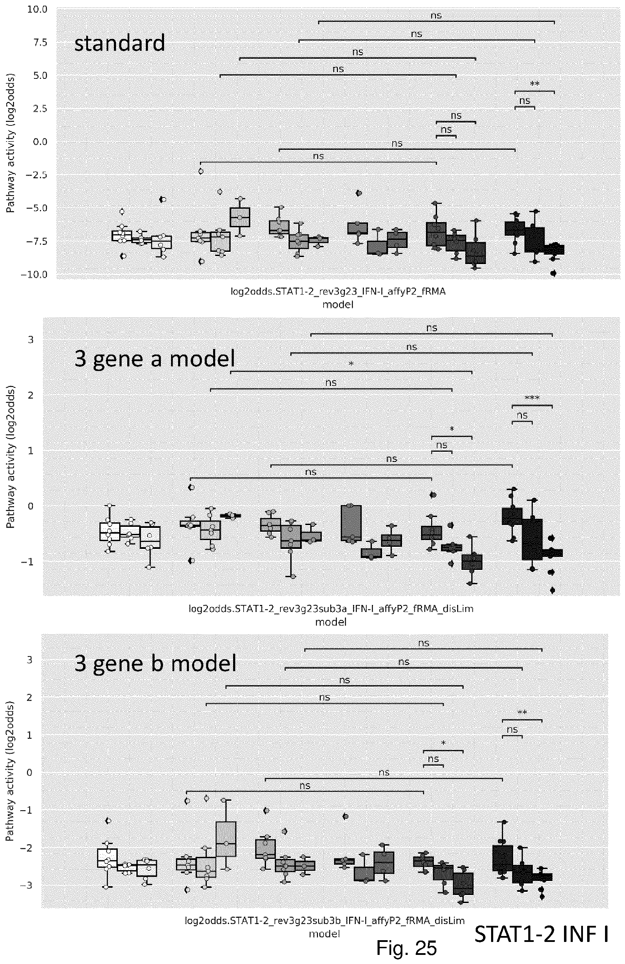

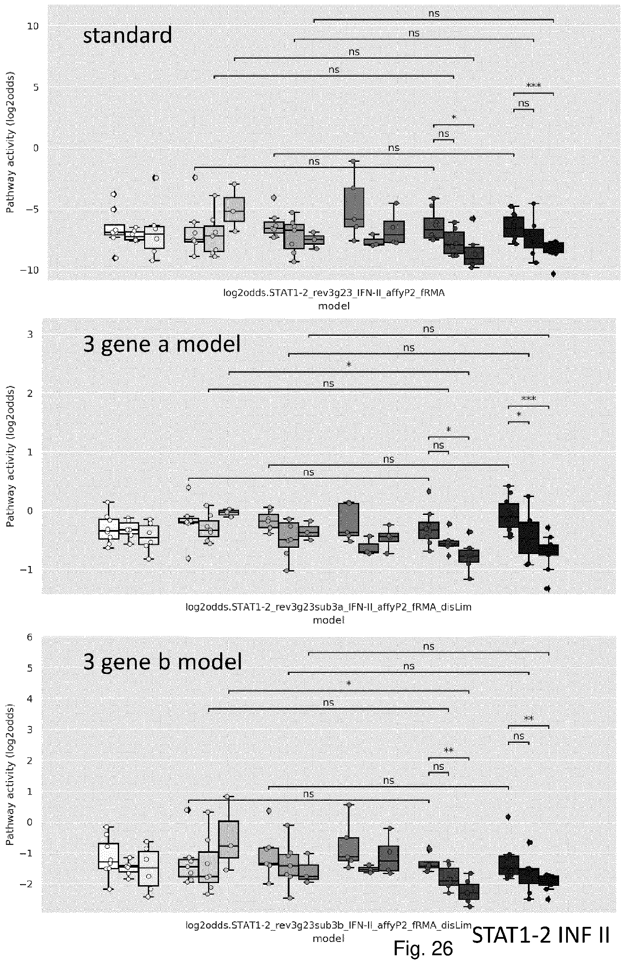

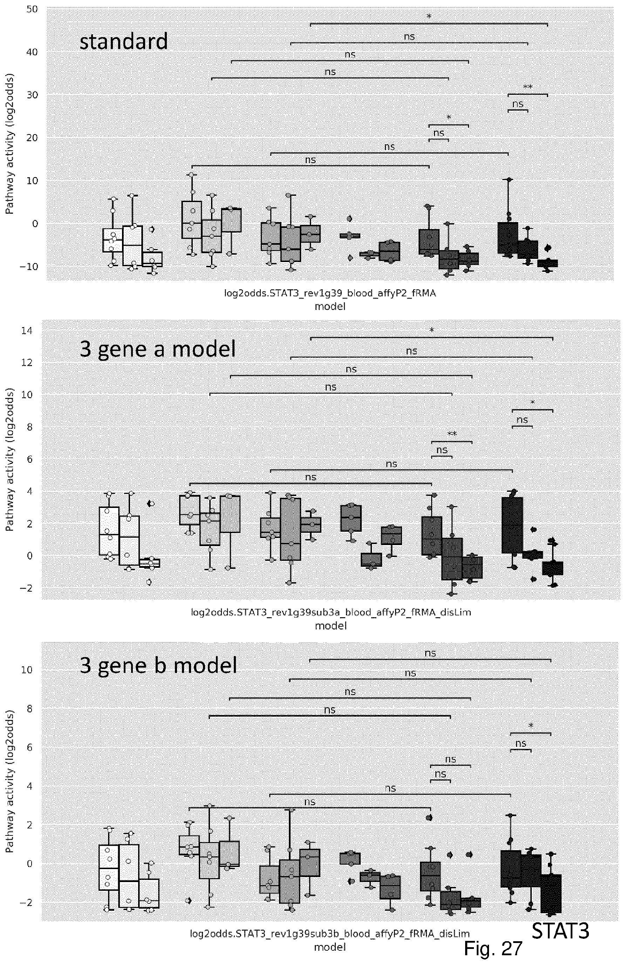

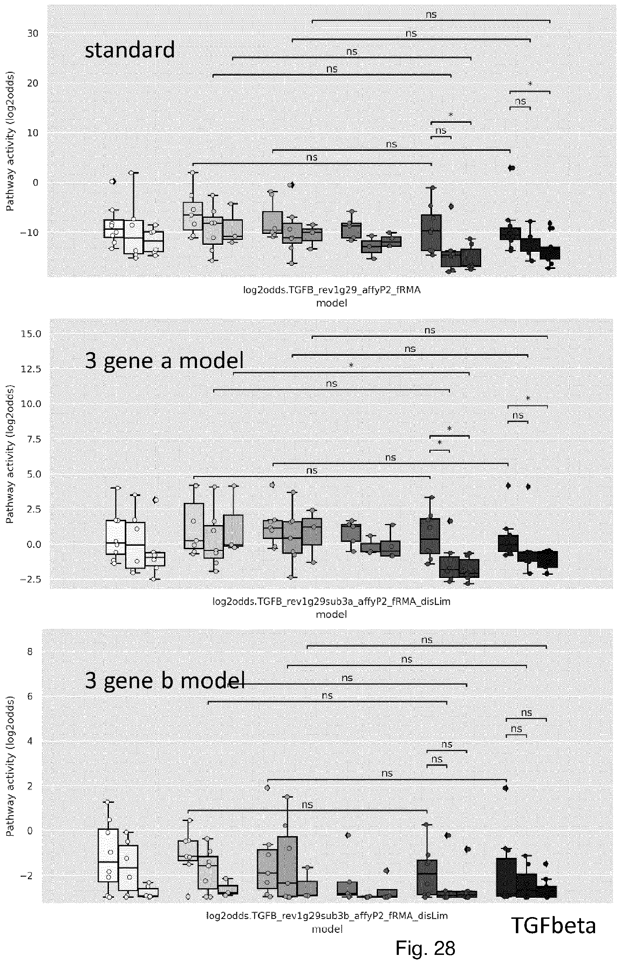

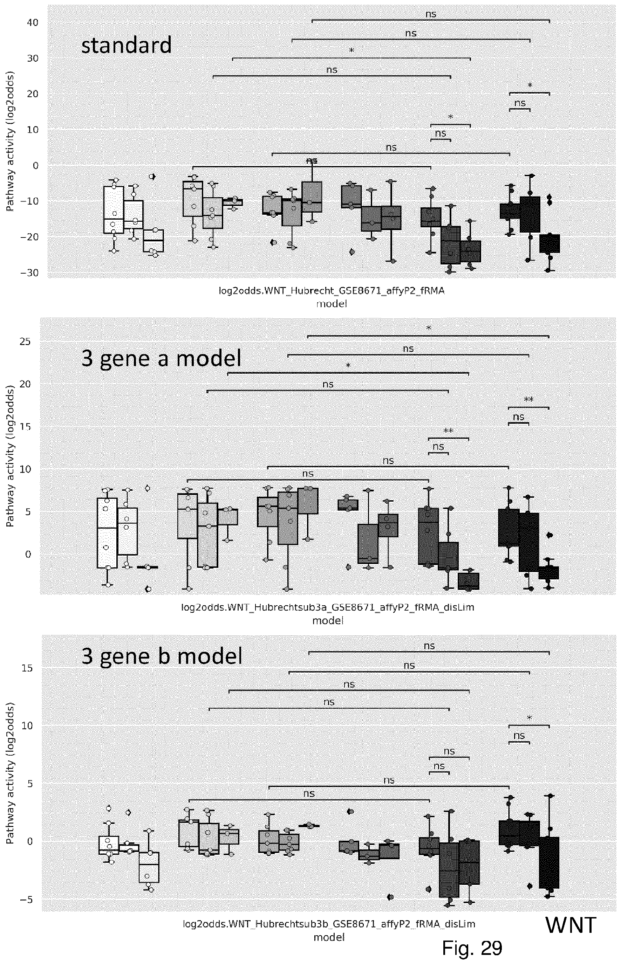

Figs. 21 to 29. Pathway analysis of dataset GSE23597 containing mucosal biopsy samples from UC patients before treatment with placebo, 5 or 10 mg/kg infliximab, and at 8 and 30 weeks after treatment. Pathway activity score on Y-axis in log2odds. Two sided Mann-Whitney-Wilcoxon statistical tests were performed; p-values are indicated in the figures. P-values indicate: *p<0.05; **p<0.01; ***p<0.001. Depicted are cellular signaling pathway activity scores using a model based on the full gene set (standard) or using a random selection of three genes (3 gene a model; 3 gene b model). Fig. 21 shows the figure legend for Fig. 22 to 29. Fig. 22 shows pathway scores for the AR pathway. Fig. 23 shows pathway scores for the MAPK-AP1 pathway. Fig. 24 shows pathway scores for the NFkB pathway. Fig. 25 shows pathway scores for the STAT1/2 INF I pathway. Fig. 26 shows pathway scores for the STAT1/2 INF II pathway. Fig. 27 shows pathway scores for the STAT3 pathway. Fig. 28 shows pathway scores for the TGFbeta pathway. Fig. 29 shows pathway scores for the WNT pathway.

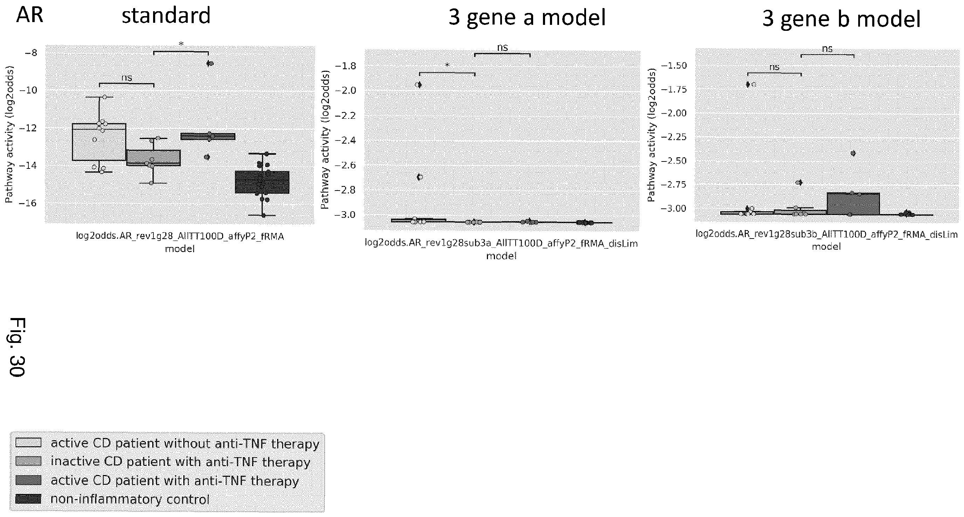

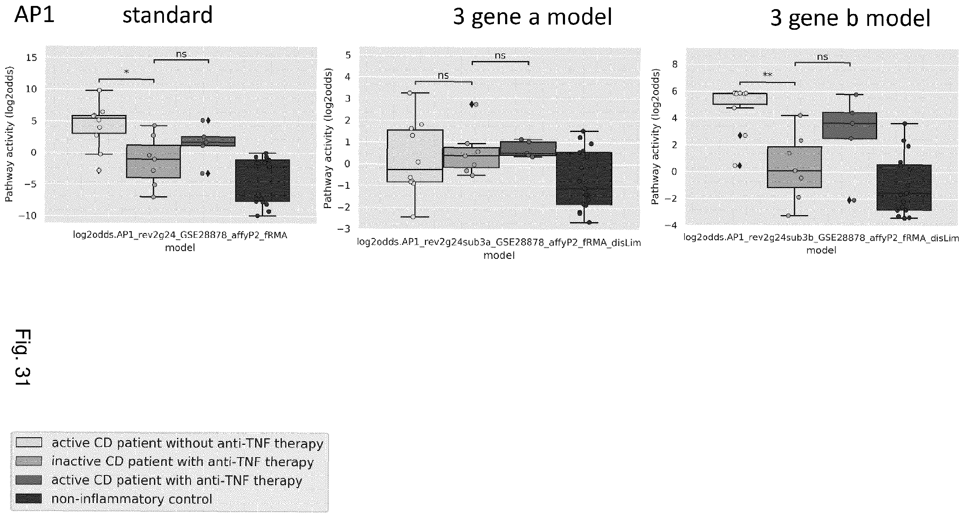

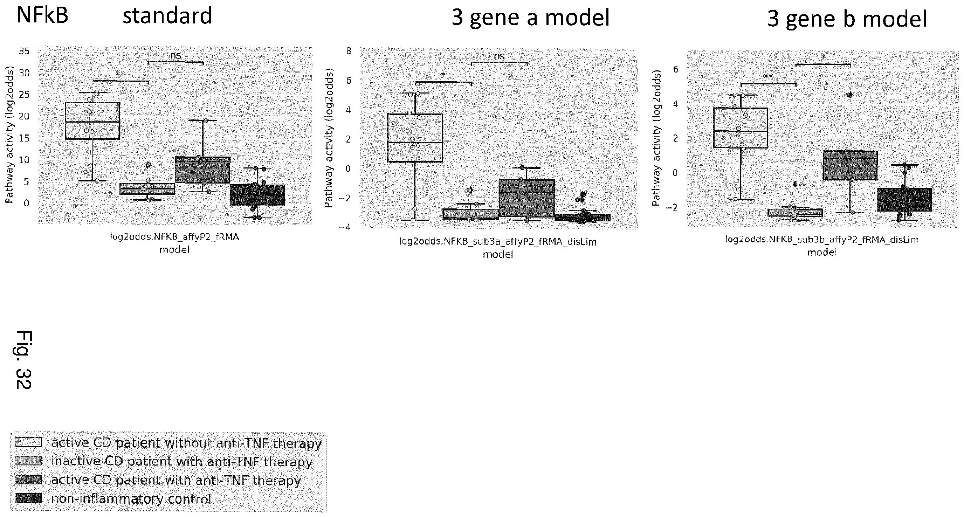

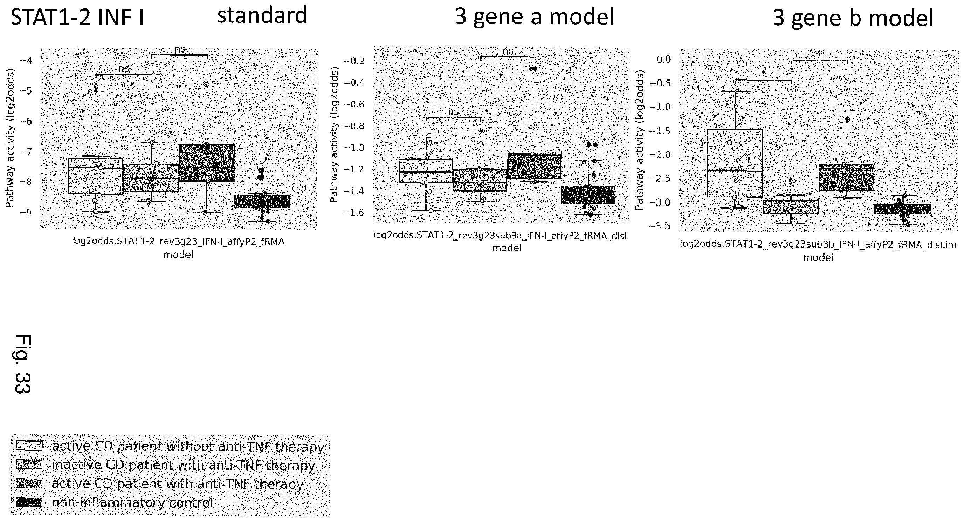

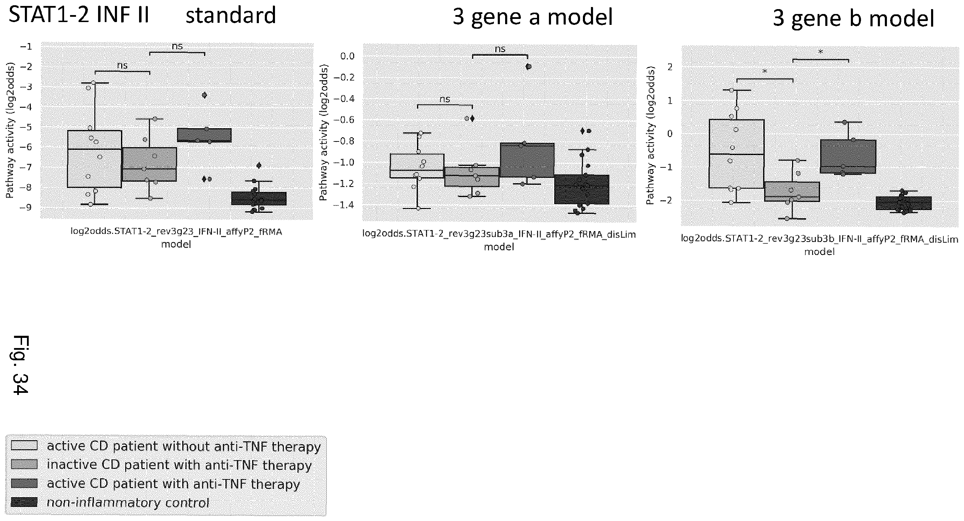

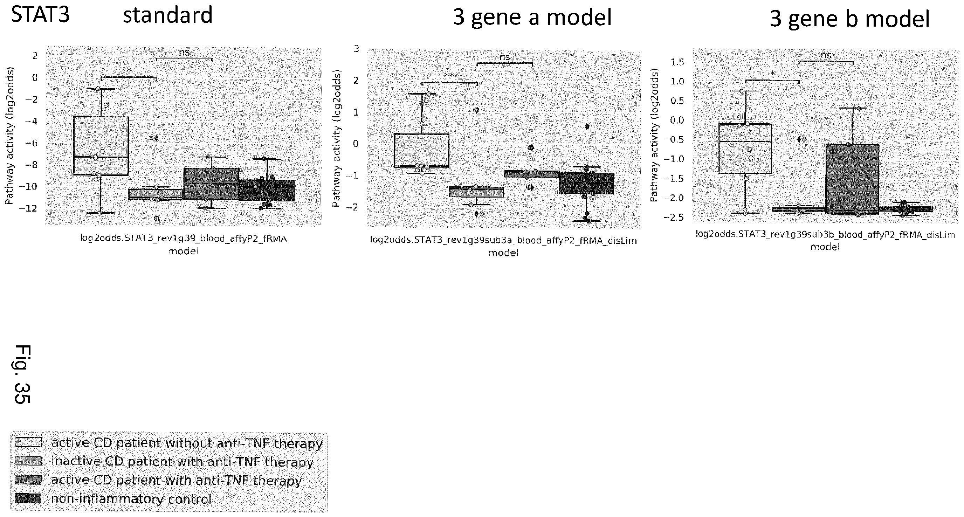

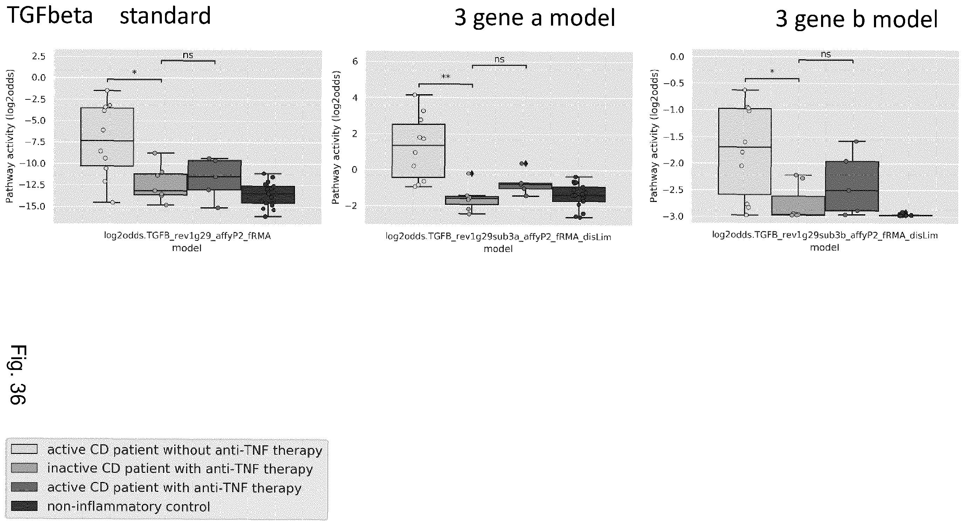

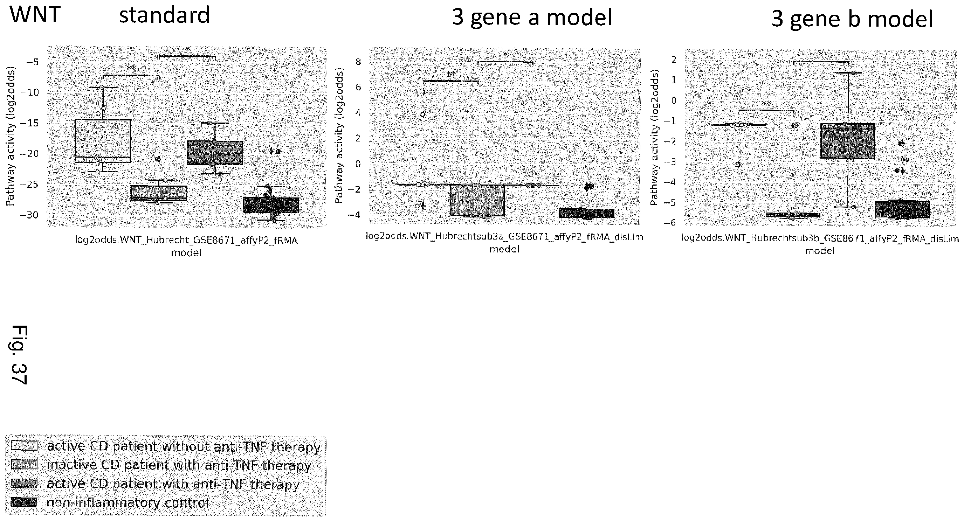

Fig. 30 to 37. Pathway analysis of dataset GSE52746 which contains colonic samples from Crohn's disease patients and healthy colonic samples from non-inflammatory controls. Crohn's disease patients includes active CD with anti-TNF therapy (non-responders) and inactive CD with anti-TNF therapy (responders). Pathway activity score on Y-axis in log2odds. Mann-Whitney-Wilcoxon statistical test were performed; p-values are indicated in the figures. P-values indicate: *p<0.05; **p<0.01; ***p<0.001. Depicted are cellular signaling pathway activity scores using a model based on the full gene set (standard) or using a random selection of three genes (3 gene a model; 3 gene b model). Fig. 30 shows pathway scores for the AR pathway. Fig. 31 shows pathway scores for the MAPK-AP1 pathway. Fig. 32 shows pathway scores for the NFkB pathway. Fig. 33 shows pathway scores for the STAT1/2 INF I pathway. Fig. 34 shows pathway scores for the STAT1/2 INF II pathway. Fig. 35 shows pathway scores for the STAT3 pathway. Fig. 36 shows pathway scores for the TGFbeta pathway. Fig. 37 shows pathway scores for the WNT pathway.

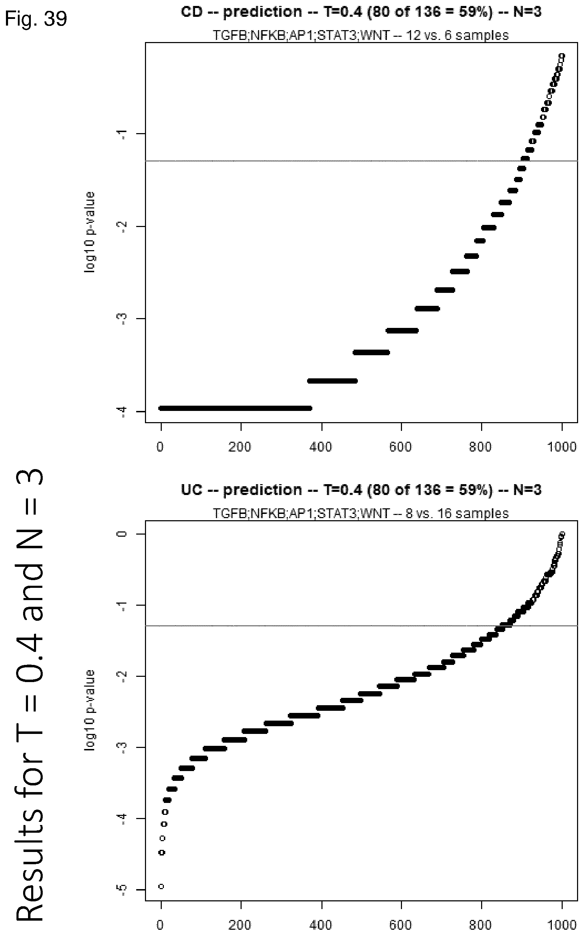

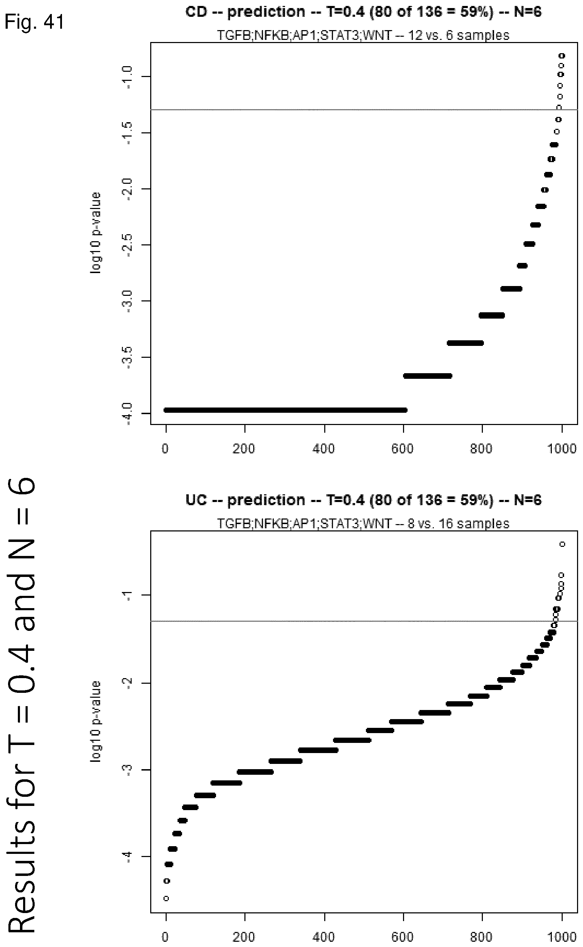

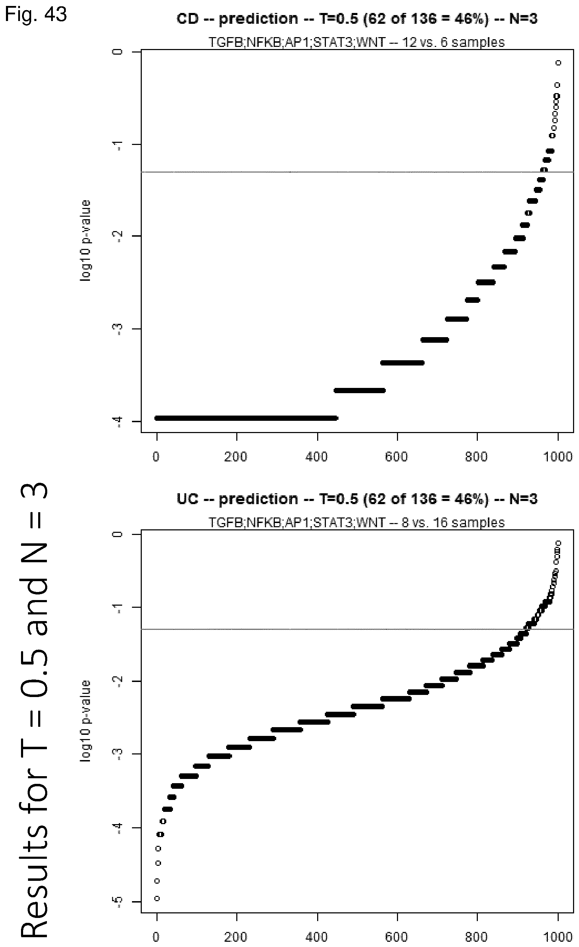

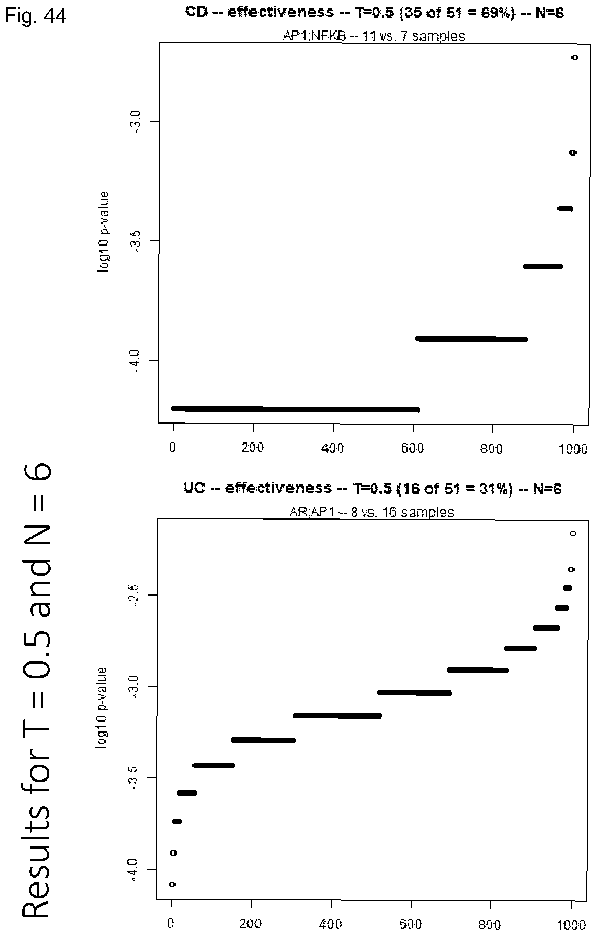

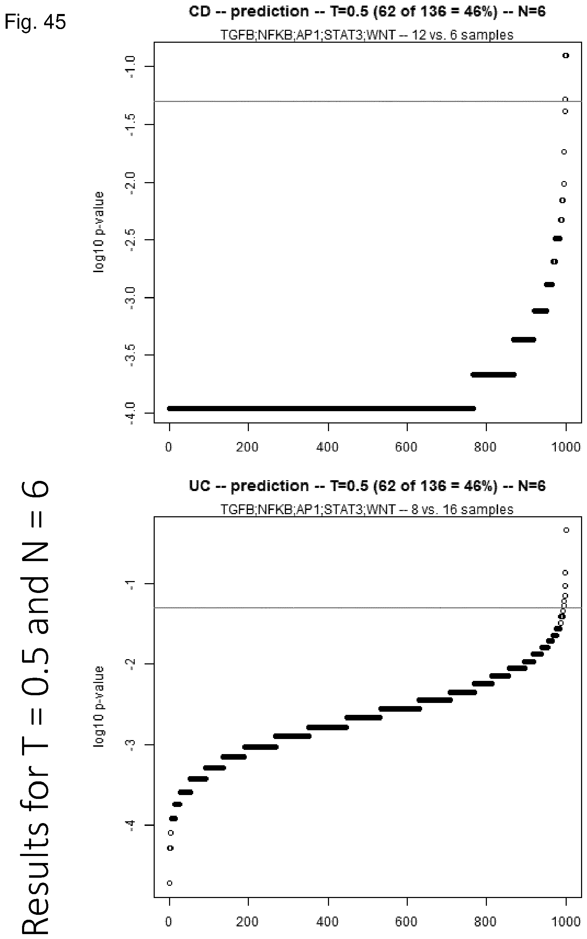

Figs. 38 to 45. For each prediction (treatment effectiveness and treatment prediction) the pathways that are significantly different in activity between groups were identified for UC and CD patients. For each pathway a sub-selection was made on the target genes, identifying those pathway target genes that most contributed to the pathway score in the present applications, the correlation of pathway activity and gene expression levels based on a threshold T. The curates target genes of the predictive pathways were pooled per application. Next 1000 random selections were made of either 3 or 6 (N = 3 or N = 6) of the pooled curated target genes based on either threshold T = 0.4 or T = 0.5. The linear classifier was applied on the samples from the two sample groups to be distinguished, and perform a Wilcox Rank Sum test, resulting in a p-value for each point, which are plotted in Figs. 38-45. Fig. 38 depicts CD and UC effectiveness plots for T = 0.4 and N = 3. Fig. 39 depicts CD and UC prediction plots for T = 0.4 and N = 3. Fig. 40 depicts CD and UC effectiveness plots for T = 0.4 and N = 6. Fig. 41 depicts CD and UC prediction plots for T = 0.4 and N = 6. Fig. 42 depicts CD and UC effectiveness plots for T = 0.5 and N = 3. Fig. 43 depicts CD and UC prediction plots for T = 0.5 and N = 3. Fig. 44 depicts CD and UC effectiveness plots for T = 0.5 and N = 6. Fig. 45 depicts CD and UC prediction plots for T = 0.5 and N = 6.

### EXAMPLES

We used the pathway analysis to define for IBD a characteristic profile of signal transduction pathway activities (AR, ER, PR, GR, HH, Notch, TGFBeta, WNT, JAK-STAT1/2, JAK-STAT3, NFkB, PI3K, MAPK).

### IBD Dataset: GSE16879

Mucosal biopsies were obtained at endoscopy in actively inflamed mucosa from 61 IBD patients (24 ulcerative colitis (UC), 19 Crohn's colitis (CDc) and 18 Crohn's ileitis (CDi)), before and 4-6 weeks after their first infliximab infusion (TNF-alpha blocker) and in normal mucosa from 12 control patients.

Using the pathway analysis approach, we defined a specific pathway activity profile for UC and CD from a mucosal biopsy which enables the prediction of anti TNFalpha drugs treatment response after diagnosis and monitoring of the treatment response.

Subsequently, based on the definition of a specific pathway activity profile for responders to TNFalpha blockers, we developed a few example computational models which can interpret the signaling pathway activity profile from an individual patient tissue or cell sample with respect to the likelihood that the patient will respond to the treatment of anti TNFalpha drugs.

### UC

Figs. 5 to 12 show the pathways scores of the signaling pathways, which are involved in response prediction, treatment effectiveness and response monitoring of UC. Each case will be described separately below.

### Response prediction to anti TNF-alpha treatment (Figs. 5-12)

In UC, the pathway activity before treatment with the anti-TNFalpha were significantly different between responders and non-responders for NFKB, TGFBeta and STAT3, using a two-sided Mann Whitney Wilcoxon test; see Figs. 5 to 12 for p-values and Table 2 for p-values, u-stats and AUC.

There are multiple ways to create computational models for clinical decision support (CDS), based on the pathways' scores. Below 5 examples are provided. Pathway activity scores are given on a log2 odds scale.
1. Use the cluster centroids (see Table 1), and determine for a test sample the distance to each of them (d1 and d2), deciding that the tissue samples is fitting the response cluster with the centroid at closest distance, e,g, response (i.e. d1 < d2), no response (i.e. d2 < d1).
2. Calculate the distance to each of the two centroids (d1 and d2), and assign a probability of no response as d2/ (d1 + d2), and a corresponding probability of response as d1 / (d1 + d2).
3. Calculate the sum of pathway scores, and assign a sample to response if it is equal or above the corresponding threshold, and to no response if below (Table 2)
4. Calculate the sum of pathway scores, and assign a sample to response (define response probability 1) if the sum equals that of centroid C1 or higher, and to no response (define response probability 0) if the sum equals that of centroid C2 or lower, and a linear interpolation if in between (giving response probability between 0 and 1).
5. Similar to 4, but first determining a response probability (points) for each pathway separately, and then adding up. The maximum sum of 4 is obtained if the test sample has all four pathway scores below those of centroid C1, indicating maximum probability of response; the minimum sum of 0 is obtained if the test sample has all four pathway scores above those of centroid C2, indicating minimum probability of response.

More sophisticated classifiers can be built by somebody skilled in the art of machine learning, by using e.g. Bayesian classification, support vector machines or neural nets.

**Table 1: Centroids (Kmeans), delta and threshold based on log2odds values.**

| Pathway (Response: Yes) | Centroid C1 (Response: No) | Centroid C2delta Threshold: (mean of centroids) | | |
|---|---|---|---|---|
| HH | -15.1 | -16.4 | 0.65 | -15.4 |
| NFKB | 9.2 | -5.4 | 14.6 | 1.9 |
| TGFbeta | -8.0 | -12.3 | 4.3 | -10.15 |
| STAT3 | -6.1 | -9.2 | 3.1 | -7.65 |
| Sum | -20 | -43.3 | 23.3 | -31.65 |

### UC response monitoring

For monitoring the effect of anti-TNFalpha treatment, we compared the pathway activity scores before and after treatment, for responders as well as non-responders. Pathways scores which were significant lower after treatment are given in table 1 both for responders, and for non-responders; for pathway figures see Fig. 3.

AR, API, NFKB and STAT3 pathways were significant lower after anti-TNFalpha treatment in responders and non-responders; see Figs. 5 to 12 for p-values and Table 3 for p-values, u-stats and AUC.

### Treatment effectiveness

To determine if a patient was a responder or a non-responder/resistance against the treatment we used a combination of significant pathways, see table 4 for p-values, u-stats and AUC.

### Dataset GSE12251 anti TNF-alpha treatment UC responders and non-responders (Figs. 1-4)

Background information about dataset GSE12251: Twenty-two patients underwent colonoscopy with biopsy before infliximab treatment. Response to infliximab was defined as endoscopic and histologic healing at week 8.

For the response prediction of anti TNF-alpha for UC patients based on pathway signaling we first analyzed pre-treatment pathway activity between the responders and non responders. See Figs. 1 to 4 for the pathway analysis and Table 5 for the P-values, U-stats and AUC. The significant pathways included TGFbeta, WNT, NFKB, STAT3 and STAT1-2.

### Dataset GSE14580: anti TNF-alpha treatment UC responders and non-responders - response prediction (data not shown)

Information about samples in dataset: Patients with active UC, refractory to corticosteroids and/or immunosuppression, underwent colonoscopy with biopsies from diseased colon within a week prior to the first intravenous infusion of 5 mg infliximab per kg body weight. Response to infliximab was defined as endoscopic and histologic healing at 4-6 weeks after first infliximab treatment.

For response prediction of anti TNF-alpha for UC patients based on pathway signaling we analyzed the pathway activity between the groups. Significant pathway models were selected using a Mann-Whitney-Wilcoxon statistical tests. Table 6 shows the p-values, U-stats and AUC. The significant pathways include TGFBeta, NFKB and STAT3.

### UC responders and non-responders before and after treatment (GSE23597) (Figs. 21-29)

Information about samples: Biopsies were obtained from UC patients before treatment with placebo, 5 or 10 mg/kg infliximab (anti-TNFalpha), and at 8 and 30 weeks after treatment.

We analyzed the signaling pathway models for the different groups before and after anti TNF-alpha treatment or placebo. The pathway analysis is shown in Figs. 21 to 29. P-values, U-stats and AUC are shown in Tables 7-9.

For response prediction, no large differences were detected before treatment between the responders and non responders (see Table 7 for p-values, u-stats and AUC).

However, for monitoring the pathways Ap1, AR, NFKB, TGFBeta, STAT1-2 and STAT3 becomes lower after treatment in the responders with the lowest pathway activity scores after 30 weeks compared before start of treatment. Furthermore, the responders after 30 week which received 10 mg dose showed a larger decrease in pathway activity for AR, API, NFKB, STAT1-2 and STAT3 than the patients who received 5mg/kg anti-TNF-alpha treatment.

For measuring the treatment effectiveness responders and non responder after treatment were compared. See table 8 for p-values. Patients who received and responded to 5 mg/kg anti-TNA-alpha showed a significant decrease in the NFKB pathway only after 30 weeks of treatment. Whereas the 10mg/kg group already had significant differences after 8 weeks compared to the non-responders. Furthermore, in the 10 mg/kg responder group also the API and STAT3 pathways (besides the NFKB) became significant different after 30 weeks of treatment compared to the non-responders.

### Crohn Disease

### CD GSE16879 (Figs. 13-20)

Mucosal biopsies were obtained at endoscopy in actively inflamed mucosa from IBD patients Crohn's colitis (CDc) and Crohn's ileitis (CDi)), before and 4-6 weeks after their first infliximab infusion and in normal mucosa from control patients.

For response prediction, treatment effectiveness and response monitoring of CD we analyzed the pathway activity scores. Figs. 13 to 20 show the pathways scores of the signaling pathways which are involved in response prediction, treatment effectiveness and response monitoring of CD.
- For response prediction the following pathways were significantly different between the responders and non-responders before treatment; API, TGFBeta, WNT, NFkB, STAT3_blood.
- For response monitoring API, NFKB, TGFbeta, STAT3blood and WNT
- and for treatment effectiveness the NFKB and AP1.
We used the Mann-Whitney-Wilcoxon statistical test on CD patients to determine which pathways were significantly different between the responders and non-responders to anti-TNFalpha treatment. See Table 10 for p-values, U-stats and AUC.

### Monitoring of treatment response in CD patients

To monitor the effect of the treatment, we compared the pathways before and after treatment. See table 11 for the P-values, U-stats and AUC. The AP1 and NFKB pathways were significantly between the responders before and after treatment.

### Effectiveness of treatment

To determine the effectiveness of the anti-TNF-alpha treatment we compared the responder and non responders towards treatment and used the significant pathways NFKB, API, WNT and STAT3 pathways, see Table 12 for p-values, U-stats and AUC.

### Dataset GSE52746 Active and non-active CD and anti-TNFalpha treatment (data not shown)

Dataset GSE52746 contains data from colonic biopsy samples from Crohn's disease patients and healthy colonic samples from non-inflammatory controls. From the Crohn's disease patients there were 5 non-responders (active CD patients) and 7 responders (inactive CD patients) after anti-TNF therapy. Using the pathway analysis we compared the different groups in the dataset (Table 13).

### Monitoring:

For monitoring we compared the Active CD patients without anti-TNF alpha treatment, which probably consist of potential non responders and responders to anti-TNFalpha treatment, and compared these with the responders after treatment (inactive CD patients). The pathways NFKB, TGFbeta, API, STAT3 and WNT were significant lower after treatment compared to the group without treatment. See table 14 for the p-values and U-stats of the Mann-Whitney-Wilcoxon statistical test and for AUCs.

### Effectiveness:

For the effectiveness we compared the responders and non-responders after anti-TNF alpha treatment. The p values of the Mann-Whitney-Wilcoxon statistical test can be found in Table 15. Only AR and WNT were significant different between the groups.

### 3 Gene models

In order to test if a subset of genes can be used for the pathway models, for each pathway two randomly selected sub-sets of three target genes (a model and b model respectively) were selected as follows:

| Pathway | a model genes | b model genes |
|---|---|---|
| AR | ABCC4; KLK2; PMEPA1 | KLK3; NKX3-1; TMPRSS2 |
| MAPK-AP1 | BCL2L11; EZR; GLRX | MMP1; MMP3; PLAU |
| NFkB | CCL4; ICAM1; NFKBIA | CCL3; IL6; TNFAIP2 |
| STAT1/2* | IRF9; PDCD1; SAMM50 | ST13; STAT1; USP18 |
| STAT3 | HSP90B1; ICAM1; JUNB | CD274; MCL1; ZEB1 |
| TGFbeta | GADD45B; IL11; JUNB | ANGPTL4; CTGF; SERPINE1 |
| WNT | CD44; DEFA6; KIAA1199 | EPHB3; IL8; KLF6 |

| | | |
|---|---|---|
| ** The STAT1*/*2 Interferon I and II models use the same target genes but are calibrated on using IFNalpha for the Interferon I model or IFNgamma for the Interferon II model, while using the same unstimulated control.* | | |

The standard pathway models use all the genes listed as the primary pathway target lists as described above.

Pathway activity scores for all datasets using the "a model" and the "b model", each using three randomly selected target genes are included in figs. 1-37. From the figures it can be concluded that using three pathway models, as compared to the "full model" (using all target genes), the same trends can be observed and in most cases three genes suffice to distinguish between groups (e.g. responders vs non-responders).

### Classification using gene subsets independent of pathway models (Figs. 38-45)

For CD and UC, we have pathways that correlate with response prediction and with treatment effectiveness. We investigated if we can define gene lists such that with each sub-list of e.g. 3 genes can build a (simple) classifier to do the same.

### Steps:

- take the pathways corresponding to the disease (CD, UC) and application (resp., eff) of interest.
- take their corresponding genes and probesets.
- per gene, take the probeset with maximum absolute correlation with the pathway score it contributes to (across all CD and UC samples; a gene may be involved in multiple pathways).
- select a candidate gene list by taking all genes with their probeset-pathway correlation above a threshold T.
- repeatedly (1000 times) choose a random sub-list of N genes from the candidate gene list.
- make a simple linear classifier with those N genes by assigning them a weight +1 or -1 depending on the sign of their correlation.
- apply that linear classifier on the samples from the two sample groups to be distinguished, and perform a Wilcox Rank Sum test, resulting in a p-value.
- check how many of those 1000 p-values are below 0.05.

The results are plotted in figs. 38 to 45.

Candidate gene lists for T = 0.4
Prediction (UC or CD):
   TGFB : +ANGPTL4, -CDKN2B, +CTGF, +GADD45B, -HMGA2, +IL11, +INPP5D, +JUNB, +MMP2, +MMP9, -OVOL1, +PDGFB, +PTHLH, +SERPINE1, +SKIL, -SMAD4, +SNAI2, +TIMP1, +VEGFA
   NFKB : +BCL2L1, +BIRC3, +CCL2, +CCL22, +CCL3, +CCL4, +CCL5, +CX3CL1, +CXCL1, +CXCL2, +CXCL3, +ICAM1, +IL1B, +IL6, +IL8, +IRF1, +MMP9, +NFKB2, +NFKBIA, +NFKBIE, +PTGS2, +SELE, +STAT5A, +TNF, +TNFAIP2, +TRAF1, +VCAM1
   AP1 : -BCL2L11, +CCND1, +DNMT1, +ENPP2, +GLRX, +MMP1, +MMP3, +MMP9, +PLAU, +PLAUR, +PTGS2, +SERPINE1, +TIMP1, +VIM
   STAT3 : +BCL2L1, +CCND1, +CD274, +FGF2, +FOS, +FSCN1, +HIF1A, +HSP90AB1, +HSP90B1, +ICAM1, +IFNG, +IL10, +JUNB, +MCL1, +MMP1, +MMP3, +MMP9, +MYC, +NOS2, +PTGS2, +SAA1, +STAT1, +TIMP1, +TNFRSF1B, +TWIST1, +VIM, +ZEB1
   WNT : +CCND1, +CD44, +COL18A1, +DKK1, -HNF1A, +IL8, +KIAA1199, +LEF1, +MYC, +TBX3, -TCF7L2
Effectiveness UC:
   AR : +CDKN1A, +EAF2, +ELL2, +FKBP5, +LCP1, +PLAU, +PMEPA1, - PRKACB, +SGK1, +TMPRSS2
   AP1 : -BCL2L11, +DNMT1, +ENPP2, +GLRX, +MMP1, +MMP3, +MMP9, +PLAU, +PLAUR, +PTGS2, +SERPINE1, +TIMP1, +VIM
Effectiveness CD:
   AP1 : -BCL2L11, +DNMT1, +ENPP2, +GLRX, +MMP1, +MMP3, +MMP9, +PLAU, +PLAUR, +PTGS2, +SERPINE1, +TIMP1, +VIM
   NFKB : +BCL2L1, +BIRC3, +CCL2, +CCL22, +CCL3, +CCL4, +CCL5, +CX3CL1, +CXCL1, +CXCL2, +CXCL3, +ICAM1, +IL1B, +IL6, +IL8, +IRF1, +MMP9, +NFKB2, +NFKBIA, +NFKBIE, +PTGS2, +SELE, +STAT5A, +TNF, +TNFAIP2, +TRAF1, +VCAM1

### (symbols indicate positive (+) or negative (-) correlation of expression levels and pathway activity)

Candidate gene lists for T = 0.5
Prediction (UC or CD):
   TGFB : +ANGPTL4, -CDKN2B, +CTGF, +GADD45B, +IL11, +INPP5D, +JUNB, +MMP2, +MMP9, +PTHLH, +SERPINE1, +SNAI2, +TIMP1
   NFKB : +BCL2L1, +BIRC3, +CCL2, +CCL22, +CCL3, +CCL4, +CCL5, +CX3CL1, +CXCL1, +CXCL2, +CXCL3, +ICAM1, +IL1B, +IL6, +IL8, +IRF1, +MMP9, +NFKB2, +NFKBIA, +NFKBIE, +PTGS2, +SELE, +STAT5A, +TNF, +TNFAIP2, +TRAF1, +VCAM1
   AP1 : +CCND1, +GLRX, +MMP1, +MMP3, +MMP9, +PLAU, +PLAUR, +PTGS2, +SERPINE1, +TIMP1, +VIM
   STAT3 : +BCL2L1, +CCND1, +CD274, +FGF2, +FSCN1, +HIF1A, +HSP90B1, +ICAM1, +IFNG, +JUNB, +MCL1, +MMP1, +MMP3, +MMP9, +PTGS2, +STAT1, +TIMP1, +TNFRSF1B, +TWIST1, +VIM, +ZEB1
   WNT : +CCND1, +CD44, +COL18A1, +DKK1, +IL8, +KIAA1199, +TBX3
Effectiveness UC:
   AR : +EAF2, +ELL2, +FKBP5, +LCP1, +PLAU, +PMEPA1, +TMPRSS2
   AP1 : +GLRX, +MMP1, +MMP3, +MMP9, +PLAU, +PLAUR, +PTGS2, +SERPINE1, +TIMP1, +VIM
Effectiveness CD:
   AP1 : +GLRX, +MMP1, +MMP3, +MMP9, +PLAU, +PLAUR, +PTGS2, +SERPINE1, +TIMP1, +VIM
   NFKB : +BCL2L1, +BIRC3, +CCL2, +CCL22, +CCL3, +CCL4, +CCL5, +CX3CL1, +CXCL1, +CXCL2, +CXCL3, +ICAM1, +IL1B, +IL6, +IL8, +IRF1, +MMP9, +NFKB2, +NFKBIA, +NFKBIE, +PTGS2, +SELE, +STAT5A, +TNF, +TNFAIP2, +TRAF1, +VCAM1

### (symbols indicate positive (+) or negative (-) correlation of expression levels and pathway activity)

Next it was investigated whether two or even one target gene could be predictive for treatment prediction or treatment effectiveness in UC or CD patients, and if an even lower strictness of gene selection could be used. Therefore, for each UC and CD patient samples (based on dataset GSE16879) the previous experiment was repeated for prediction (treatment prediction) prediction2 (treatment prediction but only target genes from the NFkB or the TGFbeta pathway are selected) or effectiveness (treatment effectiveness) for T=0.3, 0.4 and 0.5 and N=1, 2, 3 or 6. Again, for each condition, N genes were randomly selected 1000 times and it was reviewed how often the random selection of N genes was statistically significant in distinguishing responders and non-responders to anti-TNFalpha treatment. The results are plotted in Table 15, avgp refers to Average P value (of the 1000 random selections of N genes) and sigper refers to the percentage of the 1000 selections that are significant (P lower or equal to 0.05). For each condition that has at least 90 percent significant (meaning at least 900 out of the 1000 random selections of N genes) the corresponding gene sets are listed in list A.

**Table 2 Treatment prediction UC GSE16879**

| Mann-Whitney | UC_Before first infliximab treatment_Colon_response_Yes (n=8) vs. UC_Before first infliximab treatment_Colon_response_No (n=16) | | |
|---|---|---|---|
| pathway | p-value | U-value | AUC |
| AR | 2.09E-01 | 43 | 0.66 |
| AP1 | 9.27E-01 | 66 | 0.52 |
| NFKB | **5.33E-03** | 18 | 0.86 |
| TGFB | **5.33E-03** | 18 | 0.86 |
| STAT1-2 I | 5.61E-01 | 74 | 0.58 |
| STAT1-2 II | 7.83E-01 | 59 | 0.54 |
| STAT3 | **1.10E-02** | 22 | 0.83 |
| WNT | 6.18E-02 | 33 | 0.74 |

**Table 3 Monitoring UC GSE16879**

| Mann-Whitney | UC_Before first infliximab treatment_Colon_response_Yes (n=8) VS UC_After first infliximab treatment_Colon_response_Yes (n=8) | | |
|---|---|---|---|
| pathway | p-value | U-value | AUC |
| AR | **9.39E-04** | 64 | 1.00 |
| AP1 | **9.39E-04** | 64 | 1.00 |
| NFKB | 5.20E-02 | 51 | 0.80 |
| TGFB | **3.13E-02** | 53 | 0.83 |
| STAT1-2 I | **3.13E-02** | 53 | 0.83 |
| STAT1-2 II | **1.01E-02** | 57 | 0.89 |
| STAT3 | **7.41E-03** | 58 | 0.91 |
| WNT | 7.93E-01 | 35 | 0.55 |

**Table 4 Effectiveness UC GSE16879**

| Mann-Whitney | UC_After first infliximab treatment_Colon_response_Yes (n=8) VS UC_After first infliximab treatment_Colon_response_No (n=16) | | |
|---|---|---|---|
| pathway | p-value | U-value | AUC |
| AR | **2.98E-03** | 15 | 0.88 |
| AP1 | **1.05E-03** | 10 | 0.92 |
| NFKB | **2.44E-03** | 14 | 0.89 |
| TGFB | **5.33E-03** | 18 | 0.86 |
| STAT1-2 I | 5.37E-02 | 32 | 0.75 |
| STAT1-2 II | **1.84E-02** | 25 | 0.80 |
| STAT3 | **1.31E-03** | 11 | 0.91 |
| WNT | 1.88E-01 | 42 | 0.67 |

**Table 5 Treatment prediction UC GSE12251**

| Mann-Whitney | Yes (responder)(n=11) VS No (non responder)(n=11) | | |
|---|---|---|---|
| pathway | p-value | U-value | AUC |
| AR | 3.25E-01 | 45 | 0.63 |
| AP1 | 1.00E+00 | 61 | 0.50 |
| NFKB | **5.82E-03** | 18 | 0.85 |
| TGFB | **6.39E-04** | 8 | 0.93 |
| STAT1-2 I | **1.26E-02** | 22 | 0.82 |
| STAT1-2 II | **1.81E-02** | 24 | 0.80 |
| STAT3 | **2.03E-03** | 13 | 0.89 |
| WNT | **2.52E-03** | 14 | 0.88 |

**Table 6 Treatment prediction UC GSE14580**

| Mann-Whitney | UC_Yes (responder) (n=8) VS UC_No (non responder)(n=16) | | |
|---|---|---|---|
| pathway | p-value | U-value | AUC |
| AR | 2.09E-01 | 43 | 0.66 |
| AP1 | 9.27E-01 | 66 | 0.52 |
| NFKB | **5.33E-03** | 18 | 0.86 |
| TGFB | **5.33E-03** | 18 | 0.86 |
| STAT1-2 I | 5.61E-01 | 74 | 0.58 |
| STAT1-2 II | 7.83E-01 | 59 | 0.54 |
| STAT3 | **1.10E-02** | 22 | 0.83 |
| WNT | 6.18E-02 | 33 | 0.74 |

**Table 7 Treatment prediction UC GSE23597**

| Mann-Whitney | W0_reponse_No_d ose_5mg/kg (n=7) VS W0_reponse_Yes_d ose_5mg/kg (n=8) | | | W0_reponse_No_dos e_10mg/kg (n=7) VS W0_reponse_Yes_do se_10mg/kg (n=10) | | |
|---|---|---|---|---|---|---|
| pathway | p-value | U-value | AUC | p-value | U-value | AUC |
| AR | 9.54E-01 | 27 | 0.52 | 4.64E-01 | 27 | 0.61 |
| AP1 | 8.62E-01 | 26 | 0.54 | 6.61E-01 | 40 | 0.57 |
| NFKB | 7.29E-02 | 44 | 0.79 | 8.07E-01 | 32 | 0.54 |
| TGFB | 2.72E-01 | 38 | 0.68 | 2.62E-01 | 47 | 0.67 |
| STAT1-2 I | 7.72E-01 | 25 | 0.55 | 7.33E-01 | 39 | 0.56 |
| STAT1-2 II | 3.25E-01 | 19 | 0.66 | 8.07E-01 | 38 | 0.54 |
| STAT3 | 2.24E-01 | 39 | 0.70 | 9.61E-01 | 36 | 0.51 |
| WNT | 1.83E-01 | 40 | 0.71 | 7.33E-01 | 39 | 0.56 |

**Table 8 Monitoring UC GSE23597**

| Mann-Whitne y | W0_repons e_Yes_dose _5mg/kg (n=8) VS W8_repons e_Yes_dose _5mg/kg (n=6) | | | W0_repons e_Yes_dose _5mg/kg (n=8) VS W30_repon se_Yes_dos e_5mg/kg (n=6) | | | W0_repons e_Yes_dose _10mg/kg (n=10) VS W8_repons e_Yes_dose _10mg/kg (n=7) | | |
|---|---|---|---|---|---|---|---|---|---|
| pathway | p-value | U-value | AUC | p-value | U-value | AUC | p-value | U-value | AUC |
| AR | 3.33E-01 | 32 | 0.67 | **2.39E-02** | 42 | 0.88 | **0.00** | 67 | 0.96 |
| AP1 | 8.14E-02 | 38 | 0.79 | **3.68E-03** | 47 | 0.98 | **0.01** | 64 | 0.91 |
| NFKB | **3.32E-02** | 41 | 0.85 | **5.51E-03** | 46 | 0.96 | 0.07 | 54 | 0.77 |
| TGFB | 6.12E-02 | 39 | 0.81 | **2.39E-02** | 42 | 0.88 | 0.13 | 51 | 0.73 |
| STAT1-2 I | 2.20E-01 | 34 | 0.71 | 6.12E-02 | 39 | 0.81 | 0.31 | 46 | 0.66 |
| STAT1-2 II | 6.12E-02 | 39 | 0.81 | **2.39E-02** | 42 | 0.88 | 0.22 | 48 | 0.69 |
| STAT3 | 1.07E-01 | 37 | 0.77 | **4.54E-02** | 40 | 0.83 | 0.22 | 48 | 0.69 |
| WNT | 8.14E-02 | 38 | 0.79 | **2.39E-02** | 42 | 0.88 | 0.96 | 35 | 0.50 |

**Table 9 Effectiveness UC GSE23597**

| Mann-Whitne y | W8_repons e_No_dose _5mg/kg (n=7) VS W8_repons e_Yes_dos e_5mg/kg (n=6) | | | W30_repon se_No_dose _5mg/kg (n=3) VS W30_repon se_Yes_dos e_5mg/kg (n=6) | | | W8_repon se_No_dos e_10mg/kg (n=7) VS W8_repon se_Yes_do se_10mg/k g (n=7) | | |
|---|---|---|---|---|---|---|---|---|---|
| pathwa y | p-value | U-val ue | AUC | p-value | U-value | AUC | p-value | U-value | AUC |
| AR | 3.53E-01 | 28 | 0.67 | 1.56E-01 | 15 | 0.83 | 3.07E-01 | 33 | 0.67 |
| AP1 | 2.25E-01 | 30 | 0.71 | 1.56E-01 | 15 | 0.83 | **2.98E-02** | 42 | 0.86 |
| NFKB | 7.42E-02 | 34 | 0.81 | **2.82E-02** | 18 | 1.00 | 3.07E-01 | 33 | 0.67 |
| TGFB | 1.00E-01 | 33 | 0.79 | 5.28E-02 | 17 | 0.94 | 3.07E-01 | 33 | 0.67 |
| STAT1-2 I | 6.17E-01 | 25 | 0.60 | 5.28E-02 | 17 | 0.94 | 1.00E+00 | 24 | 0.51 |
| STAT1-2 II | 7.21E-01 | 24 | 0.57 | 5.28E-02 | 17 | 0.94 | 7.98E-01 | 27 | 0.55 |
| STAT3 | 1.34E-01 | 32 | 0.76 | 9.33E-02 | 16 | 0.89 | 7.98E-01 | 27 | 0.55 |
| WNT | 1.00E-01 | 33 | 0.79 | **2.82E-02** | 18 | 1.00 | 8.98E-01 | 26 | 0.53 |

**Table 10 Treatment prediction CD GSE16879**

| Mann-Whitney | CD_Before first infliximab treatment_Colon_response_No (n=6) VS CD_Before first infliximab treatment_Colon_response_Yes (n=12) | | |
|---|---|---|---|
| pathway | p-value | U-value | AUC |
| AR | 2.06E-01 | 50 | 0.69 |
| AP1 | **3.51E-02** | 59 | 0.82 |
| NFKB | **2.34E-03** | 69 | 0.96 |
| TGFB | **5.73E-03** | 66 | 0.92 |
| STAT1-2 I | 4.26E-01 | 45 | 0.63 |
| STAT1-2 II | 3.25E-01 | 47 | 0.65 |
| STAT3 | **1.00E-02** | 64 | 0.89 |
| WNT | **1.00E-02** | 64 | 0.89 |

**Table 11 Monitoring CD GSE16879**

| Mann-Whitney | CD_Before first infliximab treatment_Colon_response_Yes (n=12) VS CD_After first infliximab treatment_Colon_response_Yes (n= 11) | | |
|---|---|---|---|
| pathway | p-value | U-value | AUC |
| AR | 7.94E-02 | 95 | 0.72 |
| AP1 | **9.92E-04** | 120 | 0.91 |
| NFKB | **1.20E-04** | 129 | 0.98 |
| TGFB | 2.82E-01 | 84 | 0.64 |
| STAT1-2 I | 3.40E-01 | 82 | 0.62 |
| STAT1-2 II | 1.66E-01 | 89 | 0.67 |
| STAT3 | 3.72E-01 | 81 | 0.61 |
| WNT | 3.72E-01 | 81 | 0.61 |

**Table 12 Effectiveness CD GSE16879**

| Mann-Whitney | CD_After first infliximab treatment_Colon_response_No (n=7) VS CD_After first infliximab treatment_Colon_response_Yes (n=11) | | |
|---|---|---|---|
| pathway | p-value | U-value | AUC |
| AR | 5.72E-02 | 60 | 0.78 |
| AP1 | **4.99E-03** | 70 | 6.36 |
| NFKB | **5.78E-04** | 77 | 7.00 |
| TGFB | **1.45E-02** | 66 | 6.00 |
| STAT1-2 I | 2.77E-01 | 51 | 4.64 |
| STAT1-2 II | 1.47E-01 | 55 | 5.00 |
| STAT3 | **2.08E-03** | 73 | 6.64 |
| WNT | **4.63E-02** | 61 | 5.55 |

**Table 13 Monitoring CD GSE52746**

| | active CD patient without anti-TNF therapy (n=10) VS inactive CD patient with anti-TNF therapy (n=7) | | |
|---|---|---|---|
| pathway | p-value | U-value | AUC |
| AR | 1.30E-01 | 51 | 0.73 |
| AP1 | **1.68E-02** | 60 | 0.86 |
| NFKB | **2.11E-03** | 67 | 0.96 |
| TGFB | **2.18E-02** | 59 | 0.84 |
| STAT1-2 I | 8.07E-01 | 38 | 0.54 |
| STAT1-2 II | 6.61E-01 | 40 | 0.57 |
| STAT3 | **2.81E-02** | 58 | 0.83 |
| WNT | **2.92E-03** | 66 | 0.94 |

**Table 14 Effectiveness CD GSE52746**

| Mann-Whitney | active CD patient with anti-TNF therapy (n=5) VS inactive CD patient with anti-TNF therapy (n=7) | | |
|---|---|---|---|
| pathway | p-value | U-value | AUC |
| AR | **2.30E-02** | 3 | 0.91 |
| AP1 | 3.30E-01 | 11 | 0.69 |
| NFKB | 1.04E-01 | 7 | 0.80 |
| TGFB | 7.45E-01 | 15 | 0.57 |
| STAT1-2 I | 7.45E-01 | 15 | 0.57 |
| STAT1-2 II | 1.94E-01 | 9 | 0.74 |
| STAT3 | 5.16E-01 | 13 | 0.63 |
| WNT | **2.30E-02** | 3 | 0.91 |

**Table 15**

| T | N | disease | application | avgp | sigper |
|---|---|---|---|---|---|
| 0.3 | 1 | UC | pred iction | 0.163816 | 48 |
| 0.3 | 1 | UC | prediction2 | 0.15101 | 57 |
| 0.3 | 1 | UC | effectiveness | 0.088594 | 72 |
| 0.3 | 1 | CD | pred iction | 0.179574 | 51 |
| 0.3 | 1 | CD | prediction2 | 0.147276 | 54 |
| 0.3 | 1 | CD | effectiveness | 0.049109 | 86 |
| 0.3 | 2 | UC | pred iction | 0.10869 | 67 |
| 0.3 | 2 | UC | prediction2 | 0.062496 | 79 |
| 0.3 | 2 | UC | effectiveness | 0.039748 | 88 |
| 0.3 | 2 | CD | pred iction | 0.074105 | 74 |
| 0.3 | 2 | CD | prediction2 | 0.039602 | 82 |
| 0.3 | 2 | CD | effectiveness | 0.007279 | 98 |
| 0.3 | 3 | UC | pred iction | 0.051243 | 82 |
| 0.3 | 3 | UC | prediction2 | 0.029954 | 87 |
| 0.3 | 3 | UC | effectiveness | 0.017667 | 91 |
| 0.3 | 3 | CD | pred iction | 0.035944 | 87 |
| 0.3 | 3 | CD | prediction2 | 0.013289 | 93 |
| 0.3 | 3 | CD | effectiveness | 0.001273 | 100 |
| 0.3 | 6 | UC | pred iction | 0.012236 | 96 |
| 0.3 | 6 | UC | prediction2 | 0.005442 | 99 |
| 0.3 | 6 | UC | effectiveness | 0.003728 | 98 |
| 0.3 | 6 | CD | pred iction | 0.003404 | 98 |
| 0.3 | 6 | CD | prediction2 | 0.000723 | 100 |
| 0.3 | 6 | CD | effectiveness | 0.000147 | 100 |
| 0.4 | 1 | UC | pred iction | 0.143565 | 53 |
| 0.4 | 1 | UC | prediction2 | 0.127825 | 61 |
| 0.4 | 1 | UC | effectiveness | 0.03865 | 81 |
| 0.4 | 1 | CD | pred iction | 0.190181 | 55 |
| 0.4 | 1 | CD | prediction2 | 0.129771 | 58 |
| 0.4 | 1 | CD | effectiveness | 0.01583 | 92 |
| 0.4 | 2 | UC | pred iction | 0.078552 | 73 |
| 0.4 | 2 | UC | prediction2 | 0.044295 | 82 |
| 0.4 | 2 | UC | effectiveness | 0.005908 | 98 |
| 0.4 | 2 | CD | pred iction | 0.066315 | 78 |
| 0.4 | 2 | CD | prediction2 | 0.0372 | 85 |
| 0.4 | 2 | CD | effectiveness | 0.00157 | 100 |
| 0.4 | 3 | UC | pred iction | 0.041327 | 87 |
| 0.4 | 3 | UC | prediction2 | 0.018661 | 92 |
| 0.4 | 3 | UC | effectiveness | 0.002044 | 100 |
| 0.4 | 3 | CD | pred iction | 0.025349 | 89 |
| 0.4 | 3 | CD | prediction2 | 0.011396 | 94 |
| 0.4 | 3 | CD | effectiveness | 0.000341 | 100 |
| 0.4 | 6 | UC | pred iction | 0.009068 | 97 |
| 0.4 | 6 | UC | prediction2 | 0.003979 | 100 |
| 0.4 | 6 | UC | effectiveness | 0.000992 | 100 |
| 0.4 | 6 | CD | pred iction | 0.002672 | 99 |
| 0.4 | 6 | CD | prediction2 | 0.000537 | 100 |
| 0.4 | 6 | CD | effectiveness | 0.000129 | 100 |
| 0.5 | 1 | UC | pred iction | 0.099554 | 62 |
| 0.5 | 1 | UC | prediction2 | 0.079711 | 72 |
| 0.5 | 1 | UC | effectiveness | 0.033755 | 87 |
| 0.5 | 1 | CD | pred iction | 0.108109 | 67 |
| 0.5 | 1 | CD | prediction2 | 0.08967 | 67 |
| 0.5 | 1 | CD | effectiveness | 0.009958 | 94 |
| 0.5 | 2 | UC | pred iction | 0.036728 | 85 |
| 0.5 | 2 | UC | prediction2 | 0.026986 | 89 |
| 0.5 | 2 | UC | effectiveness | 0.003925 | 99 |
| 0.5 | 2 | CD | pred iction | 0.024161 | 88 |
| 0.5 | 2 | CD | prediction2 | 0.015855 | 92 |
| 0.5 | 2 | CD | effectiveness | 0.000855 | 100 |
| 0.5 | 3 | UC | pred iction | 0.023165 | 91 |
| 0.5 | 3 | UC | prediction2 | 0.009574 | 97 |
| 0.5 | 3 | UC | effectiveness | 0.001632 | 100 |
| 0.5 | 3 | CD | pred iction | 0.006734 | 96 |
| 0.5 | 3 | CD | prediction2 | 0.005888 | 97 |
| 0.5 | 3 | CD | effectiveness | 0.000321 | 100 |
| 0.5 | 6 | UC | pred iction | 0.005007 | 99 |
| 0.5 | 6 | UC | prediction2 | 0.003101 | 100 |
| 0.5 | 6 | UC | effectiveness | 0.000953 | 100 |
| 0.5 | 6 | CD | pred iction | 0.000485 | 100 |
| 0.5 | 6 | CD | prediction2 | 0.000281 | 100 |
| 0.5 | 6 | CD | effectiveness | 0.000117 | 100 |

### LIST A:

Gene set T=0.3, N=2, CD effectiveness: BCL2L11, DNMT1, ENPP2, EZR, GLRX, MMP1, MMP3, MMP9, PLAU, PLAUR, PTGS2, SERPINE1, TIMP1, VIM, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, SELE, STAT5A, TNF, TNFAIP2, TNIP1, TRAF1, VCAM1
Gene set T=0.3, N=3, UC effectiveness: CDKN1A, EAF2, ELL2, FKBP5, IGF1, LCP1, LRIG1, PLAU, PMEPA1, PPAP2A, PRKACB, PTPN1, SGK1, TMPRSS2, UGT2B15, BCL2L11, DNMT1, ENPP2, EZR, GLRX, MMP1, MMP3, MMP9, PLAUR, PTGS2, SERPINE1, TIMP1, VIM
Gene set T=0.3, N=3, CD prediction2: ANGPTL4, CDC42EP3, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI1, SNAI2, TIMP1, VEGFA, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TNIP1, TRAF1, VCAM1
Gene set T=0.3, N=3, CD effectiveness: BCL2L11, DNMT1, ENPP2, EZR, GLRX, MMP1, MMP3, MMP9, PLAU, PLAUR, PTGS2, SERPINE1, TIMP1, VIM, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, SELE, STAT5A, TNF, TNFAIP2, TNIP1, TRAF1, VCAM1
Gene set T=0.3, N=6, UC prediction: ANGPTL4, CDC42EP3, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI1, SNAI2, TIMP1, VEGFA, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TNIP1, TRAF1, VCAM1, BCL2L11, CCND1, DNMT1, ENPP2, EZR, GLRX, MMP1, MMP3, PLAU, PLAUR, VIM, CD274, CRP, FGF2, FOS, FSCN1, HIF1A, HSP90AA1, HSP90AB1, HSP90B1, IFNG, IL10, MCL1, MYC, NOS2, SAA1, STAT1, TNFRSF1B, TWIST1, ZEB1, CD44, COL18A1, DKK1, HNF1A, KIAA1199, LEF1, NKD1, PPARG, SP5, TBX3, TCF7L2
Gene set T=0.3, N=6, UC prediction2: ANGPTL4, CDC42EP3, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI1, SNAI2, TIMP1, VEGFA, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TNIP1, TRAF1, VCAM1
Gene set T=0.3, N=6, UC effectiveness: CDKN1A, EAF2, ELL2, FKBP5, IGF1, LCP1, LRIG1, PLAU, PMEPA1, PPAP2A, PRKACB, PTPN1, SGK1, TMPRSS2, UGT2B15, BCL2L11, DNMT1, ENPP2, EZR, GLRX, MMP1, MMP3, MMP9, PLAUR, PTGS2, SERPINE1, TIMP1, VIM
Gene set T=0.3, N=6, CD prediction: ANGPTL4, CDC42EP3, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI1, SNAI2, TIMP1, VEGFA, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TNIP1, TRAF1, VCAM1, BCL2L11, CCND1, DNMT1, ENPP2, EZR, GLRX, MMP1, MMP3, PLAU, PLAUR, VIM, CD274, CRP, FGF2, FOS, FSCN1, HIF1A, HSP90AA1, HSP90AB1, HSP90B1, IFNG, IL10, MCL1, MYC, NOS2, SAA1, STAT1, TNFRSF1B, TWIST1, ZEB1, CD44, COL18A1, DKK1, HNF1A, KIAA1199, LEF1, NKD1, PPARG, SP5, TBX3, TCF7L2
Gene set T=0.3, N=6, CD prediction2: ANGPTL4, CDC42EP3, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI1, SNAI2, TIMP1, VEGFA, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TNIP1, TRAF1, VCAM1
Gene set T=0.3, N=6, CD effectiveness: BCL2L11, DNMT1, ENPP2, EZR, GLRX, MMP1, MMP3, MMP9, PLAU, PLAUR, PTGS2, SERPINE1, TIMP1, VIM, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, SELE, STAT5A, TNF, TNFAIP2, TNIP1, TRAF1, VCAM1
Gene set T=0.4, N=1, CD effectiveness: BCL2L11, DNMT1, ENPP2, GLRX, MMP1, MMP3, MMP9, PLAU, PLAUR, PTGS2, SERPINE1, TIMP1, VIM, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, SELE, STAT5A, TNF, TNFAIP2, TRAF1, VCAM1
Gene set T=0.4, N=2, UC effectiveness: CDKN1A, EAF2, ELL2, FKBP5, LCP1, PLAU, PMEPA1, PRKACB, SGK1, TMPRSS2, BCL2L11, DNMT1, ENPP2, GLRX, MMP1, MMP3, MMP9, PLAUR, PTGS2, SERPINE1, TIMP1, VIM
Gene set T=0.4, N=2, CD effectiveness: BCL2L11, DNMT1, ENPP2, GLRX, MMP1, MMP3, MMP9, PLAU, PLAUR, PTGS2, SERPINE1, TIMP1, VIM, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, SELE, STAT5A, TNF, TNFAIP2, TRAF1, VCAM1
Gene set T=0.4, N=3, UC prediction2: ANGPTL4, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI2, TIMP1, VEGFA, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1
Gene set T=0.4, N=3, UC effectiveness: CDKN1A, EAF2, ELL2, FKBP5, LCP1, PLAU, PMEPA1, PRKACB, SGK1, TMPRSS2, BCL2L11, DNMT1, ENPP2, GLRX, MMP1, MMP3, MMP9, PLAUR, PTGS2, SERPINE1, TIMP1, VIM
Gene set T=0.4, N=3, CD prediction2: ANGPTL4, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI2, TIMP1, VEGFA, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1
Gene set T=0.4, N=3, CD effectiveness: BCL2L11, DNMT1, ENPP2, GLRX, MMP1, MMP3, MMP9, PLAU, PLAUR, PTGS2, SERPINE1, TIMP1, VIM, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, SELE, STAT5A, TNF, TNFAIP2, TRAF1, VCAM1
Gene set T=0.4, N=6, UC prediction: ANGPTL4, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI2, TIMP1, VEGFA, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1, BCL2L11, CCND1, DNMT1, ENPP2, GLRX, MMP1, MMP3, PLAU, PLAUR, VIM, CD274, FGF2, FOS, FSCN1, HIF1A, HSP90AB1, HSP90B1, IFNG, IL10, MCL1, MYC, NOS2, SAA1, STAT1, TNFRSF1B, TWIST1, ZEB1, CD44, COL18A1, DKK1, HNF1A, KIAA1199, LEF1, TBX3, TCF7L2
Gene set T=0.4, N=6, UC prediction2: ANGPTL4, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI2, TIMP1, VEGFA, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1
Gene set T=0.4, N=6, UC effectiveness: CDKN1A, EAF2, ELL2, FKBP5, LCP1, PLAU, PMEPA1, PRKACB, SGK1, TMPRSS2, BCL2L11, DNMT1, ENPP2, GLRX, MMP1, MMP3, MMP9, PLAUR, PTGS2, SERPINE1, TIMP1, VIM
Gene set T=0.4, N=6, CD prediction: ANGPTL4, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI2, TIMP1, VEGFA, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1, BCL2L11, CCND1, DNMT1, ENPP2, GLRX, MMP1, MMP3, PLAU, PLAUR, VIM, CD274, FGF2, FOS, FSCN1, HIF1A, HSP90AB1, HSP90B1, IFNG, IL10, MCL1, MYC, NOS2, SAA1, STAT1, TNFRSF1B,TWIST1, ZEB1, CD44, COL18A1, DKK1, HNF1A, KIAA1199, LEF1, TBX3, TCF7L2
Gene set T=0.4, N=6, CD prediction2: ANGPTL4, CDKN2B, CTGF, GADD45B, HMGA2, IL11, INPP5D, JUNB, MMP2, MMP9, OVOL1, PDGFB, PTHLH, SERPINE1, SKIL, SMAD4, SNAI2, TIMP1, VEGFA, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1
Gene set T=0.4, N=6, CD effectiveness: BCL2L11, DNMT1, ENPP2, GLRX, MMP1, MMP3, MMP9, PLAU, PLAUR, PTGS2, SERPINE1, TIMP1, VIM, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, SELE, STAT5A, TNF, TNFAIP2, TRAF1, VCAM1
Gene set T=0.5, N=1, CD effectiveness: GLRX, MMP1, MMP3, MMP9, PLAU, PLAUR, PTGS2, SERPINE1, TIMP1, VIM, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, SELE, STAT5A, TNF, TNFAIP2, TRAF1, VCAM1 Gene set T=0.5, N=2, UC effectiveness: EAF2, ELL2, FKBP5, LCP1, PLAU, PMEPA1, TMPRSS2, GLRX, MMP1, MMP3, MMP9, PLAUR, PTGS2, SERPINE1, TIMP1, VIM
Gene set T=0.5, N=2, CD prediction: ANGPTL4, CDKN2B, CTGF, GADD45B, IL11, INPP5D, JUNB, MMP2, MMP9, PTHLH, SERPINE1, SNAI2, TIMP1, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1
Gene set T=0.5, N=2, CD effectiveness: GLRX, MMP1, MMP3, MMP9, PLAU, PLAUR, PTGS2, SERPINE1, TIMP1, VIM, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, SELE, STAT5A, TNF, TNFAIP2, TRAF1, VCAM1
Gene set T=0.5, N=3, UC prediction: ANGPTL4, CDKN2B, CTGF, GADD45B, IL11, INPP5D, JUNB, MMP2, MMP9, PTHLH, SERPINE1, SNAI2, TIMP1, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1, CCND1, GLRX, MMP1, MMP3, PLAU, PLAUR, VIM, CD274, FGF2, FSCN1, HIF1A, HSP90B1, IFNG, MCL1, STAT1, TNFRSF1B,TWIST1, ZEB1, CD44, COL18A1, DKK1, KIAA1199, TBX3
Gene set T=0.5, N=3, UC prediction2: ANGPTL4, CDKN2B, CTGF, GADD45B, IL11, INPP5D, JUNB, MMP2, MMP9, PTHLH, SERPINE1, SNAI2, TIMP1, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1
Gene set T=0.5, N=3, UC effectiveness: EAF2, ELL2, FKBP5, LCP1, PLAU, PMEPA1, TMPRSS2, GLRX, MMP1, MMP3, MMP9, PLAUR, PTGS2, SERPINE1, TIMP1, VIM
Gene set T=0.5, N=3, CD prediction: ANGPTL4, CDKN2B, CTGF, GADD45B, IL11, INPP5D, JUNB, MMP2, MMP9, PTHLH, SERPINE1, SNAI2, TIMP1, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1, CCND1, GLRX, MMP1, MMP3, PLAU, PLAUR, VIM, CD274, FGF2, FSCN1, HIF1A, HSP90B1, IFNG, MCL1, STAT1, TNFRSF1B,TWIST1, ZEB1, CD44, COL18A1, DKK1, KIAA1199, TBX3
Gene set T=0.5, N=3, CD prediction2: ANGPTL4, CDKN2B, CTGF, GADD45B, IL11, INPP5D, JUNB, MMP2, MMP9, PTHLH, SERPINE1, SNAI2, TIMP1, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1
Gene set T=0.5, N=3, CD effectiveness: GLRX, MMP1, MMP3, MMP9, PLAU, PLAUR, PTGS2, SERPINE1, TIMP1, VIM, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, SELE, STAT5A, TNF, TNFAIP2, TRAF1, VCAM1 Gene set T=0.5, N=6, UC prediction: ANGPTL4, CDKN2B, CTGF, GADD45B, IL11, INPP5D, JUNB, MMP2, MMP9, PTHLH, SERPINE1, SNAI2, TIMP1, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1, CCND1, GLRX, MMP1, MMP3, PLAU, PLAUR, VIM, CD274, FGF2, FSCN1, HIF1A, HSP90B1, IFNG, MCL1, STAT1, TNFRSF1B, TWIST1, ZEB1, CD44, COL18A1, DKK1, KIAA1199, TBX3
Gene set T=0.5, N=6, UC prediction2: ANGPTL4, CDKN2B, CTGF, GADD45B, IL11, INPP5D, JUNB, MMP2, MMP9, PTHLH, SERPINE1, SNAI2, TIMP1, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1
Gene set T=0.5, N=6, UC effectiveness: EAF2, ELL2, FKBP5, LCP1, PLAU, PMEPA1, TMPRSS2, GLRX, MMP1, MMP3, MMP9, PLAUR, PTGS2, SERPINE1, TIMP1, VIM
Gene set T=0.5, N=6, CD prediction: ANGPTL4, CDKN2B, CTGF, GADD45B, IL11, INPP5D, JUNB, MMP2, MMP9, PTHLH, SERPINE1, SNAI2, TIMP1, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1, CCND1, GLRX, MMP1, MMP3, PLAU, PLAUR, VIM, CD274, FGF2, FSCN1, HIF1A, HSP90B1, IFNG, MCL1, STAT1, TNFRSF1B, TWIST1, ZEB1, CD44, COL18A1, DKK1, KIAA1199, TBX3
Gene set T=0.5, N=6, CD prediction2: ANGPTL4, CDKN2B, CTGF, GADD45B, IL11, INPP5D, JUNB, MMP2, MMP9, PTHLH, SERPINE1, SNAI2, TIMP1, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, PTGS2, SELE, STAT5A, TNF, TNFAIP2, TRAF1, VCAM1
Gene set T=0.5, N=6, CD effectiveness: GLRX, MMP1, MMP3, MMP9, PLAU, PLAUR, PTGS2, SERPINE1, TIMP1, VIM, BCL2L1, BIRC3, CCL2, CCL22, CCL3, CCL4, CCL5, CX3CL1, CXCL1, CXCL2, CXCL3, ICAM1, IL1B, IL6, IL8, IRF1, NFKB2, NFKBIA, NFKBIE, SELE, STAT5A, TNF, TNFAIP2, TRAF1, VCAM1

## Claims

1. A method for predicting the treatment response of a subject suffering from inflammatory bowel disease (IBD) to treatment with an anti-TNFalpha compound based on the expression levels of three or more genes, wherein the expression levels of the three or more genes are selected from the group consisting of:
ANGPTL4, BCL2L1, BCL2L11, BIRC3, CCL2, CCL22, CCL3, CCL4, CCL5, CCND1, CD274, CD44, CDKN2B, COL18A1, CTGF, CX3CL1, CXCL1, CXCL2, CXCL3, DKK1, DNMT1, ENPP2, FGF2, FOS, FSCN1, GADD45B, GLRX, HIF1A, HMGA2, HNF1A, HSP90AB1, HSP90B1, ICAM1, IFNG, IL11, IL10, IL1B, IL6, IL8, IRF1, INPP5D, JUNB, KIAA1199, LEF1, MCL1, MMP1, MMP2, MMP3, MMP9, MYC, NFKB2, NFKBIA, NFKBIE, NOS2, OVOL1, PDGFB, PLAU, PLAUR, PTGS2, PTHLH, SAA1, SELE, SERPINE1, SKIL, SMAD4, SNAI2, STAT1, STAT5A, TBX3, TCF7L2, TIMP1, TNF, TNFAIP2, TNFRSF1B, TRAF1, TWIST1, VCAM1, VEGFA, VIM, and ZEB1, and
wherein an increased expression level of CDKN2B, HMGA2, OVOL1, SMAD4, BCL2L11, HNF1A or TCF7L2, or a decreased expression level of any of the other genes corresponds to a favorable treatment response, and
wherein a decreased expression level of CDKN2B, HMGA2, OVOL1, SMAD4, BCL2L11, HNF1A or TCF7L2, or an increased expression level of any of the other genes corresponds to a not favorable treatment response.

2. Method according to claim 1, wherein the expression levels of the three or more genes are selected from the group consisting of:
ANGPTL4, BCL2L1, BIRC3, CCL2, CCL22, CCL3, CCL4, CCL5, CCND1, CD274, CD44, CDKN2B, COL18A1, CTGF, CX3CL1, CXCL1, CXCL2, CXCL3, DKK1, FGF2, FSCN1, GADD45B, GLRX, HIF1A, HSP90B1, ICAM1, IFNG, IL11, IL1B, IL6, IL8, INPP5D, IRF1, JUNB, KIAA1199, MCL1, MMP1, MMP2, MMP3, MMP9, NFKB2, NFKBIA, NFKBIE, PLAU, PLAUR, PTGS2, PTHLH, SELE, SERPINE1, SNAI2, STAT1, STAT5A, TBX3, TIMP1, TNF, TNFAIP2, TNFRSF1B, TRAF1, TWIST1, VCAM1, VIM, ZEB1.

3. Method according to any one of the preceding claims, wherein the three or more expression levels are combined by adding up their relative expression levels, with the proviso that the expression levels of the genes CDKN2B, HMGA2, OVOL1, SMAD4, BCL2L11, HNF1A or TCF7L2 are multiplied with a weight factor of -1,
preferably wherein the combined expression levels are compared to a threshold value to determine whether the treatment response is favorable or not favorable, wherein the treatment response is predicted to be favorable when the combined three or more expression levels are below the threshold value, and wherein the treatment response is predicted to be unfavorable when the combined three or more expression levels are above the threshold value.

4. Method according to any one of the preceding claims, wherein the IBD is Ulcerative Colitis (UC) or Crohn Disease (CD).

5. A method for determining the effectiveness of a treatment of a subject suffering from IBD with an anti-TNFalpha compound based on the expression levels of two or more genes,
wherein if the IBD is UC, the two or more genes are selected from the group consisting of BCL2L11, CDKN1A, DNMT1, EAF2, ELL2, ENPP2, FKBP5, GLRX, LCP1, MMP1, MMP3, MMP9, PLAU, PLAUR, PMEPA1, PRKACB, PTGS2, SERPINE1, SGK1, TIMP1, TMPRSS2 and VIM; or
wherein the IBD is CD, the two or more genes are selected from the group consisting of BCL2L1, BCL2L11, BIRC3, CCL2, CCL22, CCL3, CCL4, CCL5, CX3CL1, CXCL1, CXCL2, CXCL3, DNMT1, ENPP2, GLRX, ICAM1, IL1B, IL6, IL8, IRF1, MMP1, MMP3, MMP9, NFKB2, NFKBIA, NFKBIE, PLAU, PLAUR, PTGS2, SELE, SERPINE1, STAT5A, TIMP1, TNF, TNFAIP2, TRAF1, VCAM1 and VIM;
wherein the two or more expression levels are used to determine whether the treatment of IBD in the patient with an anti-TNFalpha compound is effective or not effective,
wherein a decreased expression level of BCL2L11 or PRKACB or an increased expression level of any of the other genes corresponds to a not effective treatment, and
wherein an increased expression level of BCL2L11 or PRKACB or a decreased expression level of any of the other genes corresponds to an effective treatment.

6. Method according to claim 5, wherein:
wherein if the IBD is UC and the two or more genes are selected from the group consisting of EAF2, ELL2, FKBP5, GLRX, LCP1, MMP1, MMP3, MMP9, PLAU, PLAUR, PMEPA1, PTGS2, SERPINE1, TIMP1, TMPRSS2 and VIM; or
wherein if the IBD is CD and the two or more genes are selected from the group consisting of BCL2L1, BIRC3, CCL2, CCL22, CCL3, CCL4, CCL5, CX3CL1, CXCL1, CXCL2, CXCL3, GLRX, ICAM1, IL1B, IL6, IL8, IRF1, MMP1, MMP3, MMP9, NFKB2, NFKBIA, NFKBIE, PLAU, PLAUR, PTGS2, SELE, SERPINE1, STAT5A, TIMP1, TNF, TNFAIP2, TRAF1, VCAM1 and VIM.

7. Method according to claim 5 or 6 , wherein the two or more expression levels are combined by adding up their relative expression levels, with the proviso that the expression levels of the genes BCL2L11 or PRKACB are multiplied with a weight factor of - 1,
preferably wherein the combined expression levels are compared to a threshold value to determine whether the treatment is effective or not effective, wherein the treatment is predicted to be effective when the combined two or more expression levels are below the threshold value, and wherein the treatment is predicted not to be effective when the combined two or more expression levels are above the threshold value.

8. Method according to any one of the preceding claims, wherein the threshold value is independently determined from one or more reference samples.

9. Method according to any one of the preceding claims, wherein the sample obtained from the subject is a gastric, colon, intestinal or rectal sample.

10. Method according to any one of the preceding claims, wherein the method further includes determining the expression levels of the target genes based on mRNA extracted from the sample obtained from the subject, preferably wherein the method further includes extracting the mRNA from the sample obtained from the subject.

11. An anti-TNFalpha compound for use of in the treatment of IBD in a subject, wherein the use comprises:
performing the method as defined in any one of the preceding claims to determine the treatment response of the subject to anti-TNFalpha treatment, and
administering the anti-TNFalpha compound of the treatment response to anti-TNFalpha treatment is found to be favorable for the subject.

12. Anti-TNFalpha compound for use according to claim 11, wherein the IBD is UC or CD.

13. Anti-TNFalpha compound for use according to claim 11 or 12, wherein the anti-TNFalpha compound is selected from the group consisting of antibody based compounds Adalimumab, Certolizumab, Etanercept, Golimumab, Infliximab, or a biosimilar or a chimeric product of any of the preceding compounds such as the CT-P13 Infliximab biosimilar, or small molecule based compounds thalidomide, lenalidomide, pomalidomide, a xanthane derivative such as pentoxifylline, bupropion, an 5-HT_{2A} agonist such as (R)DOI, TCB2, LSD and LA-SS-Az, curcumin, catechins, cannabidiol or Echinacea purpurea.

14. Kit of parts comprising primers and probes for the detection of the expression levels of three or more target genes, wherein the expression levels of the three or more genes are selected from:
the group consisting of ANGPTL4, BCL2L1, BCL2L11, BIRC3, CCL2, CCL22, CCL3, CCL4, CCL5, CCND1, CD274, CD44, CDKN2B, COL18A1, CTGF, CX3CL1, CXCL1, CXCL2, CXCL3, DKK1, DNMT1, ENPP2, FGF2, FOS, FSCN1, GADD45B, GLRX, HIF1A, HMGA2, HNF1A, HSP90AB1, HSP90B1, ICAM1, IFNG, IL11, IL10, IL1B, IL6, IL8, IRF1, INPP5D, JUNB, KIAA1199, LEF1, MCL1, MMP1, MMP2, MMP3, MMP9, MYC, NFKB2, NFKBIA, NFKBIE, NOS2, OVOL1, PDGFB, PLAU, PLAUR, PTGS2, PTHLH, SAA1, SELE, SERPINE1, SKIL, SMAD4, SNAI2, STAT1, STAT5A, TBX3, TCF7L2, TIMP1, TNF, TNFAIP2, TNFRSF1B, TRAF1, TWIST1, VCAM1, VEGFA, VIM, and ZEB1; or
the group consisting of BCL2L11, CDKN1A, DNMT1, EAF2, ELL2, ENPP2, FKBP5, GLRX, LCP1, MMP1, MMP3, MMP9, PLAU, PLAUR, PMEPA1, PRKACB, PTGS2, SERPINE1, SGK1, TIMP1, TMPRSS2 and VIM; or
the group consisting of BCL2L1, BCL2L11, BIRC3, CCL2, CCL22, CCL3, CCL4, CCL5, CX3CL1, CXCL1, CXCL2, CXCL3, DNMT1, ENPP2, GLRX, ICAM1, IL1B, IL6, IL8, IRF1, MMP1, MMP3, MMP9, NFKB2, NFKBIA, NFKBIE, PLAU, PLAUR, PTGS2, SELE, SERPINE1, STAT5A, TIMP1, TNF, TNFAIP2, TRAF1, VCAM1 and VIM.

15. Use of the kit according to claim 14 in a method as defined in any one of claims 1 to 10.
